# EUROPÄISCHE PATENTSCHRIFT

(11) **EP 2 576 600 B1**
(45) Veröffentlichungstag und Bekanntmachung des Hinweises auf die Patenterteilung: **24.01.2018**
(21) Anmeldenummer: 11721780.2
(22) Anmeldetag: 27.05.2011
(51) Int. Cl.: C07K 14/245, C12P 33/00, C12P 33/06

(54) **NEUARTIGE 7ALPHA-HYDROXYSTEROID DEHYDROGENASE-KNOCKOUT-MUTANTEN UND DEREN VERWENDUNG**
NOVEL 7ALPHA-HYDROXYSTEROID DEHYDROGENASE KNOCKOUT MUTANTS AND USE THEREOF
NOUVEAUX MUTANTS KNOCK-OUT DE LA 7ALPHA-HYDROXYSTÉROÏDE DÉSHYDROGÉNASE ET LEUR UTILISATION

(30) Priorität: 16.12.2010 EP 10015726; 27.05.2010 EP 10164003
(43) Veröffentlichungstag der Anmeldung: 10.04.2013
(73) Patentinhaber: PharmaZell GmbH, 83064 Raubling (DE)
(72) Erfinder: SCHMID, Rolf, 70569 Stuttgart (DE); BRAUN, Michael, 97980 Bad-Mergentheim-Edelfingen (DE); LIU, Luo, Beijing 102445 (CN); AIGNER, Arno, 83104 Tuntenhausen (DE); WEUSTER-BOTZ, Dirk, 80634 München (DE)
(74) Vertreter: Reitstötter Kinzebach
(86) Internationale Anmeldenummer: PCT/EP2011/058711
(87) Internationale Veröffentlichungsnummer: WO 2011/147957

(56) Entgegenhaltungen:
- EP-A1- 2 105 500
- WO-A2-03/051182
- WO-A2-2009/118176
- E. MOBUS: "Molecular Cloning, Overexpression, and Characterization of Steroid-inducible 3alpha -Hydroxysteroid Dehydrogenase/Carbonyl Reductase from Comamonas testosteroni. A NOVEL MEMBER OF THE SHORT-CHAIN DEHYDROGENASE/REDUCTASE SUPERFAMILY", JOURNAL OF BIOLOGICAL CHEMISTRY, Bd. 273, Nr. 47, 20. November 1998 (1998-11-20), Seiten 30888-30896, XP55003957, ISSN: 0021-9258, DOI: 10.1074/jbc.273.47.30888
- BRAUN M ET AL: "12 alpha-hydroxysteroid dehydrogenase from Clostridium group P, strain C 48-50. Production, purification and characterization", EUROPEAN JOURNAL OF BIOCHEMISTRY, BLACKWELL PUBLISHING, BERLIN, DE, Bd. 196, Nr. 2, 14. März 1991 (1991-03-14) , Seiten 439-450, XP002482707, ISSN: 0014-2956, DOI: DOI:10.1111/J.1432-1033.1991.TB15835.X
- DATABASE UniProt [Online] 17. April 2007 (2007-04-17), Sudarsanam P et al.: "SubName: Full=Putative uncharacterized protein;", XP002655020, gefunden im EBI accession no. UNIPROT:A4ECA9 Database accession no. A4ECA9
- PEDRO VICO, GILLES CAUET, KEN ROSE, RICHARD LATE AND ERIC DEGRYSE: "Dehydroepiandrosterone (DHEA) metabolism in Saccharomyces cerevisiae expressing mammalian steroid hydroxylase CYP7B: Ayr1p and Fox2p display 17-beta-hydroxysteroid dehydrogenase activity", YEAST, vol. 19, 24 June 2002 (2002-06-24), pages 873-886, DOI: 10.1002/yea.882

## Beschreibung

Die Erfindung betrifft neuartige mikrobielle 7α-Hydroxysteroid Dehydrogenase (7α-HSDH)-Knockout Mutanten, und deren Verwendung zur Herstellung anderen HSDHs unterschiedlicher Funktionalität, wie z.B. 3α-, 7β- oder 12α-HSDH, sowie die Verwendung der so hergestellten HSDH-Enzyme bei enzymatischen Umsetzungen von Cholsäureverbindungen, und insbesondere bei der Herstellung von Ursodesoxycholsäure (UDCS); Gegenstand der Erfindung sind insbesondere neuartige Verfahren zur Synthese von UDCS.

### Hintergrund der Erfindung:

Zur medikamentösen Behandlung von Gallensteinleiden werden seit vielen Jahren u. a. die Wirkstoffe Ursodesoxycholsäure (UDCS) und das zugehörige Diastereomer Chenodesoxycholsäure (CDCS) eingesetzt. Beide Verbindungen unterscheiden sich lediglich durch die Konfiguration der Hydroxygruppe am C-Atom 7 (UDCS: β -Konfiguration, CDCS: α-Konfiguration). Zur Herstellung von UDCS sind im Stand der Technik verschieden Verfahren beschreiben, welche rein chemisch durchgeführt werden oder aus einer Kombination chemischer und enzymatischer Verfahrensschritte bestehen. Ausgangspunkt ist jeweils Cholsäure (CS) oder die aus Cholsäure hergestellte CDCS.

So kann die klassisch chemische Methode zur UDCS-Herstellung wie folgt schematisch dargestellt werden:

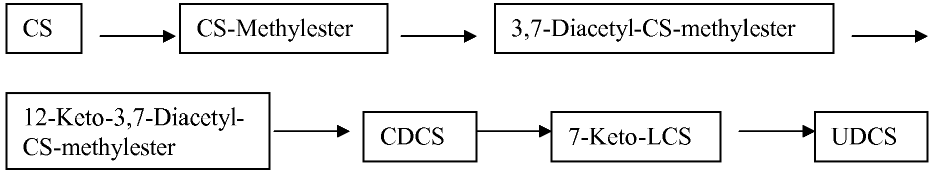

Ein gravierender Nachteil ist unter anderem folgender: da die chemische Oxidation nicht selektiv ist, müssen die Carboxy-Gruppe und die 3α und 7α-Hydroxy-Gruppe durch Veresterung geschützt werden.

Ein alternatives chemisch/enzymatische Verfahren, basierend auf der Verwendung des Enzyms 12α-HSDH ist z.B. beschrieben in der PCT/EP2009/002190 der vorliegenden Anmelderin.

Insbesondere wird dabei rekombinant hergestellte 12α-HSDH eingesetzt. Das Enzym wird dabei bevorzugt von rekombinanten E. coli Wirten produziert, daraus isoliert und zur Reaktion eingesetzt.

Die 12α-HSDH oxidiert dabei CS selektiv zu 12-Keto-CDCS. Die zwei nach der klassisch chemischen Methode erforderlichen chemischen Schützschritte entfallen dabei.

Problematisch bei diesem Verfahren ist, dass das auf diese Weise hergestellte Wertprodukt UDCS aus bisher unbekannten Gründen mit Lithocholsäure (LCS) verunreinigt ist.

Neuartige 7β-HSDHs und deren Verwendung bei der Herstellung von UDCS auf reduktivem Weg sind in der PCT/EP2010/068576 der Anmelderin beschrieben. Problematisch dabei ist, dass die rekombinanten E. coli Wirten auch endogene 7α-HSDH produzieren, Die 7α-HSDH erzeugt aus Dehydrocholsäure ein 3,12-Diketo-CDCS -Nebenprodukt. Diese 3,12-Diketo-CDCS ist dann kein Substrat der 7β-HSDH mehr, so dass neben dem gewünschten Produkt auch das unerwünschte Nebenprodukt akkumuliert wird.

Aufgabe der Erfindung ist daher die Bereitstellung eines neuen Verfahrens zur Herstellung von UDCS, welches die oben geschilderten Nachteile vermeidet. Insbesondere sollte die enzymatisch/chemische Herstellung von UDCS ohne Verunreinigungen, wie LCS oder 3,12-Diketo-CDCS ermöglicht werden.

### Kurzfassung der Erfindung:

Erfindungsgemäß wurde überraschend festgestellt, dass Ursache für das Auftreten von unerwünschten Verunreinigungen, wie z.B. LCS, nicht die unerwünschte Folge einer komplexen chemischen Nebenreaktion ist. Auch ist das Auftreten von LCS nicht die Folge einer durch 12α-HSDH selbst katalysierten Nebenreaktion. Vielmehr weist das den enzymatischen Umsetzungsschritt katalysierende 12α-HSDH-Enzym eine enzymatische Verunreinigung durch eine 7α-HSDH-Aktivität auf. Folge davon ist die Oxidation der 12-Keto-CDCS zu 7,12-Diketo-LCS, welche im Verlauf der weiteren chemischen Umsetzungsreaktionen, die zu UDCS führen, in einer chemischen Nebenreaktion zu LCS reduziert wird. Dies ist in folgendem Schema dargestellt.

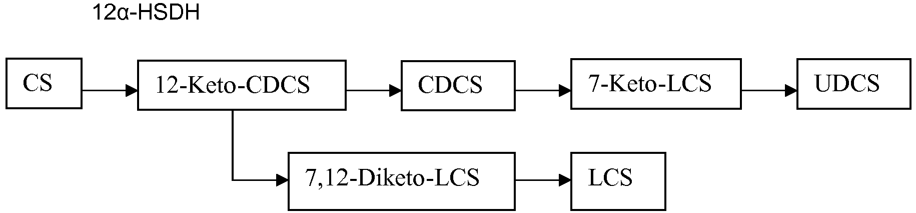

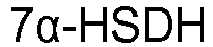

Es wurde insbesondere festgestellt, dass Ursache der Bildung von 7,12 Deketo-LCS die Verunreinigung der 12α-HSDH mit 7α-HSDH-Enzym ist. Bei der rekombinanten Herstellung von 12α-HSDH (wie deren Kurzform), vor allem bei Herstellung unter Verwendung von E. coli Wirtszellen, ist nämlich 12α-HSDH selbst nach Aufreinigung mit endogenem 7α-HSDH-Enzym verunreinigt. Beide Enzyme besitzen wegen praktisch identischer Sequenzlänge (257 bzw. 255 Aminosäuren für 12α-HSDH (Kurzform) bzw. 7α-HSDH) sehr ähnliche Molekulargewichte und auch ansonsten recht ähnliches Trennverhalten während der Aufreinigung, so dass die auf diese Weise hergestellte 12α-HSDH praktisch immer mit 7α-HSDH verunreinigt ist.

Auch die 7β-HSDH ist nicht ohne weiteres von der unerwünschten 7α-hSDH-Aktivität zu trennen.

Erfindungsgemäß gelang nun überraschenderweise die Bereitstellung von HSDHs, wie z.B. der 12α-HSDH oder der 7β-HSDH, welche keine 7α-HSDH-Aktivität mehr aufweist, erst durch gezieltes Ausschalten der Nebenaktivität in dem zur rekombinanten Herstellung der 12α-HSDH oder anderer HSDHs, wie z.B. der 7β-HSDH, verwendeten Expressionswirts.

So wird der Expressionswirt *E. coli* BL21(DE3) (Genotyp: F⁻ ompT gal dcm Ion hsdS_{B}(r_{B}⁻ m_{B}⁻) λ(DE3 [Iacl IacUV5-T7 gene 1 ind1 sam7 nin5]) (Novagen) (Journal of Molecular Biology (1985) 189, 113-130), in großem Umfang für Herstellung von rekombinaten Proteinen eingesetzt. *E. coli* BL21(DE3) enthält jedoch eine NADH-abhängige 7α-HSDH, welche z.B. die Bildung des unerwünschten Nebenproduktes Lithocholsäure bei der Herstellung von UDCS verursacht, wie nun erfindungsgemäß festgestellt wurde.

Zum Vermeiden der Nebenreaktion durch die 7α-HSDH aus *E. coli* BL21 (DE3) bei der Herstellung von UDCS, wurde erfindungsgemäß z.B. in einer ersten das Gen, das die endogene 7α-HSDH codiert, aus *E. coli* BL21 (DE3) erfolgreich duech homologe Rekombination ausgeknockt. Der resultierende Stamm *E. coli* BL21(DE3) Δ7α-HSDH erlaubt die rekombinante Herstellung von 12α-HSDH-Präparaten, welche bei der Produktion von UDCS die genannte unerwünschte Nebenreaktion nicht mehr verursachen.

Dadurch wird erfindungsgemäß, wie in folgender schematischer Darstellung gezeigt, erstmalig die Synthese von UDCS ohne die durch 7α-HSDH katalysierte Nebenreaktion ermöglicht. Die 12α-HSDH oxidiert somit CS selektiv zu 12-Keto-CDCS.

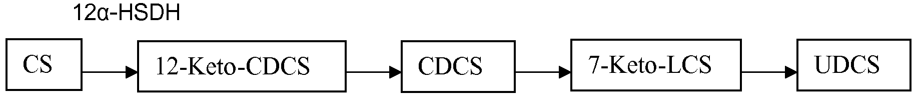

Die vorgeschlagene Lösung obiger Aufgabe ist um so überraschender, als festgestellt wurde, dass die endogene 7α-HSDH ein NADH-abhängiges Enzym ist, die rekombinant exprimierte 12α-HSDH jedoch NADPH abhängig ist. Die 7α-HSDH zeigt aber auch, wie erfindungsgemäß festgestellt wurde, Spurenaktivität in Anwesenheit von NADPH und trotz Fehlens von NADH. Diese Spurenaktivität ist ausreichend um das Wertprodukt UDCS letztendlich in unerwünschter Weise mit LCS zu verunreinigen, selbst dann wenn man den enzymatischen Syntheseschritt mit isoliertem Enzym und nicht mit ganzen Zellen durchführt. Bei Verwendung ganzer Zellen ist aufgrund der Gegenwart von zellulärem NADH aus Zellen 7α -HSDH voll aktiv, wodurch die Nebenprodukt-Bildung noch weiter drastisch erhöht würde.

Somit wird erfindungsgemäß auch die Herstellung von LCS-freier UDCS nach obigemenzymatisch/chemischem Syntheseweg unter Verwendung ganzer rekombinanter Zellen ermöglicht welche 12α-HSDH-Aktivität exprimieren, jedoch frei von störender 7α-HSDH-Aktivität sind.

### Figurenbeschreibung:

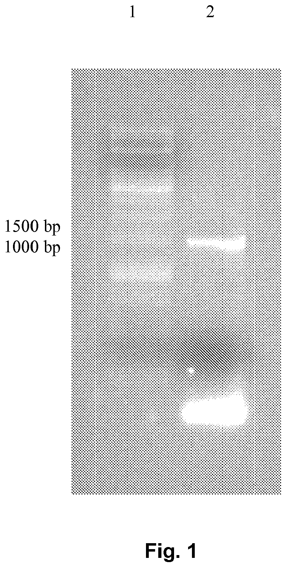

Figur 1 zeigt das Ergebnis einer Kolonie-PCR. Insbesondere ist eine Bande mit 1500 bp zu sehen, die dem durch Rekombinantion integrierten Aparemycin-Resistenzgen entspricht. Die Resistenz ist somit in das Genom integriert und das Gen der 7α-HSDH wurde ausgeknockt. Spur 1 zeigt den DNA Marker. Spur 2 das PCR Produkt mit 1500 bps.

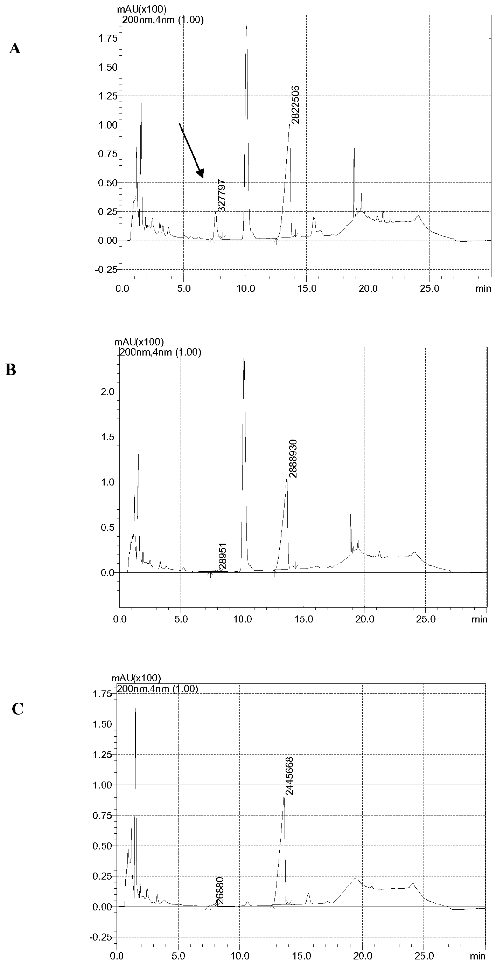

Figur 2 zeigt das Ergebnis der Inkubation von Cholsäure (CS) mit verschiedenen *E*. *coli* Kulturen. A: Cholsäure mit *E. coli* BL21(DE3); B: Cholsäure mit Knock-out Mutante *E. coli* BL21(DE3) Δ7α-HSDH; C: Cholsäure als Kontrolle. Der Pfeil in Grafik A zeigt ein Oxidationsprodukt (7-Keto-3,12-Dihydroxycholansäure; Retentionszeit (RT) = 7,5 min) bei der Umsetzung durch *E. coli* BL21 (DE3). Aufgrund einer Verunreinigung wurde auch bei dem Knockout-Stamm *E. coli* BL21(DE3) ein kleiner Peak im Bereich von 7-Keto-3,12-Dihydroxycholansäure gesehen, aber der beobachtete Peak ist nicht größer als in negativer Kontrolle (C).

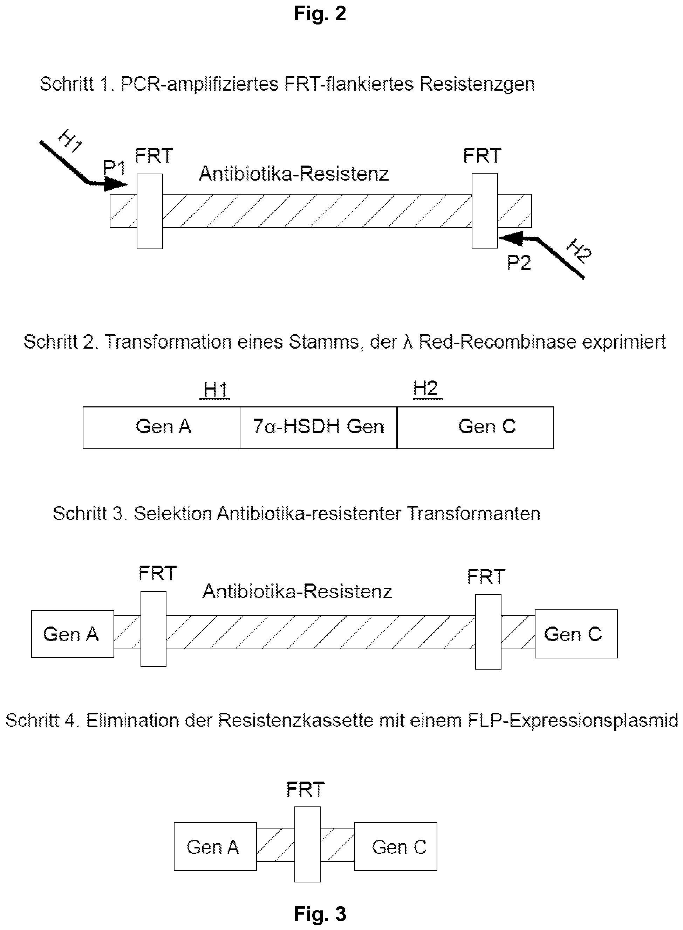

Figur 3 veranschaulicht schematisch die Strategie zur Ausschalten des 7α-HSDH-Gens mittels Knockout-Technologie. H1 und H2 bezeichnen homologe Sequenzen; P1 und P2 bezeichnen Primer. Gen A und C bezeichnen die das 7α-HSDH-Gen flankierenden Sequenzabschnitte. H1 und H2 können sowohl Sequenzabschnitte aus den flankierenden Bereichen sein (wie in Figur 3 gezeigt), oder können auch z.B. 5'- und 3'-terminale Anschnitte aus dem 7α-HSDH-Gen darstellen (Quelle: K. Datsenko und B. Wanner, PNAS June 6, 2000 vol. 97 no. 12 6640-6645.

### Spezielle Ausführungsformen der Erfindung

Gegenstand der Erfindung sind insbesondere folgende Ausführungsformen:
1. Rekombinanter Mikroorganismus, in welchem die enzymatische Aktivität der 7α-Hydroxysteroiddehydrogenase (7α-HSDH) inhibiert ist, während die enzymatische Aktivität einer davon funktionell verschiedenen Hydroxysteroiddehydrogenase (wie z.B. 3α-, 3β-, 7β-, 11α-, 11β-, 12α-, 12β-, 17α-, 17β-, 20α-, oder 20β-HSDH, insbesondere 3α-, 7β- oder 12α-HSDH) exprimierbar enthalten ist.

Die inhibierte 7α-HSDH kann dabei beliebige Cofaktorabhängigkeit zeigen, wie z.B. von NADH oder NADPH (bzw. NAD⁺ oder NADP⁺) abhängig sein.

Gegebenenfalls kann ein derartiger modifizierter Mikroorganismus zusätzlich auch so modifiziert sein, dass er nicht nur die gewünschte(n) HSDH-Aktivität(en) exprimiert sonder auch weitere Proteine oder Enzyme, welche ggf. mit der HSDH zusammenwirken können, wie z.B. Enzyme welche die Cofaktorregenerierung (wie unten näher erläutert) unterstützen. Beispiele hierfür sind Enzyme wie Alkoholdehydrogensen (ADH) und Formiatdehydrogenasen (FDH).

Insbesondere betrifft die Erfindung dabei einen rekombinanter Mikroorganismus, in welchem die enzymatische Aktivität der 7α-HSDH inhibiert ist, während die enzymatische Aktivität einer exogenen 12α-HSDH exprimierbar enthalten ist; wie insbesondere ein solcher Mikroorganismus, in welchem die endogene enzymatische Aktivität der (z.B. NADH-abhängigen) 7α-HSDH inhibiert ist, während die enzymatische Aktivität der (z.B. NADPH-abhängigen) 12α-HSDHexprimierbar enthalten ist. Grundsätzlich können dabei 7α-HSDH und 12α-HSDH gleiche oder verschiedene Cofaktor-Abhängigkeit aufweisen, d.h. 7α-HSDH und 12α-HSDH können beide von NADH oder NADPH (bzw. NAD⁺ oder NADP⁺) abhängig sein. 7α-HSDH und 12α-HSDH können aber auch von verschiedenen Cofaktoren abhängig sein; so kann 7α-HSDH z.B. von NADH (bzw. NAD⁺) abhängig sein und 12α-HSDH kann von NADPH (bzw. NADP⁺) abhängig sein oder umgekehrt. Insbesondere kann bei Abhängigkeit von verschiedenen Cofaktoren eine analytisch nachweisbare Enzymaktivität auch in Gegenwart des jeweils anderen Cofaktors beobachtet werden. So kann z.B. 7α-HSDH von NADH (bzw. NAD⁺) abhängig sein, jedoch auch in Gegenwart von NADPH (bzw. NADP⁺) analytisch nachweisbare enzymatische Aktivität Obiges gilt entsprechend wenn anstelle der 12α-HSDH eine oder mehrere andere HSDHs, wie z.B. ausgewählt unter 3α-, 3β-, 7β-, 11α-, 11β-, 12β-, 17α-, 17β-, 20α-und 20β-HSDH, exprimiert und gegebenenfalls isoliert werden sollen, wie z.B. eine 3α-und/oder 7β-HSDH.
2. Rekombinanter Mikroorganismus nach Ausführungsform 1, worin die 7α-HSDH kodierende Nukleinsäuresequenz ausgeknockt ist, insbesondere durch homologe Rekombination oder durch Gen-Disruption (insbesondere durch Insertion einer die Enzymfunktion des kodierten Genprodukts inhibierenden Nukleinsäuresequenz).
3. Rekombinanter Mikroorganismus nach einer der vorhergehenden Ausführungsformen, abgeleitet von einem 7α-HSDH exprimierenden Vorläufer, insbesondere einem 7α-HSDH endogen exprimierenden Vorläufer-Mikroorganismus, wie Bakterien der Gattung Escherichia, vor allem E. coli.
4. Rekombinanter Mikroorganismus nach Ausführungsform 3, abgeleitet von E. coli BL21.
5. Rekombinanter Mikroorganismus nach einer der vorhergehenden Ausführungsformen, welcher die von der 7α-HSDH funktionell verschiedene HSDH, wie insbesondere eine 3α-, 7β- und/oder 12α-HSDH rekombinant exprimiert.
6. Rekombinanter Mikroorganismus nach einer der vorhergehenden Ausführungsformen, wobei die inhibierte (ausgeknockte) 7α-HSDH eine Aminosäuresequenz nach SEQ ID NO: 10 aufweist oder von einer Nukleinsäuresequenz nach SEQ ID NO: 9 kodiert wird.
7. Rekombinanter Mikroorganismus nach einer der vorhergehenden Ausführungsformen, wobei die exprimierte von der 7α-HSDH funktionell verschiedene HSDH, eine der folgenden Aminosäuresequenzen aufweist:
   a) eine 3α-HSDH Sequenz ausgewählt unter: SEQ ID NO:29 und 31
   b) eine 7β-HSDH Sequenz ausgewählt unter: SEQ ID NO: 25
      und
   c) eine 12α-HSDHSequenz ausgewählt unter SEQ ID NO: 12, 14, 16; oder
   d) eine von a), b) oder c) abgeleitete, für eine HSDH kodierende Aminosäuresequenz mit einem Identitätsgrad von wenigstens 50% oder wenigstens 60 % oder wenigstens 75%,insbesondere wenigsten 85 %, wie z.B. 90, 91, 92, 93, 94, 95, 96, 97,98 oder 99%, zu einer dieser Sequenzen unter a), b) oder c) aufweist.
8. Verfahren zur rekombinanten Herstellung von einer von der 7α-HSDH funktionell verschiedenen gewünschten HSDH, wie z.B. einer 3α-, 3β-, 7β-, 11α-, 11β-, 12α-, 12β-, 17α-, 17β-, 20α-, oder 20β-HSDH, insbesondere 3α-, 7β- oder 12α-HSDH, wobei man einen rekombinanten Mikroorganismus nach einem der vorhergehenden Ansprüche in welchem die enzymatische Aktivität der 7α-HSDH inhibiert ist, unter Bedingungen kultiviert, unter denen die gewünschte HSDH exprimiert wird, und die so exprimierte HSDH isoliert, wobei in der isolierten HSDH im Wesentlichen keine funktionale 7α-HSDH nachweisbar ist

Insbesondere betrifft die Erfindung dabei die Herstellung einer, insbesondere NADPHabhängiger, 12α-HSDH, wobei man einen rekombinanten Mikroorganismus nach einer der vorhergehenden Ausführungsformen unter 12α-HSDH exprimierenden Bedingungen kultiviert, und die exprimierte 12α-HSDH isoliert, wobei das isolierte Enzym nicht durch 7α-HSDH Enzym verunreinigt ist.

Insbesondere betrifft die Erfindung dabei aber auch die Herstellung einer NADH- oder insbesondere NADPH-abhängigen 7β-HSDH, wobei man einen rekombinanten Mikroorganismus nach einer der vorhergehenden Ausführungsformen unter 7β-HSDH exprimierenden Bedingungen kultiviert, und die exprimierte 7β-HSDH isoliert, wobei das isolierte Enzym nicht durch 7α-HSDH Enzym verunreinigt ist.
9. Rekombinante HSDH, wie z.B. 3α-, 3β-, 7β-, 11α-, 11β-, 12α-, 12β-, 17α-, 17β-, 20α-, oder 20β-HSDH, insbesondere 3α-, 7β- oder 12α-HSDH, insbesondere NADPH-abhängige 7β-HSDH oder 12α-HSDH, welche nicht durch 7α-HSDH Enzym oder-Enzymaktivität, insbesondere nicht durch NADPH-abhängige 7α-HSDH oder NADH-abhängige 7α-HSDH mit NADPHabhängiger Nebenaktivität, verunreinigt ist, erhältlich aus einem rekombinaten Mikroorganismus nach einer der Ausführungsformen 1 bis 7.
10. Rekombinante HSDH, wie z.B. 3α-, 3β-, 7β-, 11α-, 11β-, 12α-, 12β-, 17α-, 17β-, 20α-, oder 20β-HSDH, insbesondere 3α-, 7β- oder 12α-HSDH nach Ausführungsform 9, umfassend eine Aminosäuresequenz, ausgewählt unter SEQ ID NOs gemäß Ausführungsform 6 und davon abgeleiteten Aminosäuresequenzen mit einem Identitätsgrad von wenigstens 50% oder wenigstens 60 % oder wenigstens 75%, insbesondere wenigsten 85 %, wie z.B. 90, 91, 92, 93, 94, 95, 96, 97,98 oder 99%, zu einer dieser Sequenzen.
11. Verfahren zur selektiven enzymatischen Oxidation von Hydroxysteroiden welche neben einer Hydroxygruppe in wenigstens einer der Positionen 3α-, 3β-, 11α-, 11β-, 12α-, 12β-, 17α-, 17β-, 20α-, oder 20β- des Steroidgerüsts, insbesondere in 12α-Position des Steroidgerüsts wenigstens eine weitere Hydroxygruppe in 7α-Position des Steroidgerüsts aufweisen, wobei man das Hydroxysteroid in Gegenwart einer 3α-, 3β-, 11α-, 11β-, 12α-, 12β-, 17α-, 17β-, 20α-, oder 20β-HSDH, insbesondere einer 12α-HSDH nach Ausführungsform 9 oder 10 oder in Gegenwart eines rekombinanten Mikroorganismus nach einer der Ausführungsformen 1 bis 7 umsetzt, und wenigstens ein gebildetes Oxidationsprodukt aus dem Reaktionsansatz gegebenenfalls isoliert.
12. Verfahren nach Ausführungsform 11, wobei das Hydroxysteroid Cholsäure (CS) oder eine Cholsäurederivat, wie insbesondere ein Salz, Amid oder Alkylester, ist.
13. Verfahren nach Ausführungsform 12, wobei CS oder ein Derivat davon zu 12-Ketochenodesoxycholsäure (12-Keto-CDCS) oder zum entsprechenden Derivat umgesetzt wird.
14. Verfahren nach einer der Ausführungsformen 11 bis 13, wobei die Reaktion in Gegenwart von NAD(P)⁺ erfolgt.
15. Verfahren nach einer der Ausführungsformen 14, wobei die verbrauchtes NAD(P)⁺ elektrochemisch oder enzymatisch regeneriert werden.
16. Verfahren zur Herstellung von Ursodesoxycholsäure (UDCS) der Formel (1) 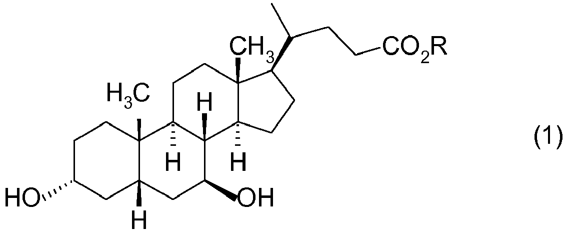 worin
   R für Alkyl, NR¹R², H, ein Alkalimetallion oder N(R³)₄⁺steht, worin die Reste R³ gleich oder verschieden sind und für H oder Alkyl stehen,
   wobei man
   a) eine Cholsäure (CS) der Formel (2) 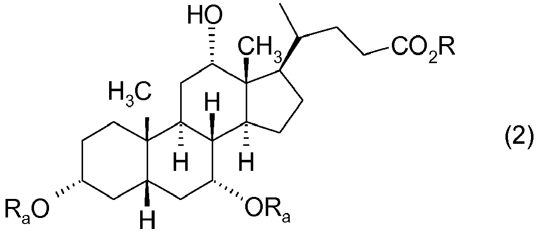 worin R die oben angegebenen Bedeutungen besitzen, und die Reste Rₐ gleich oder verschieden sind und für H oder Acyl stehen, in Gegenwart einer 12α-HSDH nach Ausführungsform 9 oder 10 oder in Gegenwart eines rekombinanten Mikroorganismus nach einer der Ausführungsformen 1 bis 7 zur korrespondierenden 12-Ketochenodesoxycholsäure (12-Keto CDCS) der Formel (3) 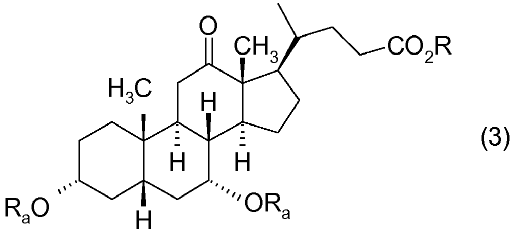 worin R und Rₐ die oben angebebenen Bedeutungen besitzen, oxidiert und anschließend
   b) 12-Keto-CDCS der Formel (3) durch Desoxygenierung zu Chenodesoxycholsäure (CDCS) der Formel (4) 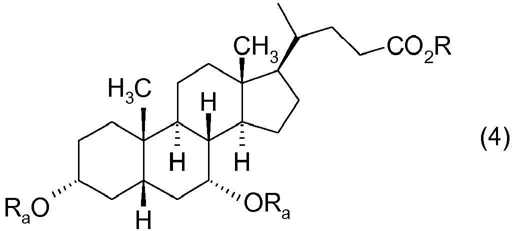 worin R und Rₐ die oben angebebenen Bedeutungen besitzen, umsetzt und
   c) CDCS der Formel (4) in Position 7 chemisch oxidiert zur 7-Keto-Lithocholsäure (KLCS) der Formel (5) 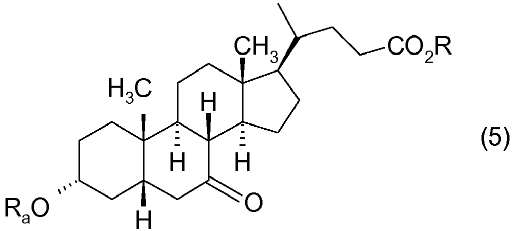 worin R und Rₐ die oben angebebenen Bedeutungen besitzen; und
   d) KLCS der Formel (5) reduziert und
   e) das Reaktionsprodukt gegebenenfalls weiter aufreinigt.
17. Verfahren nach Ausführungsform 16, wobei wenn Rₐ für Acyl steht, diese Acylgruppe nach Durchführung der Reaktionsstufe b) oder d) gegebenenfalls abspaltet.
18. Verfahren nach Ausführungsform 16 oder 17, wobei Stufe a) in Gegenwart von NAD(P)⁺ erfolgt.
19. Verfahren nach Ausführungsform 18, wobei verbrauchtes NAD(P)⁺ elektrochemisch oder enzymatisch regeneriert werden.
20. Verfahren zur selektiven enzymatischen Reduktion von Ketosteroiden, welche neben wenigstens einer Ketogruppe in Position 3, 11, 12, 17, oder 20 des Steroidgerüsts, insbesondere in Position 12 und /oder Position 3 des Steroidgerüsts wenigstens eine weitere Ketogruppe in Position 7 des Steroidgerüsts aufweisen, wobei man das Ketosteroid in Gegenwart einer 3α-, 3β-, 7β-, 11α-, 11β-, 12α-, 12β-, 17α-, 17β-, 20α-, oder 20β-HSDH, insbesondere einer 7β-HSDH und/oder einer 3α-HSDH und/oder einer 12α-HSDH nach Ausführungsform 9 oder 10, oder in Gegenwart eines rekombinanten Mikroorganismus nach einer der Ausführungsformen 1 bis 7 umsetzt, und wenigstens ein gebildetes Reduktionsprodukt aus dem Reaktionsansatz gegebenenfalls isoliert.
21. Verfahren zur Herstellung von Ursodesoxycholsäure (UDCS) der Formel (1) 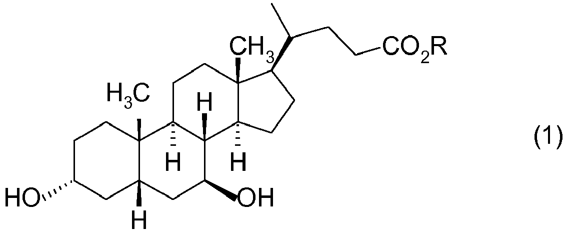 worin
   R für Alkyl, NR¹R², H, ein Alkalimetallion oder N(R³)₄⁺steht, worin die Reste R³ gleich oder verschieden sind und für H oder Alkyl stehen, wobei man
   a) gegebenenfalls eine Cholsäure (CS) der Formel (2) 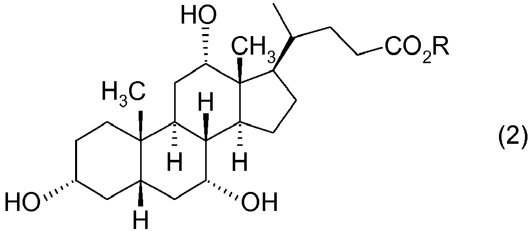 worin R die oben angegebenen Bedeutungen besitzt, zur Dehydrocholsäure (DHCS) der Formel (3) 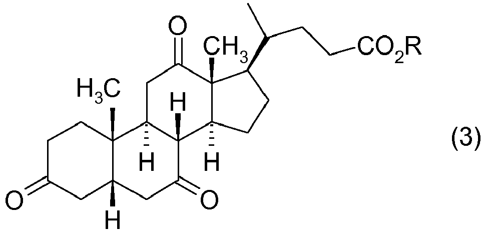 worin R die oben angegebenen Bedeutungen besitzt, chemisch oxidiert;
   b) DHCS mit einer 7β-HSDH und einer 3α-HSDH nach einer der Ausführungsformen 9 oder 10 in beliebiger Reihenfolge oder in gleichzeitiger Gegenwart beider Enzyme zur korrespondierenden 12-Keto-ursodesoxychiolsäure (12-Keto UDCS) der Formel (5) 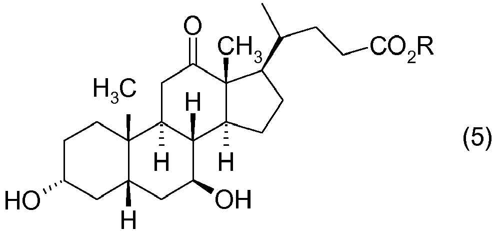 worin R die oben angegebenen Bedeutungen besitzt, reduziert und anschließend
   d) 12-Keto-UDCS der Formel (5) chemisch zu UDCS reduziert; und
   e) das Reaktionsprodukt gegebenenfalls weiter aufreinigt.
22. Verfahren nach einer der Ausführungsformen 11 bis 22, wobei der (die) enzymatische(n) Redox-Schritt(e) mit einem (insbesondere enzymatischen) Cofaktorregenerierungsschritt gekoppelt ist (sind).
23. Verfahren nach einer der Ausführungsformen 11 bis 22, wobei die HSDHs in gelöster, dispergierter oder immobilisierter Form eingesetzt wird; oder wobei das Verfahren in Gegenwart ganzen, gegebenenfalls immobilisierter Zellen eines rekombinanten Mikroorganismus nach einer der Ausführungsformen 1 bis 7 durchgeführt wird.

### Weitere Ausgestaltungen der Erfindung

### 1. Allgemeine Definitionen und verwendete Abkürzungen

Die hierin verwendeten Positionsangaben betreffend Substituenten des Steroidgerüsts erfolgen in Übereinsstimmung mit folgender Nomenklatur:

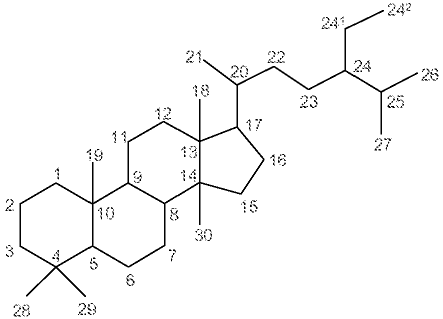

In folgender Tabelle sind die Strukturformeln, deren chemischen Namen und die verwendeten Abkürzungen tabellarisch zusammengefasst:

| Formel | Abkürzung | Chemischer Name |
|---|---|---|
| 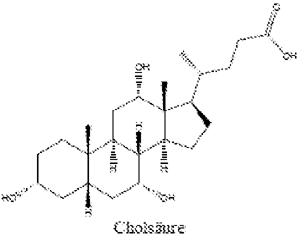 | CS | Cholsäure |
| 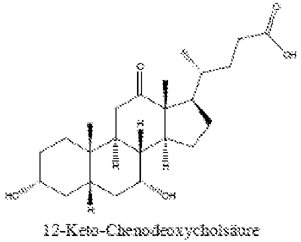 | 12-Keto-CDCS | 12-Keto-Chenadeoxycholsäure |
| 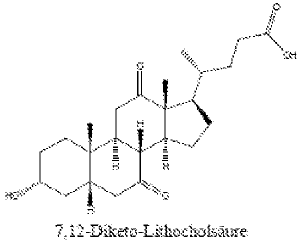 | 7,12-Diketo-LCS | 7,12-Diketo-Lithocholsäure |
| 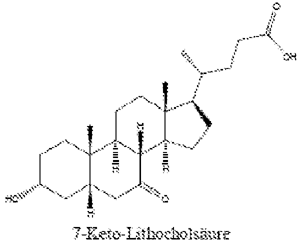 | 7-Keto-LCS (KLCS) | 7-Keto-Lithocholsäure |
| 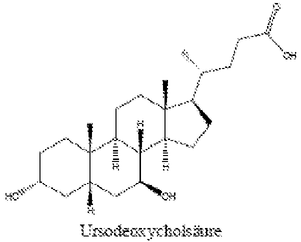 | UDCS | Ursodeoxycholsäure |

| Formel | Abkürzung | Chemischer Name |
|---|---|---|
| 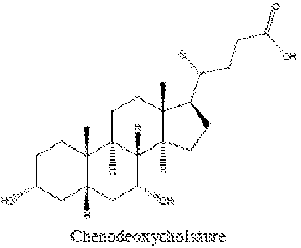 | CDCS | Chenodeoxycholsäure |
| 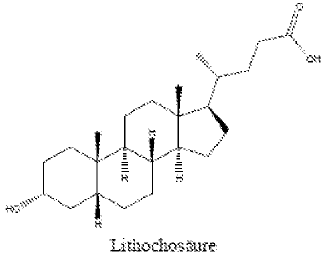 | LCS | Lithocholsäure |

Unter "Knockout"-Mutanten (oder Knockout-Stämmen) eines Organismus für ein bestimmtes Gen (wie z.B. 7α-HSDH-Gen) werden im Rahmen der vorliegenden Erfindung Mutanten des Organismus verstanden, bei denen das betreffende Gen auf DNA-Ebene "inaktiviert" ist. Eine derartige Inaktivierung ist im weitesten Sinn zu verstehen und kann beispielsweise beruhen auf einer vollständigen Deletion der Nukleinsäuresequenz des zugehörigen Strukturgens, so dass kein Protein translatiert wird, oder deren partieller Deletion, so dass damit ein unvollständiges Protein kodiert und translatiert wird, dessen Funktion im Vergleich zum Protein, das vom ursprünglichen Strukturgen kodiert wird, eingeschränkt ist, insbesondere vollständig unterbunden ist. Weiterhin kann das Strukturgen ausgeknockt werden durch dem Fachmann bekannte, sogenannte Nonsense-Mutationen, bei denen die Nukleinsäuresequenz des Strukturgens dahingehend verändert wird, dass ein oder mehrere vorzeitige Stoppkodons im Leserahmen auftreten und wesentliche Teile des Proteins nicht translatiert werden. Alternativ können sogenannte Missense-Mutationen eingeführt werden, bei denen einzelne oder mehrere, für die Funktion wesentliche Aminosäuren (z.B. Aminosäuren, die bei Enzymen für die Substratbindung, das katalytische Zentrum oder allosterische Regionen verantwortlich sind, oder Aminosäuren, die für die Bindung oder Wechselwirkung mit anderen Proteinen oder Molekülen verantwortlich sind) entfernt oder durch Aminosäure ersetzt werden, welche zu einem teilweisen oder vollständigen Verlust der betreffenden Funktion führen. Weiterhin kann die Insertion einer oder mehrerer Nukleotide vorgenommen werden, die zu einer Verschiebung des Leserahmens (Rasterverschiebung, Frameshift) und somit zur Expression eines Proteins mit eingeschränkter Funktion, und insbesondere bei Auftreten der Rasterverschiebung nahe am N-Terminus zu einem vollständigen Funktionsverlust führen. Auch die Insertion von Nukleinsäuresequenzen, die für eigene Proteine (Markerproteine) kodieren (beispielsweise Proteine, die zu AntibiotikaResistenzen führen), in das Strukturgen des Proteins, das ausgeknockt werden soll, kann herangezogen werden und bei Expression der insertierten Nukleinsäuresequenz als phänotypischer Nachweis für ein erfolgreiches Ausknocken verwendet werden. Knockout-Mutanten können weiterhin erzeugt werden durch die vorgenannten Maßnahmen der vollständigen oder partiellen Deletion oder Insertion von Nukleinsäuren in regulatorischen Bereichen, die für die Expression des betreffenden Strukturgens erforderlich sind, beispielsweise die Promotoren oder Enhancer des Strukturgens. Insbesondere erfolgt das Ausknocken jedoch durch partielle oder vollständige Deletion des Strukturgens, durch Insertion einer transkribierbaren Nukleinsäuresequenz für ein Markerprotein in die Nukleinsäuresequenz des auszuknockenden Strukturgens, oder eine Kombination beider Verfahren. Liegen mehrere Kopien des betreffenden Gens oder Homologe davon, welche die Funktion des betreffenden Gens erfüllen können, in einem Organismus oder einer Zelle vor, beispielsweise mehrere Gene auf einem Bakterienchromosom, auf einem Bakterienchromosom und gleichzeitig auf extrachromosomalen Elementen wie Plasmiden innerhalb einer prokarytischen Zelle, oder an unterschiedlichen Stellen eines eukaryotischen Chromosoms oder auf verschiedenen eukaryotischen Chromosomen, gegebenenfalls zusätzlich auf extrachrosomaler DNA in einer eukaryotischen Zelle, so können mehrere, vorzugsweise alle der Kopien oder Homologen des betreffenden Gens ausgeknockt werden.

Unter "endogen" werden genetische Informationen, wie beispielsweise Gene, verstanden, die bereits im Wildtypgenom (wie hierin definiert) enthalten sind.

Unter "exogen" werden genetische Informationen, wie beispielsweise Gene, verstanden, die im Wildtypgenom nicht enthalten sind. Werden exogene genetische Informationen, beispielsweise die erfindungsgemäßen 12α-HSDH -enthaltenden Expressionseinheiten, in das Genom eines Wildtypstammes eingebracht und dadurch ein genetisch veränderter Stamm erzeugt, so sind diese genetischen Informationen im Vergleich des erstmals erzeugten genetischen Stammes mit seinen Nachkommen endogen, dagegen exogen im Vergleich mit dem ursprünglichen Wildtypstamm, der diese genetischen Informationen nicht enthielt.

Unter dem Begriff "Wildtyp" wird erfindungsgemäß der entsprechende Ausgangsmikroorganismus verstanden und muss nicht zwingend einem natürlich vorkommenden Organismus entsprechen.

Je nach Zusammenhang kann unter dem Begriff "Mikroorganismus" der Ausgangsmikroorganismus (Wildtyp) oder ein erfindungsgemäßer, genetisch veränderter Mikroorganismus oder beides verstanden werden.

"Exprimierbar" enthalten in einem Organismus ist eine kodierende Sequenz, wenn sie entweder permanent oder zeitlich begrenzt (z.B. nach Induktion) in das entsprechenden Proteinprodukt translatiert wird.

"Inhibition" im Sinne der Erfindung umfasst eine Teilweise oder vollständige Inhibition einer Biologischen Funktion. Sie liegt erfindungsgemäß insbesondere dann vor, wenn eine aus bestimmten Gründen nicht länger erwünschte biologische Funktion, wie z.B. Enzymaktivität, analytisch nicht mehr nachweisbar ist, wie z.B. die enzymatische Aktivität der 7α-HSDH.

Werden keine anderen Angaben gemacht, so bezeichnet der Begriff "7α-HSDH" ein Dehydrogenase-Enzym, welches wenigstens die stereospezifische und/oder regiospezifische Oxidation von 12-Keto-CDCS zu 7,12-Diketo-LCS unter stöchiometrischem Verbrauch von NAD(P), insbesondere NAD katalysiert. Derartige Enzyme werden auch unter der EC Nummer 1.1.1.159 zusammengefasst. 7α-HSDHs sind insbesondere in Organismen der Darmflora, wie beispielsweise Clostridien, wie z.B. Clostridium absonum, Clostridium sordellii (Journal of Bacteriology, 1994, 4865-4874), in Escherichia coli (Journal of Bacteriology 1991, 2173-2179), in Bacteroides fragilis (Current Microbiology, 2003, 47, 475-484), sowie in Brucella, Eubacterium zu finden. Die Erfindung ist somit grundsätzlich auf all diese Mikroorganismen anwendbar.

Werden keine anderen Angaben gemacht, so bezeichnet der Begriff "12α-HSDH" ein Dehydrogenase-Enzym, welches wenigstens die stereospezifische und/oder regiospezifische Oxidation von Cholsäure (CS) zu 12-Keto-Chenodesoxycholsäure (12-Keto-CDCS) unter stöchiometrischem Verbrauch von NAD⁺ bzw. NADP⁺ katalysiert. Derartige Enzyme werden auch unter der EC Nummer 1.1.1.176 zusammengefasst. Es existieren sowohl NADP⁺-abhängige (Harris and Hylemon (1978) Biochim Biophys Acta 528(1): 148-57), als auch NAD⁺-abhängige Vertreter (Macdonald et al. 1976) Biochim Biophys Acta 450(2): 142-53. Der einzige bekannte Mikroorganismus, der eine hohe 12α-HSDH-Aktivität in Abwesenheit anderer HSDH exprimiert, stellt *Clostridium sp.* group P strain 48-50 DSM 4029 dar. Besonders geeignete 12α-HSDHs und Mutanten/Varianten davon, sind insbesondere in der PCT/EP2009/002190 der vorliegenden Anmelderin beschrieben, worauf ausdrücklich Bezug genommen wird.

Werden keine anderen Angaben gemacht, so bezeichnet der Begriff "7β-HSDH" ein Dehydrogenaseenzym, welches wenigstens die stereospezifische und/oder regiospezifische Reduktion von DHCS zu 3,12-Diketo-7β-CS insbesondere unter stöchiometrischem Verbrauch von NADPH, und gegebenenfalls die entsprechende Umkehrreaktion katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen. Geeignete Nachweisverfahren sind z.B. im folgenden experimentellen Teil beschrieben oder aus der Literatur bekannt (z.B. *Characterization of NADP-Dependent 7 β-Hydroxysteroid Dehydrogenases from Peptostreptococcus productus and Eubacterium aerofaciens.* S Hirano and N Masuda. Appl Environ Microbiol. 1982; wobei jedoch dort keine 7β-HSDH aus *Eubacterium* aerofaciens nachgewiesen werden konnte, welche die Reduktion von 7-Ketogruppen ermöglicht). Enzyme dieser Aktivität sind unter der EC Nummer 1.1.1.201 klassifiziert.

Werden keine anderen Angaben gemacht, so bezeichnet der Begriff "3α-HSDH" ein Dehydrogenaseenzym, welches wenigstens die stereospezifische und/oder regiospezifische Reduktion von 13,2-Diketo-7β-CS zu 12-Keto-UDCS, insbesondere unter stöchiometrischem Verbrauch von NADH und/oder NADPH, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus Comanomonas testosteroni (z.B. ATCC11996) erhältlich. Eine NADPH abhängige 3α-HSDH ist z.B. aus der Nagern bekannt und ebenfalls einsetzbar. (Cloning and sequencing of the cDNA for rat liver 3 alpha-hydroxysteroid/dihydrodiol dehydrogenase, J E Pawlowski, M Huizinga and T M Penning, May 15, 1991 The Journal of Biological Chemistry, 266, 8820-8825). Enzyme dieser Aktivität sind unter der EC Nummer 1.1.1.50 klassifiziert.

Unter einer "Reinform" oder einem "reinen" oder "im wesentlichen reinen" Enzym versteht man erfindungsgemäß ein Enzym mit einem Reinheitsgrad von mehr als 80, vorzugsweise mehr als 90, insbesondere mehr als 95, und vor allem mehr als 99 Gew.-%, bezogen auf den Gesamtproteingehalt, bestimmt mit Hilfe üblicher Proteinnachweismethoden, wie z.B. der Biuret-Methode oder dem Proteinnachweis nach Lowry et al.(vgl Beschreibung in R.K. Scopes, Protein Purification, Springer Verlag, New York, Heidelberg, Berlin (1982)).

Unter einem "Redoxäquivalent" versteht man eine als Elektronendonor bzw. Elektronenakzeptor brauchbare, niedermolekulare organische Verbindung, wie beispielsweise Nikotinamidderivate wie NAD⁺ und NADH⁺ bzw. deren reduzierte Formen NADH bzw. NADPH. NAD(P)⁺ steht dabei für NAD⁺ und/oder NADP⁺ und NAD(P)H steht dabei für NADH und/oder NADPH. Diese werden auch als "Cofaktoren" bezeichnet.

Unter einer "Cholsäure-Verbindung" versteht man erfindungsgemäß Verbindungen mit dem Kohlenstoffgrundgerüst, insbesondere der Steroidstruktur der Cholsäure und dem Vorhandensein von Keto- und/oder Hydroxy- oder Acyloxy-Gruppen in der Ringposition 7 und gegebenenfalls den Ringpositionen 3 und/oder 12.

Unter einer Verbindung eines speziellen Typs, wie z.B. einer "Cholsäure-Verbindung" oder einer "Ursodesoxycholsäure-Verbindung" versteht man insbesondere auch Derivate der zugrunde liegenden Ausgangsverbindung (wie z.B. Cholsäure oder Ursodesoxycholsäure).

Derartige Derivate umfassen "Salze", wie z.B. Alkalimetallsalze wie Lithium-, Natrium- und Kaliumsalze der Verbindungen; sowie Ammoniumsalze, wobei man unter einem Ammoniumsalz das NH₄⁺-Salz bzw. solche Ammoniumsalze umfasst, worin wenigstens ein Wasserstoffatom durch einen C₁-C₆-Alkylrest ersetzt sein kann. Typische Alkylreste sind insbesondere C₁-C₄-Alkylreste, wie Methyl, Ethyl, n- oder i-Propyl-, n-, sec- oder tert-Butyl, sowie n-Pentyl und n-Hexyl und die ein- oder mehrfach verzweigten Analoga davon

"Alkylester" erfindungsgemäßer Verbindungen sind insbesondere Niedrigalkyl-Ester, wie z.B. C₁-C₆-Alkylester. Als nicht limitierende Beispiele sind zu nennen Methyl-, Ethyl-, n- oder i-Propyl-, n-, sec- oder tert-Butylester, oder längerkettige Ester, wie z.B. n-Pentyl- und n-Hexylester sowie die ein- oder mehrfach verzweigten Analoga davon.

"Amide" sind insbesondere Umsetzungsprodukte erfindungsgemäßer Säuren mit Ammoniak oder primären oder sekundären Monoaminen. Derartige Amine sind beispielsweise Mono- oder Di-C₁-C₆-Alkyl-monoamine, wobei die Alkylreste unabhängig voneinander gegebenenfalls weiter substituiert sein können, wie z.B. durch Carboxy-, Hydroxy-, Halogen (wie F, Cl, Br, J)-, Nitro- und Sulfonatgruppen.

Erfindungsgemäße "Acylgruppen" sind insbesondere nichtaromatische Gruppen mit 2 bis 4 Kohlenstoffatomen, wie z.B. Acetyl, Propionyl und Butyryl, sowie aromatische Gruppen mit gegebenenfalls substituiertem einkernigen aromatischem Ring, wobei geeignete Substituenten z.B. ausgewählt sind unter Hydroxy-, Halogen (wie F, Cl, Br, J)-, Nitro- und C₁-C₆-Alkylgruppen, wie z.B. Benzoyl oder Toluoyl.

Die erfindungsgemäß eingesetzten bzw. hergestellten Hydroxysteroidverbindungen, wie z.B. Cholsäure, Ursodesoxycholsäure, 12-Keto-Chenodesoxycholsäure, Chenodesoxycholsäure und 7-Keto-Lithocholsäure können in stereoisomerenreiner Reinform oder im Gemisch mit anderen Stereoisomeren im erfindungsgemäßen Verfahren eingesetzt oder daraus erhalten werden. Vorzugsweise werden jedoch die eingesetzten bzw. die hergestellten Verbindungen in im Wesentlichen stereoisomerenreiner Form eingesetzt bzw. isoliert.

Unter einer "Immobilisierung" versteht man erfindungsgemäß die kovalente oder nicht-kovalente Bindung eines erfindungsgemäß verwendeten Biokatalysators, wie z.B. einer 7β-HSDH an einem festen, d.h. in dem umgebenden flüssigen Medium im Wesentlichen unlöslichen, Trägermaterial.

### 2. Proteine, insbesondere erfindungsgemäß herstellbare rekombinante Enzyme

### 2.1 12α-HSDHs

Derartige 12α-HSDHs sind insbesondere beschrieben in der WO 2009/118176 der Anmelderin.

Erfindungsgemäß exprimierte 12α-HSDHs sind insbesondere erhältlich aus *Clostridium* sp. und weisen ein Molekulargewicht, bestimmt durch SDS-Polyacrylamid-Gelelektrophorese (SDS-PAGE) unter reduzierenden Bedingungen im Bereich von mehr als etwa 26 kD, insbesondere mehr als etwa 26, 5, wie etwa 27 bis 30 kD auf. Sie besitzen insbesondere ein berechnetes Molekulargewicht von mehr als etwa 29 kD, insbesondere etwa 29,1 bis 29, 5 kD, wie insbesondere 29,359 kD für 12α-HSDH Langversion bzw. etwa 27,8 für 12α-HSDH-Kurzversion. Die Molekulargewichtangaben beziehen sich dabei auf das Molekulargewicht der Proteinuntereinheiten des Enzyms; ohne darauf beschränkt zu sein, besteht das native Protein z.B. aus 4, insbesondere in etwa gleich großen, solchen Untereinheiten.

Insbesondere ist ein derartiges Protein erhältlich aus *Clostridium* sp. group P strain 48-50 (DSM4029). Das Enzym kann z.B. in einer spezifischen Aktivität im Bereich von mehr als etwa 10, 15, 20 oder 25 U/mg, wie z.B. 20 bis 100 U/mg oder 25 bis 80 U/mg bereitgestellt werden. Die Bestimmung der spezifischen Aktivität erfolgt dabei unter den im experimentellen Teil angegebenen Standardbedingungen.

Erfindungsgemäß verwendet werden insbesondere 12α-HSDHs, umfassend wenigstens eines der folgenden Aminosäure-Sequenzmotive:
a) LINN
b) RMGIFD
c) N-terminale Sequenz, ausgewählt unter
   (1) MDFIDFKEMGRMGIFDGKVAIITGGGKAKSIGYGIAVAYAK
   (2) MDFIDFKEMGRMGI
   (3) ITGGGKAKSIGYGIA
   (4) IFDGK
   (5) GIFDGK
d) FGDPELDI oder davon abgeleitete Sequenzen, wie z.B.: GDPELDI, FGDPELD, DPELDI, FGDPEL, GDPEL, DPELD, GDPELD

Weiterhin sind die erfindungsgemäß verwendeten 12α-HSDH Enzyme dadurch gekennzeichnet, dass sie kein N-terminales, (d.h. im Bereich des N-terminalen Endes von etwa 1 bis 30 Aminosäureresten) Sequenzmotiv des Typs TGX₃GXG, worin X für beliebige Aminosäurereste steht, aufweisen.

Verwendet werden insbesondere auch 12α-HSDHs,
a) umfassend eine der Aminosäuresequenzen gemäß SEQ ID NO: 12, 14 oder 16, jeweils beginnend bei Position +1 oder +2; oder
b) umfassend eine von einer Sequenz gemäß a) abgeleiteten Aminosäuresequenz mit einer prozentualen Sequenz-Identität von wenigsten 60 %; oder
c) kodiert von einer ein Protein gemäß a) und b) kodierenden Nukleinsäuresequenz; oder
d) kodiert von einer kodierenden Nukleinsäuresequenz gemäß SEQ ID NO: 11, 13 oder 15; oder von einer davon abgeleiteten, an die jeweilige Codon-Nutzung eines zur Expression verwendeten Organismus angepassten Sequenz; oder
e) kodiert von einer der Nukleinsäuresequenzen gemäß SEQ ID NO: 11, 13 oder 15; abgeleiteten kodierenden Sequenz mit einer prozentualen Sequenz-Identität von wenigstens 60 %.

Die Anpassung der Nukleinsäuresequenz an die Codon-Nutzung kann dabei nach üblichen Methoden erfolgen, wie z.B. unter zugänglich unter: http://slam.bs.jhmi.edu/cgi-bin/gd/gdRevTrans.cgi.

Anwendbar sind auch 12α-HSDH-Mutanten mit modifizierter Cosubstrat-Nutzung und/ oder verringerter Produktinhibition; und insbesondere solche Mutanten, abgeleitet von einer 12α-Hydroxysteroiddehydrogenase gemäß obiger Definition, mit wenigstens einer die Cosubstrat-Nutzung modifizierenden Mutation im Sequenzmotiv VLTGRNE.Nicht-limitierende Beispiele für solche Mutanten umfassen solche mit wenigstens eine der folgenden Aminosäuresubstitutionen in diesem Motiv: G →D ; R →A; sowie Mutanten mit wenigstens einer die Produktinhibition verringernden Mutation im Bereich der die Substratbindungstasche des Enzyms bildenden Aminosäurereste; wie z.B. umfassend wenigstens die Mutation von Aminosäure Q, entsprechend Position 98 bzw 100 von SEQ ID NO:16; insbesondere umfassend eine Mutation entsprechend Q98H in SEQ ID NO:16.

Weitere potentielle Aminosäuresubstituenten an Position 98 (bezogen auf SEQ ID NO: 16) umfassen: A,N,D,C,E,G,H,M,S,T,V. Basierend auf dem Homologiemodell der erfindungsgemäßen 12α-HSDH-HSDH wird angenommen, dass hier eine Substitution zu einer Schwächung der Carboxylbindung des Produkts führt. Daher wurde auch die benachbarte Position S100 (bezogen auf SEQ ID NO:16) zu folgenden Aminosäuren mutiert: A,N,D,C,Q,E,G,H,M,T,V,K.

Eine Gruppe erfindungsgemäßer 12α-HSDH-Mutanten umfasst somit eine oder zwei Mutationen in Position 98 bzw. 100 (gemäß SEQ ID NO:16) ausgewählt unter:
Q → A,N,D,C,E,G,H,M,S,T,V;
S → A,N,D,C,Q,E,G,H,M,T,V,K.

### 2.2 7β-HSDHs

Derartige 7β-HSDHs sind insbesondere beschrieben in der PCT/EP2010/068576 der Anmelderin, wie insbesondere:
7β-HSDHs erhältlich aus anaeroben Bakterium, insbesondere der Gattung *Collinsella,* wie dem Stamm *Collinsella aerofaciens* DSM 3979 (ATCC 25986), insbesondere umfassend eine Aminosäuresequenz gemäß SEQ ID NO:25 und davon abgeleitete funktionale Äquivalente.

Die aus *Collinsella aerofaciens* DSM 3979 erhältliche 7β-HSDH ist insbesondere durch wenigstens eine weitere der folgenden Eigenschaften, wie z.B. 2, 3, 4, 5, 6 oder 7 oder aller solcher Eigenschaften, gekennzeichnet:
a) Molekulargewicht (SDS-Gelelektrophorese): etwa 28-32 kDa, insbesondere etwa 29 bis 31 kDa oder etwa 30 kDa;
b) Molekulargewicht (Gelfiltration, unter nicht denaturierenden Bedingungen, wie insbesondere ohne SDS): etwa 53 bis 60 kDa, insbesondere etwa 55 bis 57 kDa, wie 56.1 kDa, womit sich das erfindungsgemäße Enzym deutlich von dem von Hirano et al (s.o.) beschriebenen 7β-HSDH Enzym aus *C*. *aerofaciens* ATCC25986 mit 45kDa unterscheidet. Dies belegt die dimere Beschaffenheit (Quartätstruktur) der 7β-HSDH aus *Collinsella aerofaciens* DSM 3979;
c) Stereoselektiven Reduktion der 7-Carbonyl-Gruppe von 7-Keto-LCS in eine 7β-Hydroxy-Gruppe;
d) pH Optimum für die Oxidation von UDCS im Bereich von pH 8,5 bis 10,5, insbesondere 9 bis 10;
e) pH Optimum für die Reduktion von DHCS und 7-Keto-LCS im Bereich von pH 3,5 bis 6,5, insbesondere bei pH 4 bis 6, wodurch überraschend die Möglichkeit gegeben ist oxidative (Merkmals d)) und reduktive Prozesse durch Wahl des pH-Wertes zu beeinflussen;
f) wenigstens einen kinetischen Parameter aus folgender Tabelle für wenigstens eines der dort genannten Substrate/Cofaktoren; im Bereich von ±20%, insbesondere ±10%, ±5%, ±3% ±2% oder ±1% um den in der folgenden Tabelle jeweils konkret genannten Wert.

| | K_{M} (µM) | Vₘₐₓ (U/mg Protein)^{b)} | k_{cat} (1 µmol/(µmol×min)) |
|---|---|---|---|
| NADPH^{+c)} | 5.32 | 30.58 | 944.95 |
| NADPH^{c)} | 4.50 | 33.44 | 1033.44 |
| UDCS | 6.23 | 38.17 | 1179.39 |
| 7-Keto-LCS | 5.20 | 30.77 | 950.77 |
| DHCS | 9.23 | 28.33 | 875.35 |
| NAD⁺ | -^{a)} | - | Spuren |
| NADH | - | - | Spuren |

| | | | |
|---|---|---|---|
| ^{a)} konnten aufgrund der sehr geringen Aktivität nicht bestimmt werden ^{b)} 1 U = 1 µmol/min ^{c)} womit sich das erfindungsgemäße Enzym deutlich von dem von Hirano et al (s.o.) beschriebenen 7β-HSDH Enzym aus C. aerofaciens ATCC25986 unterscheidet, wofür deutlich niedrigere K_{M} und Vₘₐₓ Werte (0,4 bzw 0,2) beschrieben wurde und keine Aktivität für NADPH gemessen wurde. | | | |

g) Phylogenetische Sequenzverwandtschaft der prokaryotischen 7β-HSDH aus *Collinsella aerofaciens* DSM 3979 mit der tierischen 11β-HSDH-Untergruppe, umfassend *Cavia porcellus, Homo sapiens* und *Mus musulus,* verwandt.

Beispielsweise zeigt eine erfindungsgemäße 7β-HSDH folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f).

Solche 7β-HSDHs katalysieren die stereospezifische Reduktion (Hydrierung) eines 7-Ketosteroids zum korrespondierenden 7β-Hydroxysteroid, und/oder die regiospezifische Hydrierung (Reduktion) eines Ketosteroids umfassend eine Ketogruppe in 7-Position und wenigstens eine weitere Ketogruppe am Steroidgerüst zum korrespondierenden 7β-Hydroxysteroid, wie insbesondere von Dehydrocholsäure (DHCS) in 7-Position zur korrespondierenden 3,12-Diketo-7β-Cholansäure, und sind z.B. NADPH abhängig.

Geeignete 7β-HSDHs besitzen eine Aminosäuresequenz gemäß SEQ ID NO:25 (Accession NO: ZP_01773061) oder einer davon abgeleiteten Sequenz mit einem Identitätsgrad von wenigstens 60%, wie z.B. wenigstens 65, 70, 75, 80, 85, oder 90, wie z.B. wenigstens 91, 92, 93, 94, 95, 96, 97, 98, 99 oder 99,5% zu dieser Sequenz; gegebenenfalls zusätzlich gekennzeichnet durch eine der folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f) gemäß obiger Definition.

### 2.3 Andere Proteine

In entsprechender Weise herstellbar unter Verwendung eines erfindungsgemäßen 7α-HSDHinhibierten Mikroorganismus sind z.B.:
eine 3α-HSDH, wie beispielsweise bekannt aus *Pseudomonas testosteroni* (J. Biol. Chem. 276 (13), 9961-9970 (2001)); (SEQ ID NO: 28, 29) oder *Rattus norvegicus* (SEQ ID NO: 30,31);
eine 3β-HSDH, wie beispielsweise bekannt aus Clostridium innocuum (Applied and Environmental Microbiology, June 1989, p. 1656-1659) oder aus Pseudomonas testosteroni;
eine 20β-HSDH, wie beispielsweise bekannt aus Organismen der Gruppe Streptomyces (The Journal of Biological Chemistry, 1977, Vol 252 No 1, Jan 10, 205-211);
eine 20α-HSDH, wie beispielsweise aus Clostridien, insbesondere aus Clostridium scindens (Journal of Bacteriology, June 1989, p. 2925-2932), und aus Tetrahymena pyriformis (Biochem.J. (1994) 297, 195-200) bekannt;
eine 17β-HSDH, wie bekannt aus Pilzen wie Cylindrocarpon radicola (J. Biochemistry 103, 1988, 1039-1044) und Cochliobolus lunatus (J. Steroid Biochem. Molec.Biol. Vol 59, 1996, No. 2, p. 205-214), aus Bakterien der Familie Streptomyces (Hoppe-Seyler's Z. Physiol. Chem, Bd. 356, 1975, 1843-1852), Pseudomonas (The Journal of Biological Chemistry, Vol. 252 No.II, June 10, 1977, p. 3775-3783) und Alcaligenes (The Journal of Biological Chemistry, Vol. 260, No. 25, Nov 5, 1985, p. 13648-13655);
eine 17α-HSDH, wie bekannt aus Eubacterium sp. (Journal of Lipid Research, Vol. 35, 1994, p. 922-929);
oder eine 11 β-HSDH, wie bekannt aus höheren Säugetieren.

### 2.4 Funktionale Äquivalente

Die vorliegende Erfindung ist nicht auf die konkret offenbarten Proteine bzw. Enzyme mit HSDH-Aktivität, wie z.B. 3α-, 3β-, 7β-, 11α-, 11β-, 12α-, 12β-, 17α-, 17β-, 20α-, oder 20β-HSDH, insbesondere 3α-, 7β- oder 12α-HSDHAktivität beschränkt, sondern erstreckt sich vielmehr auch auf funktionale Äquivalente davon.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. 7β- oder 12α-HSDH-Aktivität, besitzen.

So versteht man beispielsweise unter "funktionalen Äquivalenten" Enzyme, die im verwendeten Test auf HSDH-Aktivität, wie 7β- oder 12α-HSDH-Aktivität eine um mindestens 1%, wie z.B. mindestens 10% oder 20 %, wie z.B. mindestens 50 % oder 75% oder 90 % höhere oder niedrigere Aktivität eines Enzyms, umfassend eine hierin definierte Aminosäuresequenz aufweisen. Funktionale Äquivalente sind außerdem vorzugsweise zwischen pH 4 bis 11 stabil und besitzen vorteilhaft ein pH-Optimum in einem Bereich von pH 6 bis 10, wie insbesondere 8,5 bis 9,5, sowie ein Temperaturoptimum im Bereich von 15°C bis 80°C oder 20°C bis 70°C, wie z.B. etwa 45 bis 60°C oder etwa 50 bis 55°C.

Die HSDH-Aktivität, wie 7β- oder 12α-HSDH -Aktivität kann mit Hilfe verschiedener bekannter Tests nachgewiesen werden. Ohne darauf beschränkt zu sein, sei ein Test unter Verwendung eines Referenzsubstrates, wie z. B. Cholsäure, unter standardisierten Bedingungen wie im experimentellen Teil definiert, zu nennen.

Unter "funktionalen Äquivalenten" versteht man erfindungsgemäß insbesondere auch "Mutanten", welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, -Substitutionen, -Deletionen und/oder-Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| **Ursprünglicher Rest** | **Beispiele der Substitution** |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln; Glu |
| Met | Leu ; Ile |
| Phe | Met; Leu; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75% ins besondere wenigsten 85 %, wie z.B. 90, 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### 3. Nukleinsäuren und Konstrukte

### 3.1 Nukleinsäuren

Gegenstand der Erfindung sind auch Nukleinsäuresequenzen, die für ein Enzym mit HSDH-Aktivität, wie z.B. 3α-, 3β-, 7β-, 11α-, 11β-, 12α-, 12β-, 17α-, 17β-, 20α-, oder 20β-HSDH-Aktivität, insbesondere 3α-, 7β- oder 12α-HSDH-Aktivität, kodieren oder Konstrukte, die zur Herstellung erfindungsgemäßer Knockout-Mutanten verwendet werden.

Die vorliegende Erfindung betrifft auch Nukleinsäuren mit einem bestimmten Identitätsgrad zu den hierin konkret beschriebenen Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

| Multiple alignment parameters: | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |
| | |

| Pairwise alignment parameter: | |
|---|---|
| FAST algorithm | on |
| K-tuple size | 1 |
| Gap penalty | 3 |
| Window size | 5 |
| Number of best diagonals | 5 |

Alternativ dazu kann die Identität auch nach Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel-und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Gegenstand der Erfindung sind auch Derivate der konkret offenbarten oder ableitbaren Nukleinsäuresequenzen.

So können weitere erfindungsgemäße Nukleinsäuresequenzen z.B. von SEQ ID NO:1 bis 9, 11, 13 ,15, 17 bis 22, 24, 26, 27, 28, 30, und 33 bis 37 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der oben erwähnten erfindungsgemäßen Nukleinsäuresequenz sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Dem Fachmann sind darüber hinaus Verfahren zur Erzeugung funktionaler Mutanten bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der durch diese codierten Protein sind dem Fachmann seit langem geläufig, wie beispielsweise
- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcárel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der sogenannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen , wie z.B. 1 bis 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die erfindungsgemäßen Ergebnisse liefern wichtige Informationen in bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

Nicht limitierende Beispiele solcher hot spot-Regionen der erfindungsgemäßen HSDH sind im Folgenden zusammengefasst:
35-40, insbesondere 37-38, (jeweils bezogen auf die Aminosäuresequenz von HSDH_kurz (SEQ ID NO: 14).
90-105, 93 -100 oder 96-100, insbesondere 97 und/oder 98, (jeweils bezogen auf die Aminosäuresequenz von HSDH_kurz (SEQ ID NO: 14).

### 3.2 Konstrukte

Gegenstand der Erfindung sind außerdem Expressionskonstrukte, enthaltend unter der genetischen Kontrolle regulativer Nukleinsäuresequenzen eine für ein erfindungsgemäßes Polypeptid kodierende Nukleinsäuresequenz; sowie Vektoren, umfassend wenigstens eines dieser Expressionskonstrukte.

Unter einer "Expressionseinheit" wird erfindungsgemäß eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere Sequenz SEQ ID NO: 11, 13 oder 15 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, Ipp-, lac-, lpp-lac-, lacl^{q-,} T7-, T5-, T3-, gal-, trc-, ara-, rhaP (rhaP_{BAD})SP6-, lambda-P_{R}- oder im lambda-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, λgt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, pGHIac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### 4 Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der erfindungsgemäßen Vektoren sind rekombinante Knockout-Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit HSDH-Aktivität, wie z.B. 3α-, 3β-, 7β-, 11α-, 11β-, 12α-, 12β-, 17α-, 17β-, 20α-, oder 20β-HSDH-Aktivität, insbesondere 3α-, 7β- oder 12α-HSDH-Aktivität, gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

### 5. Enzymatisch/chemische Herstellung von UDCS

### 5.1 Oxidatives Verfahren

Diese ist beispielsweise beschrieben in der WO 2009/118176 der Anmelderin.

Zur medikamentösen Behandlung von Gallensteinleiden werden seit vielen Jahren u. a. die Wirkstoffe Ursodesoxycholsäure (UDCS) und das zugehörige Diastereomer Chenodesoxycholsäure (CDCS) eingesetzt. Beide Verbindungen unterscheiden sich lediglich durch die Konfiguration der Hydroxygruppe am C-Atom 7 (UDCS: β -Konfiguration, CDCS: α-Konfiguration). Zur Herstellung von kommerziellen Mengen UDCS wird bisher bevorzugt ein Verfahren verwendet, bei dem CDCS als Rohstoff eingesetzt wird. CDCS wiederum wird vorzugsweise aus Cholsäure (CS) hergestellt.

### 5.1.1 CDCS Herstellung

Als Alternative zum ausschließlich chemischen Verfahren wird erfindungsgemäß eine enzymkatalysierte Oxidation von CS zu 12-Ketochenodesoxycholsäure (12-Keto-CDCS, CAS 2458-08-4), die dann weiter zu CDCS umgesetzt wird, bereitgestellt. Dieser Syntheseweg beinhaltet nur zwei Schritte und ist damit im Vergleich zu der rein chemischen Route deutlich einfacher durchzuführen.

### 1. Schritt: enzymatische Oxidation

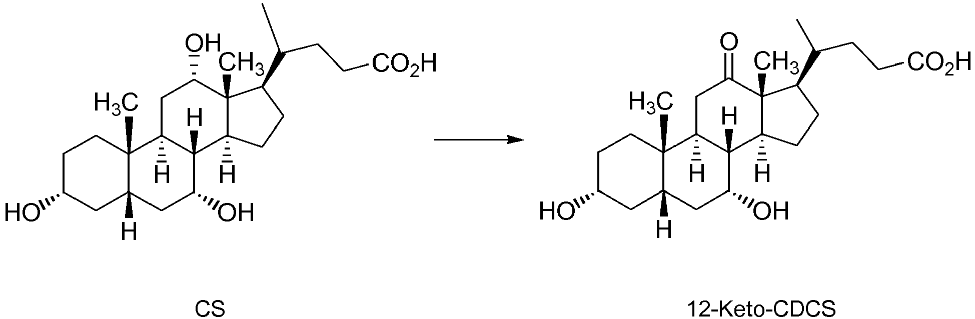

### 2. Schritt: Desoxygenierung

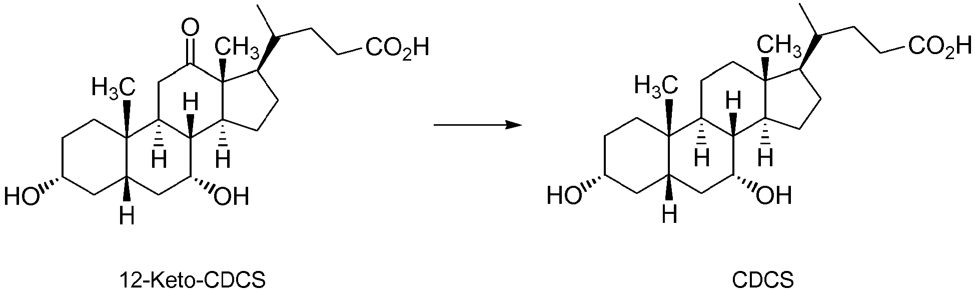

Gemäß Schritt 1 wird Cholsäure mittels 12α-HSDH zu 12-Ketochenodesoxycholsäure (12-Keto-CDCS) NADP⁺-abhängig oxidiert. Diese Reaktion ist reversibel. 12α-HSDHs gehören der Enzymklasse 1.1.1.176 an und werden hauptsächlich in Bakterien der Gattung *Clostridium* gefunden.

Die enzymatische Oxidation erfolgt erfindungsgemäß bevorzugt mittels einer erfindungsgemäßen 12α-HSDH (Lang- oder Kurzversion) und Kofaktorregenerierung mittels einer ADH, wie z.B. ADH ms oder ADH t.

Die Desoxygenierung gemäß Schritt 2 ist eine klassische chemische Wolff-Kishner-Reduktion und wird analog zur oben beschriebenen Desoxygenierung der CDCS III durchgeführt. Ein wesentlicher Vorteil dieser Route ist, dass durch die Selektivität des Enzyms die Verunreinigung Lithocholsäure nicht entsteht.

### 5.1.2 Umsetzung von CDCS zu UDCS

Als Rohstoff für UDCS (CAS 128-13-2) wird obiges CDCS verwendet. Im ersten Syntheseschritt wird die Hydroxylgruppe in Position 7 der CDCS zum entsprechenden Keton oxidiert. Es resultiert die 7-Ketolithocholsäure (3α-Hydroxy-7-ketocholansäure, kurz: KLCS, CAS 4651-67-6). Im zweiten Schritt folgt die stereoselektive Reduktion der Ketogruppe in Position 7. Ziel ist es, mit möglichst hoher Diastereoselektivität UDCS zu erhalten. In der Regel enthält die UDCS direkt nach der Reduktion noch einige Prozent des Diastereomers CDCS. Um zum Wirkstoff UDCS zu gelangen, muss UDCS roh in einem dritten Schritt gereinigt werden.

### 1. Schritt: Oxidation

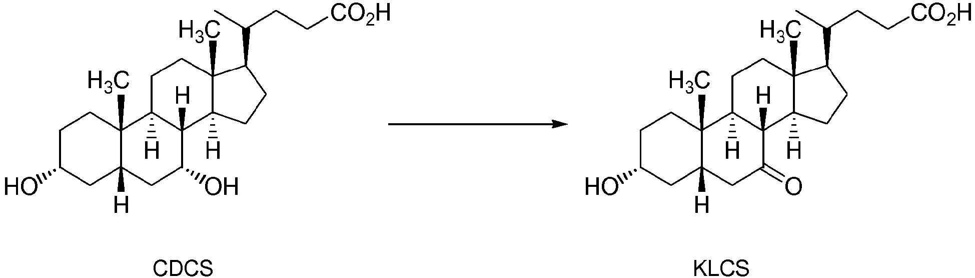

### 2. Schritt: Reduktion

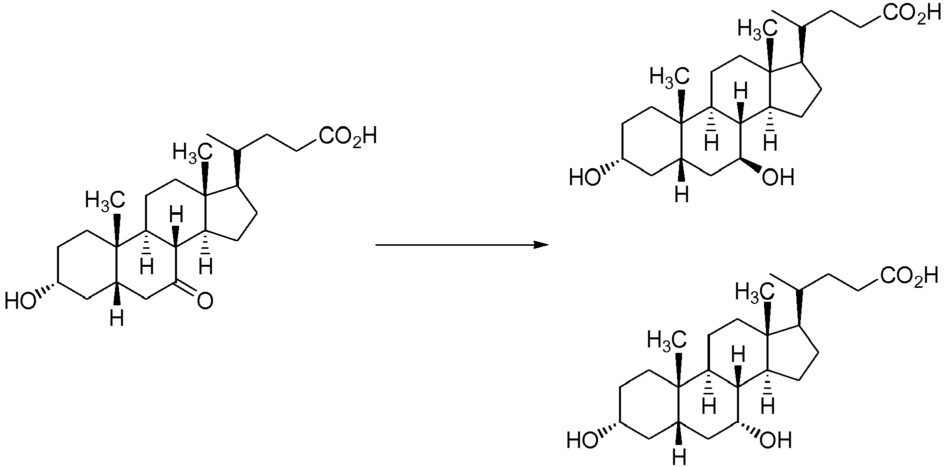

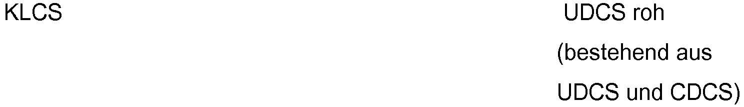

### 3. Schritt: Reinigung

### UDCS roh -> UDCS rein

Die Oxidation der CDCS erfolgt üblicherweise mit wässriger Natriumhypochloritlösung. In der Literatur findet sich noch die Chromsäureoxidation als Alternative. KLCS fällt als Feststoff an, der dann im zweiten Schritt weiter verarbeitet wird. Die Reduktion kann mit Natriummetall in Alkoholen durchgeführt werden. Es resultiert ein Rohprodukt mit einer Zusammensetzung von UDCS : CDCS von ca. 85 : 15. In alternativen Verfahren wird KLCS mit Wasserstoff an einem Nickelkatalysator (Raney-Nickel) in Alkoholen (wie z.B. aliphatischen Alkoholen) als Lösungsmittel zusammen mit einer Base, wie Kalium-t-butylat oder Kaliumhydroxid, reduziert (EP-A-0 230 085). Zusätzlich ist noch die Reduktion mit Kalium und Lithium (höhere Selektivität als Natrium, C. Giordano et al. Angew. Chem. 1985, 97, 510) sowie Zink (ES 489661) und elektrochemisch (US 4 547 271) möglich.

Bei der Aufreinigung von UDCS roh zu UDCS rein handelt es sich um eine Trennung diastereomerer Salze. Sie erfolgt durch Herstellung, Isolierung und nachfolgende Spaltung eines geeigneten Salzes von UDCS. In der Literatur werden folgende alternativen Reinigungsmethoden genannt: Herstellung, Umkristallisation und Spaltung eines korrespondierenden UDCS-Esters (EP-A-0386 538), Extraktionen (JP 60006699) und chromatographische Verfahren.(IT 2000MI1177).

### 5.2 Reduktives Verfahren

Ein solches ist beispielsweise beschrieben in der PCT/EP2010/068576 der Anmelderin,

### 5.2.1 Chemische Umsetzung von CS zu DHCS

Die Hydroxygruppen von CS werden mit Chromsäure bzw. Chromaten in saurer Lösung (z.B. H₂SO₄) zu Carbonylgruppe in an sich bekannter Weise auf klassisch-chemischem Weg oxidiert. Dadurch entsteht DHCS.

### 5.2.2 Enzymatische oder mikrobielle Umsetzung von DHCS zu 12-Keto-UDCS

In wässriger Lösung wird DHCS durch 3α-HSDH und 7β-HSDH bzw. Mutanten davon spezifisch zu 12-Keto-UDCS in Anwesenheit von NADPH bzw. NADH reduziert. Der Cofaktor NADPH bzw. NADH kann durch eine Alkoholdehydrogenase (ADH) bzw. Formiatdehydrogenase (FDH) bzw. Glucosedehydrogenase (GDH) bzw. Mutanten davon und in Gegenwart von Isopropanol bzw. Natium-formiat bzw. Glucose regeneriert werden. Die Reaktion läuft unter milder Bedingung. Beispielsweise kann die Reaktion bei pH = 6 bis 9, insbesondere etwa pH = 8 und bei etwa 10 bis 30, 15 bis 25 oder etwa 23°C durchgeführt werden. 3α-HSDH und 7β-HSDH können dabei schrittweise, d.h. in beliebiger Reihenfolge nacheinander, oder aber gleichzeitig, d.h. in Kombination eingesetzt werden.

Im Falle eines mikrobiellen Umsetzungsschrittes können rekombinante Mikroorganismen, welche die erforderliche(n) Enzymaktivität(en) exprimieren in Gegenwart des umzusetzenden Substrates (DHCS) anaerob oder aerob in geeigneten Flüssigmedien kultiviert werden. Geeignete Kultivierungsbedingungen sind dem Fachmann an sich bekannt. Sie umfassen Umsetzungen im pH bereich von beispielsweise 5 bis 10 oder 6 bis 9, bei Temperaturen im Bereich von 10 bis 60 oder 15 bis 45 oder 25 bis 40 oder 37 °C. Geeignete Medien umfassen z.B. LB und TB Medien. Die Umsetzungsdauer kann dabei z.B. batchweise oder kontinuierlich oder in sonstigen üblichen Verfahrensvarianten erfolgen (wie oben beschrieben). Die Unsetzungsdauer kann dabei z.B. im Bereich von Minuten bis mehreren Stunden oder tagen Liegen, und z.B. 1h bis 48 h betragen. Gegebenenfalls kann, wenn Enzymaktivität nicht kontinuierlich exprimiert wird, diese durch Zugabe eines geeigneten Induktors, nach Erreichen einer Zielzelldichte , z,B, von etwa OD₆₀₀ = 0,5 bis 1,0, eingeleitet werden.

### 5.2.3: Chemische Umsetzung von 12-Keto-UDCS zu UDCS

Die 12-Carbonylgruppe von 12-Keto-UDCS wird mittels Wolff-Kishner-Reduktion in an sich bekannter Weise entfernt, dadurch entsteht UDCS aus 12-Keto-UDCS. In der Reaktion wird zuerst die Carbonylgruppe mit Hydrazin zum Hydrazon umgesetzt. Anschließend wird das Hydrazon in Gegenwart einer Base (z.B. KOH) auf 200 °C erhitzt, hierbei wird Stickstoff abgespaltet und UDCS entsteht.

### 6. Rekombinante Herstellung von HSDHs

Die Erfindung umfasst auch die rekombinante Herstellung von HSDHs, wie z.B. 3α-, 3β-, 7β-, 11α-, 11β-, 12α-, 12β-, 17α-, 17β-, 20α-, oder 20β-HSDH, insbesondere 3α-, 7β- oder 12α-HSDHs oder funktioneller, biologisch aktiver Fragmente davon, wobei man einen HSDHproduzierenden Knockout-Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Knockout-Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C und. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Ankerfungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### 7. Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" " in K. Drauz und H. Waldmann, Enzyme Catalysis in Organic Synthesis 2002, Vol.III, 991-1032, Wiley-VCH, Weinheim beschrieben.

### 8. Herstellung von Knockout-Mutanten

Verfahren zum Ausknocken, beispielsweise Verfahren zum Deletieren oder Insertieren von Nukleinsäuren oder Nukleinsäuresequenzen, sind dem Fachmann bekannt und umfassen beispielsweise fachübliche Verfahren unter Verwendung von Oligonukleotidprimern und PCR sowie Verfahren unter Verwendung von Plasmiden und Restriktionsenzymen, mit deren Hilfe gewünschte modifizierte Nukleinsäuresequenzen in die Plasmide eingebaut werden können. Diese und weitere molekulargenetische Verfahren sind allgemein bekannt und beispielsweise beschrieben in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984), Ausubel, F.M. et al., Current Protocols in Molecular Biology, Greene Publishing Assoc. and Wiley Interscience (1987); "Gene Expression Technology", In: Methods in Enyzmology, Bd 185, Elsevier, 1990; "Bacterial Genetic Systems", in: Methods in Enyzmology, Bd. 204, Elsevier, 1991; "Recombinant DNA, Part E", in Methods in Enyzmology, Bd. 154, Elsevier, 1987; und C.D. Smolke (Hrsg.), The metabolic pathway engineering handbood: tools and applications, CRC Press. Boca Raton, 2010; C. Mühlhardt, "Der Experimentator: Molekularbiologie/Genomics", 6. Aufl., Spektrum Akademischer Verlag, Heidelberg 2010; D. Clark, N. Pazdernik, "Molekulare Biotechnologie", Spektrum Akademischer Verlag, Heidelberg, 2009; M. Jahnson (Hrsg.), "Gentechnische Methoden", 4. Aufl., Spektrum Akademischer Verlag, Heidelberg, 2006; J.M.S. Bartlett, D. Stirling (Hrsg.), "Methods in Molecular Biology", Bd. 226, "PCR Protocols", Springer Verlag GmbH, 2003

Ein weiteres bekanntes Verfahren zur Herstellung von Knockout-Mutanten basiert auf homologer Rekombination. Dabei wird insbesondere ein Markergen mit flankierenden Sequenzen versehen, die homolog sind zu Sequenzen des auszuknockenden Gens, und über geeignete Vehikel (z.B. Plasmide, Insertionssequenzen, Transposons) zusammengebracht mit der auszuknockenden Zielsequenz. Diese befindet sich vorzugsweise in einem Wirtsorganismus, beispielsweise im Chromosom (je nach Wirtsorganismus Bakterienchromosom oder eukaryotisches Chromosom) oder auf einem extrachromosomalen Nukleinsäurekonstrukt (z.B. einem Plasmid) im betreffenden Wirt. Zellfreie oder in-vitro-Systeme sind jedoch ebenfalls denkbar. Bei Vorhandensein geeigneter Rekombinationssysteme (z.B. Rekombinasen) erfolgt eine homologe Rekombination, wobei das Markergen aufgrund der Homologie der flankierenden Sequenzen in die Zielsequenz integriert wird und im Austausch der zwischen den flankierenden Sequenzen der Zielsequenz liegenden Nukleinsäurebereich entfernt wird. Das Genprodukt des auf diese Weise insertierten Markergens kann zur Selektion der Knockout-Mutanten verwendet werden. Das Markergen kodiert vorzugsweise für ein Protein, das eine Antibiotikaresistenz vermittelt, so dass eine Selektion über Antibiotika enthaltende Festmediumplatten oder Flüssigmedien erfolgen kann. Das Markergen kann jedoch auch für andere Proteine kodieren, beispielsweise floureszierende Proteine, z.B. "Green Fluorescent Protein" (GFP), so dass eine Selektion über Koloniebetrachtung im Fluoreszenzmikroskop oder über fluoreszenzaktivierte Zellsortierung (FACS) erfolgen kann. Das Prinzip der homologen Rekombination ist allgemein bekannt und ist, neben weiteren Formen der Rekombination, beispielsweise beschrieben R.Y. Stanier, J.L. Ingraham, M. L. Wheelis, P. R. Painter (Hrsg.) "General Microbiology", 5. Aufl., 1986, Macmillan Education Ltd., Houndmills, London, S. 278 - 285, weiterhin in "DNA Cloning, Volume II, D.M. Glover (Hrsg.), IRL Press, Oxford, Washington D.C., 1986, S. 57-65; Karl Drlica, "DNA und Genklonierung - ein Leitfaden", G. Fischer Verlag, Stuttgart, Jena, 1995, Kapitel "Transposition". S. 155-161, und D.H. Jones und S.C. Winistorfer, "Recombination and Site-Directed Mutagenesis using Recombination PCR", in: J.M.S. Bartlett, D. Stirling (Hrsg.), "Methods in Molecular Biology", Bd. 226, "PCR Protocols", Springer Verlag GmbH, 2003, Kapitel 70, S. 517-525; und A.S. Waldman (Hrsg.), "Methods in Molecular Biology", Bd. 262, Genetic Recombination - Reviews and Protocols", Springer Verlag GmbH, 2004; F.P. Miller, A.F. Vandome, J. McBrewster (Hrsg.), "Genetic Recombination", Alphascript Publishing, 2009; F.P. Miller, A.F. Vandome, J. McBrewster (Hrsg.), "Gene Targeting", Alphascript Publishing, 2010 A. Aguilera und R. Rothstein, Molecular Genetics of Recombination, Springer-Verlag GmbH, 2007. Spezielle Veröffentlichungen über Gen-Knockout-Verfahren sind, insbesondere für eukaroytische Zellen und Tiere "Gene Knockout Protocols", Ralf Kühn und Wolfgang Wurst (Hrsg.), Humana Press., 2. Auflage 2009, sowie Wolf S.E. und Woodside, K.J, Transgenic and gene knock-out techniques and burn research, J. Surg. Res. 123(2):328-339, 2005, beschrieben.

Desweiteren sind kommerzielle Kits zur Erzeugung von Gen-Knockouts erhältlich, z.B. das "TargeTron gene knockout system" von Sigma-Aldrich, insbesondere für E.coli und verwandte Bakterienarten.

Beispiele für weitere, zur Erzeugung von Knockout-Mutanten oder genetischen Modifikationen geeignete Systeme sind das Cre/Lox-System, unter anderem beschrieben in Sauer B und Henderson N, Proc. Natl. Acad. Sci. USA 85(14):5166-5170, 1988, oder das "Red/ET Recombination"-System (Gene Bridges GmbH, Heidelberg, Deutschland; beschrieben im Anwenderhandbuch "Red/ET Recombination - Cloning without Restrictions Enzymes", Gene Bridges GmbH, ferner unter anderem beschrieben in den Patenten US 6,355,412, US 6,509,156 und EP 4139561).

### 9. Cofaktor-Regenerierung

Der Bedarf an Cofaktor kann durch die Kopplung mit einem cofaktorregenerierenden Enzym verringert werden. Dazu können verschiedenen Methoden des Standes der Technik verwendet werden.

Der Stand der Technik lehrt beispielsweise die Verwendung von Glutamatdehydrogenase (GLDH) (Carrea, G., et al., Biotechnology and Bioengineering, 1984. 26(5): p. 560-563). Die GLDH reoxidiert NADPH zu NADP⁺ unter gleichzeitiger reduktiver Aminierung von α-Ketoglutarat zu Glutamat.

Alternativ dazu werden auch Alkoholdehydrogenasen ADH-Tb, ADH-Lb und ADH-ms zur Cofaktorregenerierung vorgeschlagen (Fossati, E., et al., Biotechnol Bioeng, 2006. 93(6): p. 1216-20). Die ADH wandelt Aceton in 2-Propanol unter Regenerierung von NADP⁺ um.

Weiter sekundäre Alkoholdehydrogenasen zur Regenerierung des NADH bzw. NAD, sind z.B. solche, die aus Hefen der Gattungen Candida und Pichia, wie z.B.: Carbonylreduktase aus Candida parapsilosis (CPCR) (US 5,523,223 und US 5,763,236; Enzyme Microb. Technol. 1993 Nov; 15(II):950-8), Pichia capsulata (DE 10327454.4), Pichia farinosa (A 1261/2005, KI. C12N), Pichiafinlandica (EP 1179595 A1), Candida nemodendra (A 1261/2005, KI. C12N), Pichia trehalophila (A 1261/2005, KI. C12N), Rhodotorula mucilaginosa (A 1261/2005, KI. C12N), Lodderomyces elongisporus (A 1261/2005, KI. C12N) und Pichia stipidis (A 1261/2005, KI. C12N) isolierbar sind. Des weiteren kann die Regenerierung des NADH auch mit sekundären Alkoholdehydrogenasen wie isoliert aus Bakterien der Klasse der Actinobacteria, z.B. aus Rhodococcus erythropolis (US 5,523,223), Norcardia fusca (Biosci. Biotechnol. Biochem., 63 (10) (1999), Seiten 1721-1729; Appl. Microbiol. Biotechnol. 2003 Sep; 62(4):380-6, Epub 2003 Apr 26), Rhodococcus ruber (J. Org. Chem. 2003 Jan 24; 8(2):402-6.) oder Microbacterium spec. (A 1261/2005, KI. C12N), erfolgen.

Geeignete sekundäre Alkoholdehydrogenasen / Oxidoreduktasen zur Regenerierung von NADPH bzw. NADP, sind beispielsweise solche, wie soliert aus Organismen der Ordnung Lactobacillales {Lactobacillus kefir (US 5,200,335), Lactobacillus brevis (DE 19610984 A1; Acta Crystallogr. D. Biol. Crystallogr. 2000 Dec; 56 Pt 12:1696-8), Lactobacillus minor (DE 10119274), Leuconostoc carnosum (A 1261/2005, KI. C12N) oder solche, wie beschrieben, aus Thermoanerobium brockii, Thermoanerobium ethanolicus oder Clostridium beijerinckii.

Eine Cofaktorregenerierung durch Lactatdehydrogenase (LDH; Umsetzung von Pyruvat zu Lactat unter Regenerierung von NAD⁺) wird in der EP-A-1 731 618 beschrieben.

Weitere geeignete Dehydrogenases zur Cofactorregenerierung sind Glucosedehydrogenasen (GDH), Formiatdehydrogenasen (FDH) welche insbesondere wenigstens die enzymatische Oxidation von Ameisensäure zu CO₂ katalysiert, Glucose-6-Phosphat-Dehydrogenase, oder Phosphit-Dehydrogenasen,

### EXPERIMENTELLER TEIL

Werden keine anderen Angaben gemacht, so können die im Rahmen der vorliegenden Erfindung durchgeführten Klonierungsschritte, wie z.B. PCR (Polymerase-Kettenreaktion), Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikroorganismen, Vermehrung von Phagen und Sequenzanalyse rekombinanter DNA wie bei Sambrook et al. (1989) a.a.O. beschrieben durchgeführt werden.

Die Knockout-Experimente durch homologe Rekombination erfolgen nach der Beschreibung von K. Datsenko und B. Wanner, (PNAS June 6, 2000 vol. 97 no. 12 6640-6645), siehe Figur 3.

### A. ALLGEMEINE ANGABEN

### 1. Materialien:

**LB-Medium:**

| | | |
|---|---|---|
| | Trypton | 10 g/l |
| | Hefeextrakt | 5 g/l |
| | NaCl | 5 g/l |
| | dH₂O | ad 1 Liter |
| Nur zur Herstellung von festen Nährböden | Agar | 15 g/l |

### 2. Methoden

### 2.1 Standardbedingungen für 12α-HSDH Aktivitätsbestimmung

Die Aktivität wird wie folgt definiert: 1 U des Enzyms entspricht der Enzymmenge, welche die Umsetzung von 1 µmol/min einer 5 mM Cholsäurelösung in Kaliumphosphat-Puffer (50 mM, pH 8,0) bei Raumtemperatur (d.h. ca. 20 °C-23 °C) katalysiert.

Die Reaktionsmischung enthält in einem Gesamtvolumen von 1 ml:

| | |
|---|---|
| 880 µl | 50 mM Kaliumphosphat Puffer, pH 8.0 |
| 10 µl | 100 mM Cholsäure (gelöst in Wasser, pH 8 eingestellt mit 2 M KOH) |
| 10 µl | Enzymlösung (in Puffer wie oben, im Bereich von 1 zu 10 U/ml) |
| 100 µl | 1 mM NADP+ (in Puffer wie oben) (damit Start der Reaktion) |

Die Zunahme der Absorption bei 340 nm wird gemessen und die Aktivität wird als Enzym-Unit berechnet, (U, i.e. µmol/min) mit der Molar-Extinction-Coefficient von 6.22 mM⁻¹×cm⁻¹.

### 2.2 Standardbedingungen für 7β-HSDH Aktivitätsbestimmung

Die Reaktionsmischung enthält ein Gesamtvolumen von 1 ml:

| | |
|---|---|
| 880 µl | 50 mM Kaliumphosphat Puffer, pH 8.0 |
| 10 µl | 10 mM UDCS (gelöst in Wasser, pH 8) |
| 10 µl | Enzymlösung (in Puffer wie oben, im Bereich von 1 zu 10 U/ml) |
| 100 µl | 1 mM NADP+ (in Puffer wie oben) |

Die Zunahme der Extinktion bei 340 nm wird gemessen und die Aktivität wird als Enzym-Unit (U, i.e. µmol/min) unter Verwendung des molaren Extinktionskoeffizienten von 6.22 mM⁻¹×cm⁻¹, berechnet,

### 2.3 HPLC Methode zur Analyse von Umsetzungsprodukten der Cholsäure.

Säule: Purospher® STAR RP-18 (Hitbar® RT 125-4 vorgepackt, Vorsäule LiChroCART® STAR RP18, Merck)
HPLC System: LC20AD (Shimadzu, Japan)
Flussrate: 1 ml/min.
Probe: 20 µl bei 1 mg/ml
Detektionswellenlänge: 200 nm

**Gradient:**

| Zeit (min) | Pumpe A (%) Acetonitrile | Pumpe B (%) Wasser (pH 2.6) |
|---|---|---|
| 0 | 35 | 35 |
| 8 | 35 | 35 |
| 16 | 43 | 57 |
| 18 | 70 | 30 |
| 23 | 70 | 30 |
| 25 | 35 | 35 |
| 30 | 35 | 65 |

pH 2.6 eingestellt mit orthophosphoriger Säure 85%

### Retentionszeiten:

7-Keto-3,12-Dehydroxycholansäure 7,5 min
Cholsäure 13,5 min

### 3. Expressionsvektoren und Plasmide und deren Herstellung

### plJ773 (SEQ ID NO:1)

Das Plasmid plJ773 enthält das Gen, das für Aparamycin-Resistenz codiert (B. Gust et al. PNAS February 18, 2003 vol. 100 no. 4 1541-1546). Diese Resistenz wird benötigt für die Selektion nach erfolgter Rekombination.

### pJOE6038.1 (abgeleitet von plJ790) (SEQ ID NO:2)

Das Plamid pJOE6038.1 (B. Gust et al. PNAS February 18, 2003 vol. 100 no. 4 1541-1546) enthält das Lambda Red-Rekombinationssystem, das aus drei Genen Gam, Bet und Exo besteht. (K. Murthy, J. Bacteriology, Apr. 1998, p. 2063-2071). Gam inhibitiert die Wirt-Exonuclease RecBCD V und ermöglicht ,dass Bet und Exo die Rekombination durchführt.

### pCP20

Zum Ausschneiden des Selektionsmarks wird das Plasmid pCP20 (K. Datsenko und B. Wanner, PNAS June 6, 2000 vol. 97 no. 12 6640-6645) eingesetzt, das die zum Ausschneiden erforderliche FLP-Rekombinase kodiert. Die FLP-Rekombinase schneidet DNA zwischen den FRT-Stellen.

### pET28a(+),

pET28a(+), (Novagen, Darmstadt) ist ein Vektor, welcher eine MCS unter der Kontrolle eines T7-Promotors und -Transkriptionsstarts enthält und einen T7-Terminator besitzt und zur Expression von 12α-HSDH diente. Die Expression wird mittels Isopropyl-*β*-D-thiogalactopyranosid (IPTG) induziert.

### pET28a(+)-7β-HSDH

Das Plasmid wurde wie folgt hergestellt (vgl auch PCT/EP20100/ 068576 der Anmelderin):
Zunächst wurde die 7β-HSDH kodierende Sequenz PCR-amplifiziert. Die PCR-Produkte erhielt man unter Verwendung der genomischen DNA von *Collinsella aerofaciens* ATCC 25986 (DSM 3979) als Template und den Primer 5'-gggaattcCATATGAACCTGAGGGAGAAGTA-3' (SEQ ID NO:26) und 5'- cccAAGCTTCTAGTCGCGGTAGAACGA-3' (SEQ ID NO:27). Die *Nde*I und *Hind*III Schnittstellen in den Primer-Sequenzen sind unterstrichen. Das PCR-Produkt wurde mittels PCR-Purification-Kit (Qiagen) aufgereinigt und dann mit den Enzymen *Nde*I und *Hind*III geschnitten.

Das verdaute PCR-Produkt wurde gereinigt und in den pET-28a(+)-Vektor unter Verwendung der T4-Ligase kloniert, um den Expressionsvektor zu erzeugen. Das resultierende Expressionskonstrukt wurde anschließend in *E. coli* DH5α-Zellen transformiert. Das zu erwartende Protein sollte 20 Aminosäurereste umfassendes ein Signalpeptid und einen N-terminalen 6xHis-Tag sowie eine Thrombin-Schnittstelle aufweisen. Die Sequenz der insertierten DNA wurde durch Sequenzierung überprüft (vgl SEQ ID NO: 24 und 25).

### pET22b(+) 3α-HSDH:

Dies ist ein pET22b(+)-Vektor in das die 3α-HSDH Sequenz aus *Comamonas testosteroni* (vgl SEQ ID NO: 28 und 29) über die Schnittstellen *Nde* I und *Eco*R I in üblicher Weise einkloniert wurde (Oppermann et al.,J Biochem, 1996, 241(3):744-749).

### pET21a(+) FDH D221G

Dies ist ein pET21a(+)-Vektor, in den die Formiatdehydrogenase aus *Mycobacterium vaccae* N10 (SEQ ID NO:32) über die Schnittstellen *Nde* I und *Eco*R I einkloniert wurde. Durch ortsgerichtete Mutagenese wurde der Aspartatrest (D) an Position 221 (ohne Berücksichtigung von Methionin in Position 1) bzw. Position 222 (gerechnet ab Methionin in Position 1; vgl SEQ ID NO: 38 und 23) der Formiatdehydrogenase durch einen Glycinrest ersetzt. Die so erhaltenen FDH Mutante kann nicht nur NADH, sondern auch NADHP regenerieren. Die Formiatdehydrogenase trägt an der Position 1202 (berechnet unter Berücksichtigung des Start--Codons ATG) der Nukleotidsequenz eine einzelne Basendeletion, was zum Austausch der letzten Aminosäure Valin gegen ein Alanin führt. Gleichzeitig führt diese Basendeletion zum Ausschalten des Stoppcodons und zur Aktivierung des ursprünglich außerhalb des Leserahmens liegenden His·Tags (vgl. SEQ ID NO: 22 und 23). (FDH D221G ohne His Tag siehe SEQ ID NO: 37 und 38).

Das Expressionsplasmid wurde wie folgt hergestellt (vgl auch EP Anmeldung 10015726 der Anmelderin):

### a) Klonierung pET21a(+) FDH

Als Templat der Amplifizierung dient genomische DNA von *Mycobacterium vaccae* N10, welche von der culture collection of the Biology Department of Moscow State University, Russia bezogen wurde (Hinterlegungsnummer 43292). Primer für die Amplifizierung waren
fdh_for (5'-CGATCATATGGCAAAGGTCCTGTGCGTTC-3') (SEQ ID NO:33) und
fdh_rev (5'-GCTAGAATTCTCAGCCGCCTTCTTGAACT-3') (SEQ ID NO:34),
bezogen von Eurofins MWG GmbH, Ebersberg. Die Erkennungsstellen für die Restriktionsenzyme sind unterstrichen. Der rev-Primer enthält die *EcoRI* Schnittstelle, der for-Primer enthält die *NdeI*-Schnittstelle.

Die PCR-Ansätze und PCR-Programme sind in den Tabellen angegeben.

**Tabelle: PCR-Ansatz für die Amplifizierung der Formiatdehydrogenase aus Mycobacterium vaccae**

| Komponente | Volumen [µl] |
|---|---|
| 10x Taq Puffer (mit Mg²⁺⁾ | 5 |
| dNTPs (10 mM) | 1 |
| Fdh_for (100 µM) | 0,5 |
| Fdh_rev (100 µM) | 0,5 |
| Templat-DNA (>=100 ng/µL) | 1 |
| Taq DNA Polymerase (5 U/mL) | 0,5 |
| Destilliertes Wasser | 41,5 |

**Tabelle: PCR-Programm für die Amplifizierung der Formiatdehydrogenase aus Mycobacterium vaccae**

| Segment | Zyklenzahl | Denaturierung | Annealing | Elongation |
|---|---|---|---|---|
| 1 | 1 | 94°C, 2 min | | |
| 2 | 5 | 94°C, 30 sec | 55,6°C, 30 sec | 72°C, 75 sec |
| 3 | 25 | 94°C, 30 sec | 58,6 °C, 30 sec | 72°C, 75 sec |
| 4 | 1 | | | 72°C, 75 sec |

1-5 µg in Wasser gelöste DNA (pET21a(+) bzw. FDH-PCR-Produkt) werden mit 5µl 10x NEBuffer *Eco*RI, 2,5 µl Ndel (20 U/mL) und 2,5 µl EcoRI (20 U/mL) (jeweils New England Biolabs, Frankfurt) versetzt und mit destilliertem Wasser auf 50 µl Gesamtvolumen aufgefüllt. Die Ansätze wurden jeweils 1 h bei 37 °C inkubiert. Im Anschluss werden die geschnittenen DNA-Fragmente auf ein 1 %-iges Agarosegel (1 % (m/v) Agarose, 0,05 % (v/v) Ethidiumbromid) aufgetragen und die DNA-Fragmente 55 Minuten bei 120 V elektrophoretisch aufgetrennt. Anschließend wurden die Banden der richtigen Größe (1,2 kb bei dem FDH-Gen, 5,4 kb bei dem pET21a(+)-Plasmid) mit einem Skalpell aus dem Agarosegel ausgeschnitten und mit Hilfe des QIAQuick Gel Extraction Kit (QIAGEN, Hilden) gemäß Herstellerprotokoll isoliert.

100 ng geschnittene Vektor-DNA und 111 ng geschnittene FDH-DNA wurden mit 1 µl T4 Ligase (3 U/µL) und 1 µl 10x Ligase Puffer (jeweils New England Biolabs, Frankfurt) versetzt und mit destilliertem Wasser auf ein Gesamtvolumen von 10 µl aufgefüllt. Der Ligationsansatz wurde über Nacht bei 4°C inkubiert.

Nach Beendigung des Ligationsschritts werden die 10 µL Ligationsansatz zu 200 µl nach Standardprotokoll hergestellte, chemisch kompetente *E. coli* DH5α gegeben. Danach erfolgt 30 Minuten lang ein Inkubationsschritt auf Eis, gefolgt von einem Hitzeschock bei 42°C (90 Sekunden). Anschließend werden 600 µl steriles LB-Medium zum Transformationsansatz zugegeben und die Zellen bei 200 rpm und 37°C in einem Schüttelinkubator 45 Minuten inkubiert. Im nächsten Schritt wird der Ansatz bei 3000 rpm 60 Sekunden in einer Tischzentrifuge abzentrifugiert, 700 µl vom Überstand werden verworfen, die Zellen im restlichen Überstand resuspendiert und auf einer LB-Agarplatte mit 100 mg/l Ampicillin ausplattiert. Die Agarplatte wird im Anschluss bei 37°C über Nacht inkubiert.

### b) Herstellung pET21a(+) FDH D221G

Folgende Primer wurden verwendet:

| | |
|---|---|
| mt1: | 5'- C CTG CAC TAC ACC G**G**C CGT CAC CGC CTG C - 3' (SEQ ID NO: 35) |
| NI_fdh_R: | 5' -GCTCGAATTCTCAGACCGCCTTC--3'(SEQ ID NO:36) |

Zunächst wurde mit Hilfe des mt-Primers und des Primers NI_fdh_R ein Set von zwei komplementären Megaprimern erzeugt. Als Template wurde Plasmid pET21a(+)FDH verwendet. Das verwendete PCR-Programm ist folgender Tabelle zu entnehmen:

**Tabelle: PCR-Programm Megaprimer**

| Segment | Zyklenzahl | Denaturierung | Annealing | Elongation |
|---|---|---|---|---|
| 1 | 1 | 94°C, 2 min | | |
| 2 | 30 | 94°C, 30 sec | 60°C, 30 sec | 72°C, 40 sec |
| 3 | 1 | 72°C, 5 min | | |

Durch Kombination von Primer mt1 mit dem Primer NI_fdh_R ergibt sich die Länge des Megaprimer zu 650 bp. Mit diesem PCR-Produkt der ersten PCR erfolgt eine Gelelektrophorese und Isolierung der gewünschten Bande aus dem Gel. Eine zweite PCR wird als Whole Plasmid PCR mit den Megaprimern als Primer und der Plasmid-DNA (pETfdh) als Template durchgeführt. Den Reaktionsansatz und das Temperaturschema für die Whole Plasmid PCR zeigen die folgenden Tabellen. Der 2x EZClone enzyme mix, die EZClone Solution 1, der 1,1 kb Marker und das *DpnI* stammten aus dem GeneMorph II EZClone Domain Mutagenesis Kit (Stratagene).

**Tabelle: Ansatz für eine MEGA WHOP PCR (Gesamtvolumen 50 µL)**

| Komponente | |
|---|---|
| Megaprimer | 250 ng (∼ 2,5 µL aus einer Standard-PCR) |
| Template (pETfdh) | 50 ng |
| 2x EZClone enzyme mix | 25 µL |
| EZClone Solution 1 | 3 µL |
| dest. Wasser | ad 50 µL |

Der erste Schritt im PCR-Programm (68°C, 5 min) dient dazu, die durch die *Taq*-Polymerase unspezifisch angehängten Basen durch die 3'->5' Exonukleaseaktivität der in der MEGA WHOP PCR eingesetzten Polymerase zu entfernen.

**Tabelle: PCR-Programm MEGA WHOP**

| Segment | Zyklenzahl | Denaturierung | Annealing | Elongation |
|---|---|---|---|---|
| 1 | 1 | | | 68°C, 5 min |
| 2 | 1 | 95°C, 1 min | | |
| 3 | 25 | 95°C, 50 sec | 60°C, 50 sec | 68°C, 13 min |

Das PCR-Produkt ist ein doppelsträngiges Plasmid mit Einzelstrangbrüchen, die erst in *E. coli* geschlossen werden. Zu dem 50 µL PCR-Produkt wurden 10 U *DpnI* zugesetzt und der Ansatz bei 37°C für zwei Stunden inkubiert. *DpnI* baut nur methylierte DNA ab, d.h. die eingesetzte Template-DNA, nicht aber den Megaprimer oder das synthetisierte Plasmid. Das Template-Plasmid muss mit einem dam⁺-Stamm (wie DH10B oder JM109) erzeugt werden, um methylierte Ausgangs-DNA zu erhalten.

Auf diese Weise erhält man das oben genannte Expressionsplasmid pET21a(+) FDH D221G

### 4. Mikroorganismen

| **Stamm** | **Genotyp** |
|---|---|
| *Escherichia coli* BL21 (DE3) | F⁻ ompT gal dem Ion hsdS_{B}(r_{B}⁻m_{B}⁻) λ(DE3 [lacl lacUV5 T7 gene 1 ind1 sam7 nin5]) |

### B. BEISPIELE

### Beispiel 1: Herstellung einer 7α-HSDH Deletionsmutante des E.coli Stamms BL21(DE3) (E. coli BL21(DE3) Δ7α-HSDH) (Typ1((Knock-out durch homologe Rekombination)

### 1.1 Sequenzinformation zur 7α-HSDH aus E. coli BL21(DE3)

### Aminosäuresequenz: (SEQ ID NO:10)

Länge: 255 Aminosäuren
Typ: Protein
Quelle: *E. coli* BL21(DE3)

### Nukleotidsequenz (SEQ ID NO:9)

Länge: 768 Basenpaare
Typ: Nukleinsäuren
Quelle: *E. coli* BL21(DE3)
Accession: NC_012971 REGION: 1642470..1643237 (kodierender Strang)

### 1.2 Herstellung der Aparamycin-Deletionskassette

Folgende Primer für die Ausknockung der 7α-HSDH aus *E. coli* BL21(DE3) wurden hergestellt.
Vorwärts: 5'-GTGTTTAATTCTGACAACCTGAGACTCGACGGAAAAATG*ATTCCGGG GATCCGTCGACC*-3' (SEQ ID NO: 3)
Rückwärts: 5'-TTAATTGAGCTCCTGTACCCCACCACCGGAGACGGTTTA*TGTAGGCT GGAGCTGCTTC*-3' (SEQ ID NO: 4)

Die homologen Sequenzen zur 7α-HSDH-Sequenz sind unterstrichen, wobei die unterstrichene Sequenz aus dem Vorwärts-Primer für
"VFNSDENLRLDGK" (N->C)
und die unterstrichene Sequenz aus dem Rückwärts-Primer für
"TVSGGGVQELN" (N->C)
kodiert.

Die homologen Sequenzen zur Aparamycin-Resistenz-Kassette sind in kursiv markiert. Die Aparamycin-Resistenz-Kassette aus Plasmid plJ773 ist von FRT-Stellen frankiert (B. Gust et al. PNAS February 18, 2003 vol. 100 no. 4 1541-1546) und erlaubt, die Resistenz nach erfolgter Rekombination aus dem Genom wieder zu entfernen.

Die PCR mit der Aparamycin-Resistenz-Kassette aus Plasmid plJ773 als Template wurde nach Standard-Protokoll wie Sambrook (1998) durchgeführt. Das so hergestellte PCR-Produkt mit zwei Homologie-Armen aus dem Gen der 7α-HSDH wurde aufgereinigt.

### 1.3 Rekombination/Deletion der Zielsequenz

Das Plasmid pJOE6038.1, das mit der invertierten Nhel-Schnittstelle von plJ790 (B. Gust et al. PNAS February 18, 2003 vol. 100 no. 4 1541-1546) ausgestattet ist, kodiert eine Exonuclease (Lambda Red-Recombinase, K. Murthy, J. Bacteriology, Apr. 1998, p. 2063-2071). Das Plasmid pJOE6038.1 ist temperatursensitiv und geht bei Kultivierung bei 37°C verloren. Das Plasmid pJOE6038.1 wurde mittels Elektroporation (25µF, 200 Ohm und 2,5 kV) in *E. coli* BL21 (DE3) transformiert. Eine Kolonie wurde in 5 ml LB gepickt und über Nacht bei 30°C kultiviert. 40 ml LB Medium wurde 1:100 angeimpft und bei 30°C inkubiert. Bei einer OD₆₀₀ von ca. 0,3 wurde die Kultur mit 0,4% L-Arabinose induziert und bei 37°C für 1 h inkubiert, um die Exonuclease (Lambda Red-Recombinase) zu exprimieren, die die Rekombinantion ermöglicht.

Die Zellen wurden abzentrifugiert und mit 30 ml eiskaltem Wasser gewaschen. Die Zellen wurden erneut abzentrifugiert und mit 700 µl eiskaltem Wasser resuspendiert. 50 µl davon wurden mit 5 µl PCR-Prudukt (aus obigem Schritt 1) auf Eis für 30 min inkubiert. Nach Elektroporation (25µF, 200 Ohm und 2,5 kV) wurde sofort 1 ml vorgekühltes LB Medium dazu pipettiert. Die Zellen wurden bei 37°C für 2 Stunden 45 Minuten inkubiert. Danach wurden die Zellen auf LB-Aparamycin-Agar-Platte ausplattiert. Eine Kolonie wurde gepickt. Kolonie-PCR mit 7α-HSDH Primern zeigte eine 1500 bps Bande (Aparmycin-Resistenz). Dies zeigt, dass das Gen der 7α-HSDH ersetzt und die Aparamycin-Resistenz dabei in das Genom integriert wurde (siehe Figur 1). Der so erhaltene Knock-out-Stamm wird bezeichnet als *E. coli* BL21 (DE3) Δ7α-HSDH Apar^{R}.

### 1.4 Ausschneiden der Resistenz

Das für die Ausknockung verwendete Resistenzgen flankiert von zwei FRT-Stellen sollte wieder ausgeschnitten werden. Zum Ausschneiden wurde das Plasmid pCP20 (K. Datsenko und B. Wanner, PNAS June 6, 2000 vol. 97 no. 12 6640-6645) eingesetzt, das die zum Ausschneiden erforderliche FLP-Rekombinase kodiert. Die FLP-Rekombinase schneidet DNA zwischen den FRT-Stellen. Das Plasmid pCP20 ist auch temperatursensitiv und geht bei Kultivierung bei 37°C verloren. Das Plasmid pCP20 wurde mittels Elektroporation (25µF, 200 Ohm und 2,5 kV) in den Knockout-Stamm *E. coli* BL21 (DE3) Δ7α-HSDH Apar^{R} transformiert. 4 Kolonien wurden auf LB-0 ausgestrichen und bei 42°C über Nacht kultiviert, wobei die FLP-Rekombinase exprimiert wurde und diese das Aparamycin-Resistenz-Gen ausschnitt. Man erhält so den Knockout-Stamm *E. coli* BL21 (DE3) Δ7α-HSDH.

Zum Test auf Verlust der Resistenz wurde 50 Kolonien auf LB-Aparamycin Medium und LB-0 Medium gestrichen. Die Kolonien, die nur auf LB-0 gewachsen sind, tragen keine Aparmycin-Resistenz mehr. Eine Kolonie wurde in 5 ml LB gepickt. 20 mM Cholsäure wurden zu 5 ml Kultur zugegeben. Es konnte keine Oxidationsprodukt 7-Keto-3,12-Dihydroxycholansäure (RT: 7,5 min) mit dem Knock-out-Stamm mittels HPLC nachgewiesen werden. Dagegen zeigte *E. coli* BL21 (DE3) deutlich 7α-HSDH Aktivität gegen Cholsäure (RT: 13,5 min). Das 7α-HSDH Gen wurde somit erfolgreich ausgeknockt (siehe Figur 2).

Der 7α-HSDH-freie Stamm *E. coli* BL21 (DE3) Δ7α-HSDH wurde somit erfolgreich hergestellt.

### Beispiel 2: Heterologe Expression der 12α-HSDH mit E. coli BL21(DE3) Δ7α-HSDH (Typ1)

Zur Expression von 12α-HSDH diente der Vektor pET28a(+), Novagen, Darmstadt, welcher eine MCS unter der Kontrolle eines T7-Promotors und -Transkriptionsstarts enthält und einen T7-Terminator besitzt. Die Expression wird mittels Isopropyl-β-D-thiogalactopyranosid (IPTG) induziert.

Dazu wurden 12α-HSDH (Kurzversion) kodierende Sequenzen PCR-amplifiziert (vgl auch PCT/EP2009/002190 der Anmelderin). Die PCR-Produkte erhielt man unter Verwendung der genomischen DNA von Clostridium sp. group P strain 48-50 als Template und den Primerpaaren:
(5'-*GGTATTC***CATATG**ATCTTTGACGGAAAGGTCGC-3' (SEQ ID NO: 5)und
5'-*CG***GGATCC**CTAGGGGCGCTGACCC-3') (SEQ ID NO: 6).

Kursive Nukleotid-Reste sind Überhang. Fettgedruckte Reste kodieren eine Schnittstelle. Die übrigen Reste sind die homologe Sequenz zu 12α-HSDH.

Die PCR-Produkte wurden auf ein Agarose-Gel aufgetragen, aufgetrennt und aus diesem ausgeschnitten. Anschließend wurden sie mit Hilfe von Ndel und BamHI restringiert und mit dem ebenfalls mit Ndel und BamHI geschnittenen pET28a(+)-Vektor ligiert.

Der N-terminale His-Tag wurde anschließend mittels Quick-Change mit folgenden Primern entfernt. Das Plasmid wurde sequenziert und verifiziert.
5'-GTTTAACTTTAAGAAGGAGATATACCATGATCTTTGACGGAAAGGTCGCAATCATT-3' (SEQ ID NO: 7)
5'-AATGATTGCGACCTTTCCGTCAAAGATCATGGTATATCTCCTTCTTAAAGTTAAAC-3' (SEQ ID NO: 8)

Zur Untersuchung der Expression mit *E. coli* BL21 (DE3) Δ7α-HSDH wurden chemisch kompetente Zellen nach unten angegebenem Protokoll hergestellt. Vektor pET28a+ (Novagen, enthält T7-Promotor und Kanamycin-Resistenz) mit 12α-HSDH (ohne His-Tag) wurde in *E. coli* BL21(DE3) Δ7α-HSDH transformiert.

### Herstellung von chemisch kompententen Zellen:

1 ml Übernacht-Kultur von *E. coli* wurde in 100 ml LB Medium angeimpft. Die Zellen wurden bei 37 °C und 180 rpm bis zu OD₆₀₀ 0.4 - 0.6 inkubiert. Die Zellen wurden auf Eis für 15 min gekühlt und bei 4 °C und 4000 rpm für 10 min zentrifugiert. Der Überstand wurde entfernt und die Zellen wurden in 40 ml vorgekühlter Tfbl Lösung (Kaliumacetat 30 mM, Rubidiumchlorid 100 mM, Calciumchlorid 10 mM, Manganchlorid 50 mM, Glycerin 15%) resuspendiert. Nach 15 min Inkubation auf Eis wurden die Zellen abzentrifugiert. Der Überstand wurde entfernt. Die Zellen wurden in 4 ml vorgekühlt Tfbll Lösung (MOPS 10 mM, Calciumchlorid 75 mM, Rubidiumchlorid 10 mM, Glycerin 15%) resuspendiert. Zur Transformation wurde 1 µl Plasmid DNA in 200 µl Kompetente Zellen zugegeben und die Mischung wurde auf Eis für 10 min inkubiert. Der Hitzeschock der Zellen wurde bei 42 °C für 40 sec durchgeführt. Danach wurde sofort 800 µl LB-Medium zugegeben. Die Zellen wurden bei 37 °C und 200 rpm für 1 Stunde inkubiert. Die transformierten Zellen wurden abzentrifugiert und der Überstand wurde entfernt. Das Pellet wurde auf einer LB-Agar-Platte ausplattiert. Die Platte wurde bei 37 °C zur Bildung von Kolonien inkubiert.

Eine Kolonie wurde in 5 ml LB gepickt. Nach 4 Stunden wurden 50 ml LB mit 5 ml Vorkultur angeimpft. Bei einer OD₆₀₀ von ca. 0,8 wurden die Zellen mit 0,5 mM IPTG induziert. Die Zellen wurden bei 25°C und 140 Upm übernacht inkubiert. Dann wurden die Zellen abzentrifugiert, resuspendiert und aufgeschlossen. Die 12α-HSDH-Aktivität wurde nach oben angegebener Methode bestimmt. Die Ausbeute von 12α-HSDH betrug ca. 50 kU pro Liter LB-Medium.

### Beispiel 3: Herstellung einer E. coli 7α-HSDH Knockout-Mutante (E. coli BL21 (DE3) hdhA⁻KanR⁺) (Typ2) (Knock-out durch Gen-Disruption)

Ziel ist die Deletion der störenden 7α-HSDH Aktivität im Expressionsstamm *E. coli* BL21 (DE3).

Mit der im Folgenden beschriebenen Methode wird in das Zielgen der 7α-HSDH ein Antibiotika-Resistenzgen insertiert, wodurch das Zielgen ausgeschalten wird.

### 3.1 Sequenzinformation zur 7α-HSDH aus E. coli BL21(DE3)

### Vgl. Beispiel 1

### 3.2 Verwendete Primer

Folgende Primer für das Ausschalten der 7α-HSDH aus *E. coli* BL21 (DE3) wurden hergestellt:
Primer für das Retargeting des LI.LtrB Introns, das im TargeTron™ Gene Knockout System" (s. Kapitel 3.3) verwendet wird:
   467|468a-IBS (SEQ ID NO:17)
      AAAAAAGCTTATAATTATCCTTATAGGACGTCATGGTGCGCCCAGATAGGGTG
   467|468a-EBS1d (SEQ ID NO:18)
      CAGATTGTACAAATGTGGTGATAACAGATAAGTCGTCATGTTTAACTTACCTTTCTTTGT
   467|468a-EBS2 (SEQ ID NO:19)
      TGAACGCAAGTTTCTAATTTCGGTTTCCTATCGATAGAGGAAAGTGTCT
   EBS Universal : 5'-CGAAATTAGAAACTTGCGTTCAGTAAAC
      (Gemäß User Guide für die Control reaction)

Insertion des umprogrammieren Introns an folgender Stelle
Location
467|468a GCAGCTTTAGATGATGCATAGGAAGTCATG - intron - TTTATATTTTTATTT

### 3.3 Herstellung der Knockout-Mutante

Die Herstellung der Knockout-Mutante wurde mit Hilfe des Kits TargeTron™ Gene Knockout System von Sigma Aldrich nach Herstellerangaben durchgeführt. Es wurde zur Aufreinigung des PCR-Produkts gemäß Schritt B.6. des TargeTron™ Gene Knockout System der QIAquick PCR Purification Kit von Qiagen genutzt.

Die Ligation des HindIII/BsrGI-verdauten Intron-PCR-Produkts in den linearisierten pACD4K-C Vektor wurde wie folgt durchgeführt: Die Reaktion erfolgte über Nacht bei 16°C.

**20µl-Ansatz:**

| | |
|---|---|
| 2 µl | pACD4K-C linear vector (40 ng) |
| 6 µl | HindIII/BsrGI-digested intron PCR product |
| 2 µl | ATP (10 mM) |
| 2 µl | Ligase-Puffer (10 x) (Fermentas) |
| 2 µl | T4-Ligase (Fermentas) |
| 6 µl | H₂O |

5 µl Ligationsreaktionslösung wurden zu 200 µl chemisch-kompetenten Zellen *E. coli* BL21 (DE3) gegeben und für 20 min auf Eis inkubiert. Die weitere Transformation erfolgte wie von Herstellerseite beschrieben.

Die Transformations-Ansätze wurden auf LB-Agar-Platten, enthaltend 33 µg/mL Kanamycin, ausplattiert. Es wurden Kanamycin-resistente Zellen gepickt und diese jeweils über mehrere Nächte in 5 ml LB-Übernacht-Kulturen überimpft (jeweils mit 5 µl einer Kanamycin-Lösung (33 mg/ml)). Abschließend wurde eine 200 ml LB-Kultur (mit 200 µl Kanamycin-Lösung (33 mg/mL)) mit einer Übernachtkultur angeimpft und für 5 h bei 37°C und 180 rpm im Schüttelinkubator inkubiert. Anschließend wurde die Temperatur auf 42°C für 1 Stunde erhöht. Mit dieser Kultur wurde eine 5 mL LB-Übernacht-Kulturen angeimpft (jeweils mit 5 µl einer Kanamycin-Lösung (33 mg/mL)). Nach Inkubation über Nacht bei 37°C und 180 rpm wurde die Kultur auf einer LB-Agar-Platte mit 33 µg/mL Kanamycin ausgestrichen. Nach Übernacht-Inkubation bei 37°C wurden Kolonien gepickt und auf LB-Agar-Platte mit 33 µg/mL Kanamycin und 34 µg/mL Chloramphenicol ausgestrichen.

Nach Übernacht-Inkubation bei 37°C wurden Chloramphenicol-sensitive Mutanten gefunden. Dies ist erforderlich, um den Verlust des Plasmids, welches das induzierbare Knockout-System trägt und nach erfolgreichen Knockout nicht mehr benötigt wird, zu bestätigen

Die Kanamycin-Resistenz ist erst bei Insertion aktiv. Auf dem Vektor pACD4K-C hat sie eine Deletion. Eine Kolonie, die sowohl auf Kanamycin und Chloramphenicol wächst, hat also höchstwahrscheinlich den gewünschten Knockout, aber auch noch den pACD4K-C-Vektor. Erst, wenn die Stämme nur noch Kanamycin-resistent sind, haben sie den pACD4K-C-Vektor verloren, aber auch den gewünschten Knockout.

### 3.4 Nachweis des Knockouts

Das 7α-HSDH-Gen wurde über Kolonie-PCR mit den Primern
7alpha-ko-check_fwd (5'- TTAATTGAGCTCCTGTACCCCACCACC- 3') SEQ ID NO:20 und 7alpha-ko-check_rev (5' - GTGTTTAATTCTGACAACCTGAGACTCGAC - 3') SEQ ID NO:21 amplifiziert. Das entstandene Fragment hatte eine Länge von ca. 2,5-3 kb und wurde mit dem Primer 7alpha-ko-check_fwd sequenziert. Anhand der Sequenzierung konnte nachgewiesen werden, dass die DNA-Sequenz der 7α-HSDH durch ein Insert aus dem pACD4K-Vektor unterbrochen ist, was den Knockout der 7α-HSDH zur Folge hat. (Sequenzierdaten nicht gezeigt)

### 3.5 Expression von 12alpha- und 7beta-HSDH-Mutanten mit dem KO Stamm

Die Brauchbarkeit dieses Knockout-Stamms wurde beispielsweise durch Enzymexpressionen von 12α- und 7β-HSDH-Mutanten im Schüttelkolben-Maßstab verifiziert (Ergebnisse nichtgezeigt).

### Beispiel 4: Enzymatische Umsetzung von DHCS zu 12-Keto-UDCS durch die 7β-HSDH, FHD D221G und 3α-HSDH, jeweils hergestellt unter Verwendung des Knock-out Stamm E. coli BL21(DE3)Δ7α-HSDH (Typ 1)

In diesem Beispiel soll eine zweistufige enzymatische Umsetzung von DHCS zu 12-Keto-UDCS bei gleichzeitiger Cofaktorregenerierung mit einer speziellen FDH-Mutante untersucht werden. Da die verwendete FDH Mutante D221G sowohl NADP⁺ als auch NAD⁺ als Cofaktor akzeptiert, muss für die NADH-abhängige 3α-HSDH kein zusätzliches Cofaktorregenerierungssystem in den Reaktionsansatz eingebracht werden.

Die Umsetzung wird beispielhaft durch die beiden im Folgenden grafisch dargestellten Teilreaktionen veranschaulicht. FDH* bezeichnet die Mutante FDH D221G.

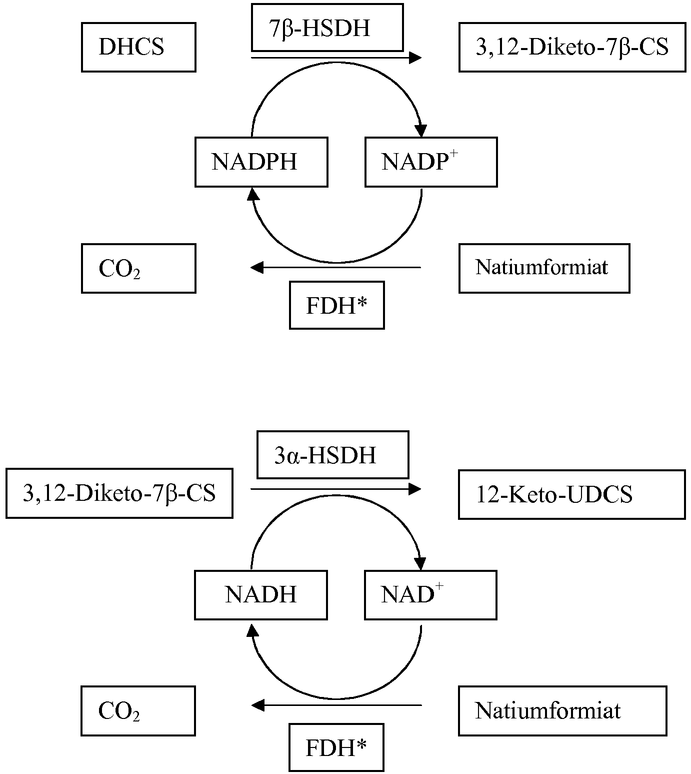

Hierzu wurden die Enzyme 7β-HSDH aus *Collinsella aerofaciens,* 3α-HSDH aus *Comamonas testosteroni* und die FDH-Mutante D221G abgeleitet von FDH aus *Mycobacterium vaccae* getrennt voneinander in dem Knock-out Stamm *E. coli* BL21(DE3)Δ7α-HSDH (Typ 1), hergestellt gemäß obigem Beispiel 1, exprimiert und zur Umsetzung verwendet.

### 4.1 Verwendete Plasmide,

Plasmide für die Expression von 7β-HSDH, 3α-HSDH und FDH D221G:
pET28a(+)-7β-HSDH,
pET22b(+)-3α-HSDH und
pET21a(+)-FDH-D221G.

### 4.2 Bakterienstämme und Kulturbedingungen:

Der gemäß Beispiel 1 hergestellte Knock-out Stamm *E. coli* BL21 (DE3)Δ7α-HSDH (Typ1) wurde bei 37°C in LB-Medium, enthaltend die erforderlichen Antibiotika, gezüchtet. Nach Induktion mit 0,5 mM IPTG nach Erreichen von OD₆₀₀ = 0,8 wurde die Inkubation bei 140 Upm über einen Zeitraum von 12 Stunden bei 25°C fortgeführt.

### 4.3 Enzymüberexpression und Reinigung

Überexpression von 7β-HSDH, 3α-HSDH und der FDH-Mutante D221G in *E. coli* BL21(DE3)Δ7α-HSDH und Enzym-Reinigung erfolgten unter folgenden Bedingungen:
*Der E. coli* Knock-out Stamm wurde mit dem Expressionskonstrukt transformiert. Dazu wurde der das Expressionskonstrukt enthaltende Stamm in LB-Medium (2 × 400 ml in 2 Liter-Schüttelflaschen) enthaltend 30 µg/ml Kanamycin vermehrt.

Die Zellen wurden durch Zentrifugation (10,000×g, 15 min, 4°C) geerntet. Das Pellet wurde in 20 ml Phosphatpuffer (50 mM, pH 8, enthaltend 0.1 mM PMSF) resuspendiert. Die Zellen wurden unter konstanter Kühlung durch einminütige Ultraschallbehandlung (40 W Leistung, 40% Arbeitsintervall und 1 min Pause) unter Verwendung eines Ultraschallgeräts Sonifier 250 (Branson, Deutschland) aufgeschlossen. Der Aufschluss wurde dreimal wiederholt. Das Zellextrakt wurde zentrifugiert (22,000xg, 20 min, 4°C). Schließlich wurde die Probe in einen neuen Schlauch überführt und bei -20°C zur weiteren Analyse gelagert. Die Proteinkonzentration wurde unter Verwendung eines BCA Testkits (Thermo, USA) nach Herstellerangaben bestimmt. Außerdem wurde die Probe durch 12.5%ige SDS-PAGE und Färben mit Coomassie Brilliant Blue analysiert. Die Reinheit des Proteins wurde densitometrisch mit Hilfe von Scion Image Beta 4.0.2 (Scion, USA) bestimmt. Die Zellextrakte von 7β-HSDH, 3α-HSDH und FDH waren Rohextrakte. Eine weitere Aufreinigung erübrigte sich, da die 7α-HSDH schon ausgeknockt wurde.

Die Ausbeuten je Liter Kulturmedium (Schüttelkolben bei OD₆₀₀ ∼ 6) waren wie folgt:
7β-HSDH: 3883U (für DHCS und NADPH)
3α-HSDH: 6853 U (für DHCS und NADH)
FDH-Mutante: 47 U (für Natriumformiat und NAD⁺).

Gehalt und Reinheit der Proteine wurde mittels SDS-PAGE und Densitometerscanning mittels Scieon Image Beta 4.0.2 (Scieon, USA) bestimmt.

### 4.4 Enzymatische Synthese von 12-Keto-UDCS im präparativen Maßstab

Ein 800 ml Reaktionsansatz, enthaltend 7β-HSDH (2,4 U x ml⁻¹), 3α-HSDH (2,4 U x ml⁻¹), FDH D221G (0,325 U x ml⁻¹), NADP⁺ (10 µM), NAD⁺ (10 µM), Natriumformiat (250 mM), DHCS (10 mM, 3,2 g) und Kaliumphosphat-Puffer (50 mM, pH 6), wurde bei 24°C gerührt. Sämtliche drei Enzyme, die in diesem Experiment verwendet wurden, wurden als Zellrohextrakte ohne zusätzlichen Reinigungsschritt eingesetzt. Nach 12 Stunden wurde die Reaktion durch Entfernung der Enzyme mittels Ultrafiltration unter Verwendung einer Membran mit einer Porengröße von 10 kDa (Millipore, USA) gestoppt. Das Produkt im Filtrat wurde durch Ansäuern mit Salzsäure auf pH 2 und anschließende Papierfiltration gereinigt. Nach Trocknen des Produktes bei 60°C über Nacht erhielt man 2,9 g des gewünschten Produktes.

Das Produkt wurde mittels HPLC und NMR analysiert.

### Analysendaten (partiell):

¹H NMR (deuteriertes DMSO, 500 MHz) δ = 3,92 (2H, m, H-3α und H-7β) und
¹H- NMR (deuteriertes DMSO, 125 MHz) δ =69,38 (CH, 3-C); δ = 69,09 (CH, 7-C); δ = = 213,86 (C, 12-C)
Ausbeute: 90,6 %
Reinheit: 99 %

Zuordnung der SEQ ID NOs:

| SEQ ID NO: | Beschreibung | Typ |
|---|---|---|
| 1 | pIJ773 | NS |
| 2 | pJOE6038.1 | NS |
| 3 | PCR Primer | NS |
| 4 | PCR Primer | NS |
| 5 | PCR Primer | NS |
| 6 | PCR Primer | NS |
| 7 | PCR Primer | NS |
| 8 | PCR Primer | NS |
| 9 | 7α-HSDH | NS |
| 10 | 7α-HSDH | AS |
| 11 | 12α-HSDH; L | NS |
| 12 | 12α-HSDH; L | AS |
| 13 | 12α-HSDH; K | NS |
| 14 | 12α-HSDH; K | AS |
| 15 | 12α-HSDH Mutante 37D12; K | NS |
| 16 | 12α-HSDH Mutante 37D12 ; K | AS |
| 17 | Primer 467\|468a-IBS | NS |
| 18 | Primer 467\|468a-EBS1d | NS |
| 19 | Primer 467\|468a-EBS2 | NS |
| 20 | Primer 7alpha-ko-check_fwd | NS |
| 21 | Primer 7alpha-ko-check_rev | NS |
| 22 | FDH D221G mit Deletion und His-Tag | NS |
| 23 | FDH D221G mit Deletion und His-Tag | AS |
| 24 | 7β-HSDH | NS |
| 25 | 7β-HSDH | AS |
| 26 | Primer S 7beta_rev_HindIII | NS |
| 27 | Primer | NS |
| 28 | 3α-HSDH (C. testosteroni) | NS |
| 29 | 3α-HSDH (C. testosteroni) | AS |
| 30 | 3α-HSDH (R. norvegicus) | NS |
| 31 | 3α-HSDH (R. norvegicus) | AS |
| 32 | FDH Wildtyp, M. vaccae | AS |
| 33 | Primer fdh_for | NS |
| 34 | Primer fdh_rev | NS |
| 35 | Primer mt1 | NS |
| 36 | Primer NI_fdh_R | NS |
| 37 | FDH D221G | NS |
| 38 | FDH D221G | AS |

| | | |
|---|---|---|
| AS = Aminosäuresequenz NS= Nukleinsäuresequenz L = Langform K = Kurzform | | |

Auf die Offenbarung der hierin zitierten Publikationen wird ausdrücklich Bezug genommen.

## Patentansprüche

1. Rekombinanter Mikroorganismus, in welchem die enzymatische Aktivität der 7α-Hydroxysteroiddehydrogenase (7α-HSDH) vollständig inhibiert ist, während die enzymatische Aktivität einer davon funktionell verschiedenen Hydroxysteroiddehydrogenase exprimierbar enthalten ist, wobei der rekombinante Mikroorganismus von einem 7α-HSDH exprimierenden Vorläufer-Mikroorganismus abgeleitet ist.

2. Rekombinanter Mikroorganismus nach Anspruch 1, worin die 7α-HSDH kodierende Nukleinsäuresequenz durch homologe Rekombination oder durch Gen-Disruption ausgeknockt ist.

3. Rekombinanter Mikroorganismus nach einem der vorhergehenden Ansprüche, welcher die von der 7α-HSDH funktionell verschiedene HSDH rekombinant exprimiert.

4. Rekombinanter Mikroorganismus nach einem der Ansprüche 2 und 3, wobei die ausgeknockte 7α-HSDH eine Aminosäuresequenz nach SEQ ID NO: 10 aufweist oder von einer Nukleinsäuresequenz nach SEQ ID NO: 9 kodiert wird.

5. Rekombinanter Mikroorganismus nach einem der vorhergehenden Ansprüche, wobei die exprimierte, von der 7α-HSDH funktionell verschiedene HSDH eine der folgenden Aminosäuresequenzen aufweist:
a) eine 3α-HSDH Sequenz ausgewählt unter SEQ ID NO: 29 und 31
b) eine 7β-HSDH Sequenz ausgewählt unter SEQ ID NO: 25
c) eine 12α-HSDH Sequenz ausgewählt unter SEQ ID NO: 12, 14 und 16; oder
d) eine davon abgeleitete, für eine HSDH kodierende Aminosäuresequenz mit einem Identitätsgrad von wenigstens 50% zu einer der Sequenzen gemäß a), b) und c).

6. Verfahren zur rekombinanten Herstellung einer von der 7α-HSDH funktionell verschiedenen gewünschten HSDH, wobei man einen rekombinanten Mikroorganismus nach einem der vorhergehenden Ansprüche, in welchem die enzymatische Aktivität der 7α-HSDH inhibiert ist, unter Bedingungen kultiviert, unter denen die gewünschte HSDH exprimiert wird, und die so exprimierte HSDH isoliert, wobei in der isolierten HSDH im Wesentlichen keine funktionale 7α-HSDH nachweisbar ist.

7. Verfahren nach Anspruch 6, wobei die gewünschte rekombinante HSDH, ausgewählt ist unter einer 3α-, 3β-, 7β-, 11α-, 11β-, 12α-, 12β-, 17α-, 17β-, 20α-, oder 20β-HSDH.

8. Verfahren nach Anspruch 7, wobei die gewünschte rekombinante HSDH eine der folgenden Aminosäuresequenzen umfasst:
a) eine 3α-HSDH Sequenz ausgewählt unter SEQ ID NO: 29 und 31
b) eine 7β-HSDH Sequenz ausgewählt unter SEQ ID NO: 25
c) eine 12α-HSDH Sequenz ausgewählt unter SEQ ID NO: 12, 14 und 16; oder
d) eine davon abgeleitete Aminosäuresequenz mit einem Identitätsgrad von wenigstens 50% zu einer der Sequenzen gemäß a), b) und c).

9. Verfahren zur selektiven enzymatischen Oxidation von Hydroxysteroiden welche neben einer Hydroxygruppe in wenigstens einer der Positionen 3α-, 3β-, 11α-, 11β-, 12α-, 12β-, 17α-, 17β-, 20α-, oder 20β- des Steroidgerüsts, wenigstens eine weitere Hydroxygruppe in 7α-Position des Steroidgerüsts aufweisen, wobei man das Hydroxysteroid in Gegenwart einer 3α-, 3β-, 11α-, 11β-, 12α-, 12β-, 17α-, 17β-, 20α-, oder 20β-HSDH, hergestellt nach Anspruch 6, 7 oder 8 oder in Gegenwart eines rekombinanten Mikroorganismus nach einem der Ansprüche 1 bis 5 umsetzt, und wenigstens ein gebildetes Oxidationsprodukt aus dem Reaktionsansatz gegebenenfalls isoliert.

10. Verfahren nach Anspruch 9, wobei das Hydroxysteroid Cholsäure (CS) oder ein Cholsäurederivat, wie insbesondere ein Salz, Amid oder Alkylester, ist.

11. Verfahren nach Anspruch 10, wobei CS oder ein Derivat davon zu 12-Ketochenodesoxycholsäure (12-Keto-CDCS) oder zum entsprechenden Derivat umgesetzt wird.

12. Verfahren zur Herstellung von Ursodesoxycholsäure (UDCS) der Formel (1) 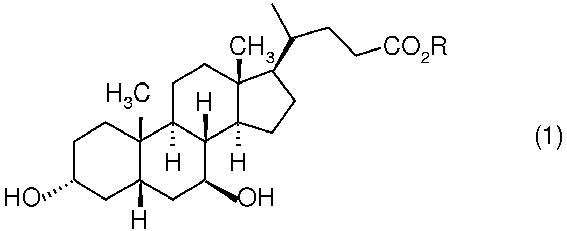 worin
R für Alkyl, H, ein Alkalimetallion oder N(R³)₄⁺steht, worin die Reste R³ gleich oder verschieden sind und für H oder Alkyl stehen,
wobei man
a) eine Cholsäure (CS) der Formel (2) 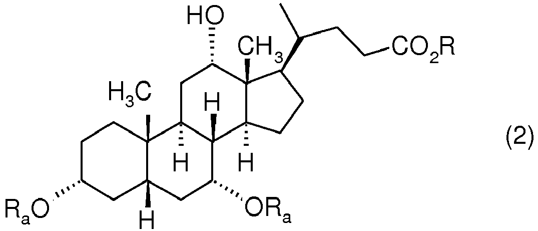 worin R die oben angegebenen Bedeutungen besitzt, und die Reste Rₐ gleich oder verschieden sind und für H oder Acyl stehen, in Gegenwart einer 12α-HSDH hergestellt nach Anspruch 6, 7 oder 8 oder in Gegenwart eines rekombinanten Mikroorganismus nach einem der Ansprüche 1 bis 5 zur korrespondierenden 12-Ketochenodesoxycholsäure (12-Keto CDCS) der Formel (3) 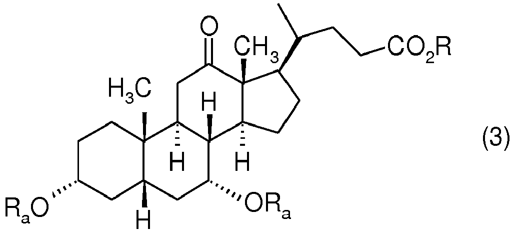 worin R und Rₐ die oben angebebenen Bedeutungen besitzen, oxidiert und anschließend
b) 12-Keto-CDCS der Formel (3) durch Desoxygenierung zu Chenodesoxycholsäure (CDCS) der Formel (4) 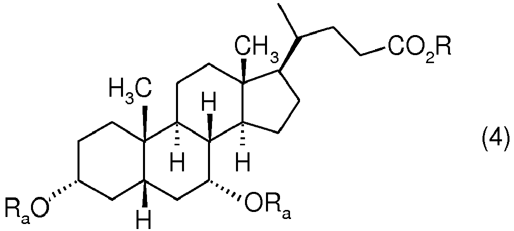 worin R und Rₐ die oben angegebenen Bedeutungen besitzen, umsetzt
c) CDCS der Formel (4) in Position 7 chemisch oxidiert zur 7-Keto-Lithocholsäure (KLCS) der Formel (5) 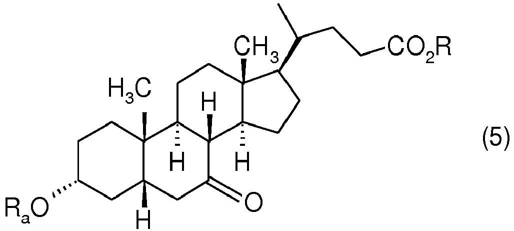 worin R und Rₐ die oben angegebenen Bedeutungen besitzen;
d) KLCS der Formel (5) reduziert und
e) das Reaktionsprodukt gegebenenfalls weiter aufreinigt.

13. Verfahren zur selektiven enzymatischen Reduktion von Ketosteroiden, welche neben wenigstens einer Ketogruppe in Position 3, 11, 12, 17, oder 20 des Steroidgerüsts, wenigstens eine weitere Ketogruppe in Position 7 des Steroidgerüsts aufweisen, wobei man das Ketosteroid in Gegenwart einer 3α-, 3β-, 7β-, 11α-, 11 β-, 12α-, 12β-, 17α-, 17β-, 20α-, oder 20β-HSDH, hergestellt nach Anspruch 6, 7 oder 8, oder in Gegenwart eines rekombinanten Mikroorganismus nach einem der Ansprüche 1 bis 5 umsetzt, und wenigstens ein gebildetes Reduktionsprodukt aus dem Reaktionsansatz gegebenenfalls isoliert.

14. Verfahren nach einem der Ansprüche 9 bis 13, wobei wenigstens eine, die gewünschte Reaktion katalysierende HSDH in gelöster, dispergierter oder immobilisierter Form eingesetzt wird; oder wobei das Verfahren in Gegenwart ganzer, gegebenenfalls immobilisierter Zellen eines rekombinanten Mikroorganismus nach einem der Ansprüche 1 bis 5 durchgeführt wird.

15. Verfahren zur Herstellung von Ursodesoxycholsäure (UDCS) der Formel (1) 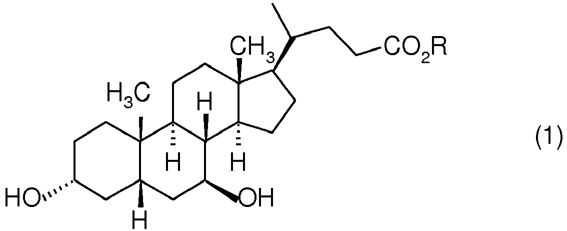 worin R für Alkyl, H, ein Alkalimetallion oder N(R³)₄⁺steht, worin die Reste R³ gleich oder verschieden sind und für H oder Alkyl stehen, wobei man
a) gegebenenfalls eine Cholsäure (CS) der Formel (2) 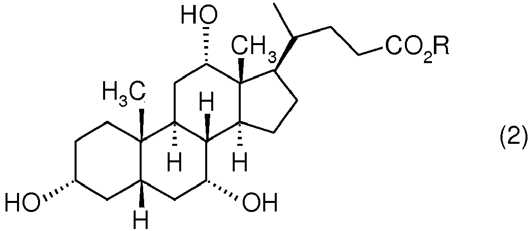 worin R die oben angegebenen Bedeutungen besitzt, zur Dehydrocholsäure (DHCS) der Formel (3) 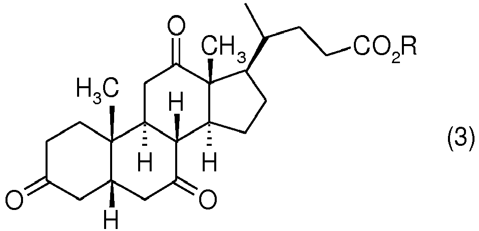 worin R die oben angegebenen Bedeutungen besitzt, chemisch oxidiert;
b) DHCS mit einer 7β-HSDH und einer 3α-HSDH hergestellt nach einem der Ansprüche 6, 7 oder 8 in beliebiger Reihenfolge oder in gleichzeitiger Gegenwart beider Enzyme zur korrespondierenden 12-Keto-ursodesoxycholsäure (12-Keto UDCS) der Formel (5) 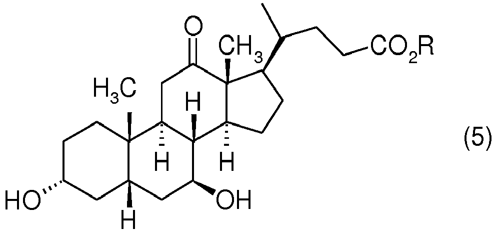 worin R die oben angegebenen Bedeutungen besitzt, reduziert und anschließend
d) 12-Keto-UDCS der Formel (5) chemisch zu UDCS reduziert; und
e) das Reaktionsprodukt gegebenenfalls weiter aufreinigt.

16. Verfahren nach einem der Ansprüche 9 bis 15, wobei der (die) enzymatische(n) Redox-Schritt(e) mit einem, insbesondere enzymatischen, Cofaktorregenerierungsschritt gekoppelt ist (sind).

## Claims

1. Recombinant microorganism in which the enzymatic activity of 7α-hydroxysteroid dehydrogenase (7α-HSDH) is fully inhibited while the enzymatic activity of a hydroxysteroid dehydrogenase functionally different therefrom is present in expressible form wherein the recombinant microorganism is derived from a 7α-HSDH-expressing precursor microorganism.

2. Recombinant microorganism according to claim 1, wherein the 7α-HSDH-encoding nucleic acid sequence is knocked out by homologous recombination or by gene disruption.

3. Recombinant microorganism according to any of the preceding claims, which recombinantly expresses the HSDH functionally different from said 7α-HSDH.

4. Recombinant microorganism according to any of claims 2 and 3, wherein the knocked-out 7α-HSDH has an amino acid sequence according to SEQ ID NO: 10 or is encoded by a nucleic acid sequence according to SEQ ID NO: 9.

5. Recombinant microorganism according to any of the preceding claims, wherein the expressed HSDH functionally different from said 7α-HSDH has any of the following amino acid sequences:
a) a 3α-HSDH sequence selected from among SEQ ID NO: 29 and 31
b) a 7β-HSDH sequence selected from among SEQ ID NO: 25
c) a 12α-HSDH sequence selected from among SEQ ID NO: 12, 14, and 16; or
d) an HSDH-encoding amino acid sequence derived therefrom with a degree of identity of at least 50% to one of the sequences of a), b) and c).

6. Process for the recombinant production of the desired HSDH functionally different from 7α-HSDH, wherein a recombinant microorganism according to any of the preceding claims, in which the enzymatic activity of 7α-HSDH is fully inhibited, is cultured under conditions expressing said desired HSDH and the expressed HSDH is isolated, wherein in said isolated HSDH essentially no functional 7α-HSDH is detectable.

7. Process according to claim 6, wherein the desired recombinant HSDH is selected from among a 3α-, 3β-, 7β-, 11α-, 11β-, 12α-, 12β-, 17α-, 17β-, 20α-, or 20β-HSDH.

8. Process according to claim 7, wherein the desired recombinant HSDH includes any of the following amino acid sequences:
a) a 3α-HSDH sequence selected from among SEQ ID NO: 29 and 31
b) a 7β-HSDH sequence selected from among SEQ ID NO: 25
c) a 12α-HSDH sequence selected from among SEQ ID NO: 12, 14, and 16; or
d) an amino acid sequence derived therefrom with a degree of identity of at least 50% to one of the sequences of a), b) and c).

9. Process for the selective enzymatic oxidation of hydroxysteroids which, besides a hydroxyl group at least at any of the 3α-, 3β-, 11α-, 11β-, 12α-, 12β-, 17α-, 17β-, 20α-, or 20β-positions of the steroid skeleton, have at least one further hydroxyl group at the 7α-position of the steroid skeleton, wherein the hydroxysteroid is reacted in the presence of a 3α-, 3β-, 11α-, 11β-, 12α-, 12β-, 17α-, 17β-, 20α-, or 20β-HSDH prepared according to claim 6, 7, or 8, or in the presence of a recombinant microorganism according to any of claims 1 to 5 and, optionally, at least one oxidation product formed is isolated from the reaction mixture.

10. Process according to claim 9, wherein the hydroxysteroid is cholic acid (CA) or a cholic acid derivative such as, in particular, a salt, amide or alkyl ester.

11. Process according to claim 10, wherein CA or a derivative thereof is reacted to give 12-ketochenodeoxycholic acid (12-keto-CDCA) or the corresponding derivative.

12. Process for the production of ursodeoxycholic acid (UDCA) of the formula (1) 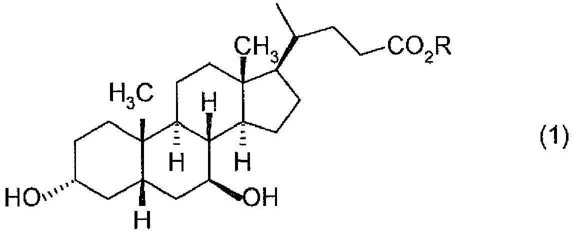 in which
R represents alkyl, H, an alkali metal ion or N(R³)₄⁺, in which the radicals R³ are identical or different and represent H or alkyl,
wherein
a) a cholic acid (CA) of the formula (2) 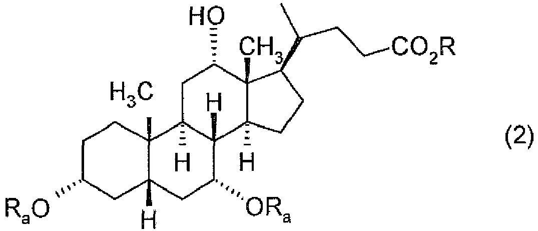 in which R have the abovementioned meanings and the radicals Rₐ are identical or different and represent H or acyl, are oxidized in the presence of a 12α-HSDH according to claim 6, 7, or 8, or in the presence of a recombinant microorganism according to any of claims 1 to 5 to give the corresponding 12-ketochenodeoxycholic acid (12-keto-CDCA) of the formula (3) 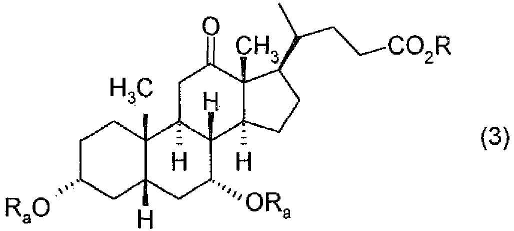 in which R and Rₐ have the abovementioned meanings, and, subsequently
b) 2-keto-CDCA of the formula (3) is reacted by deoxygenation to give chenodeoxycholic acid (CDCA) of the formula (4) 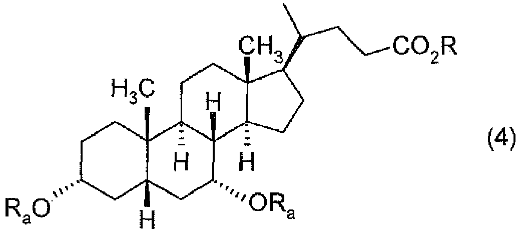 in which R and Rₐ have the abovementioned meanings,
c) CDCA of the formula (4) is oxidized chemically at the 7-position to give 7-keta-lithocholic acid (KLCA) of the formula (5) 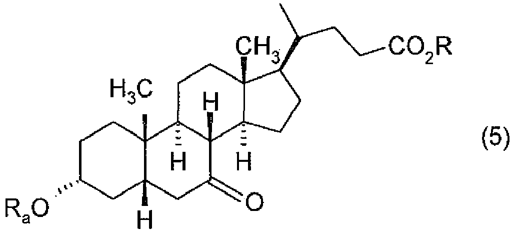 in which R and Rₐ have the abovementioned meanings;
d) KLCA of the formula (5) is reduced, and
e) the reaction product is optionally purified further.

13. Process for the selective enzymatic reduction of ketosteroids which, besides at least a keto group at the 3-, 11-, 12-, 17-, or 20-position of the steroid skeleton, have at least one further keto group at the 7-position of the steroid skeleton, wherein the ketosteroid is reacted in the presence of a 3α-, 3β-, 7β-, 11α-, 11β-, 12α-, 12β-, 17α-, 17β, 20α-, or 20β-HSDH prepared according to claim 6, 7, or 8, or in the presence of a recombinant microorganism according to any of claims 1 to 5 and, optionally, at least one reduction product formed is isolated from the reaction mixture.

14. Process according to any of claims 9 to 13, wherein at least one of the desired reaction-catalysing HSDH is employed in dissolved, dispersed or immobilized form; or wherein the process is carried out in the presence of intact, optionally immobilized cells of a recombinant microorganism according to any of claims 1 to 5.

15. Process for the production of ursodeoxycholic acid (UDCA) of the formula (1) 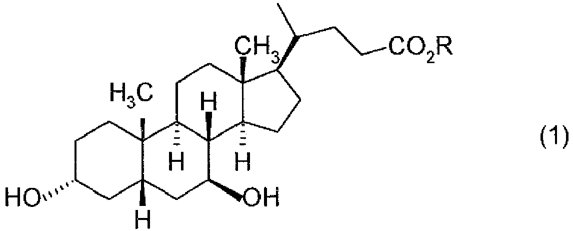 in which
R represents alkyl, H, an alkali metal ion or N(R³)₄⁺, in which the radicals R³ are identical or different and represent H or alkyl,
wherein
a) optionally, a cholic acid (CA) of the formula (2) 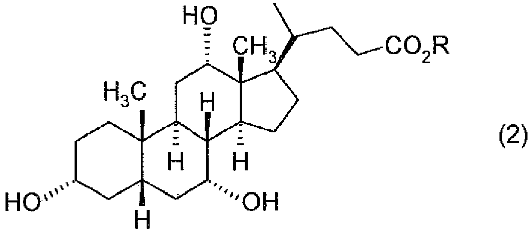 in which R has the abovementioned meanings, are chemically oxidized to give the dehydrocholic acid (DHCA) of the formula (3) 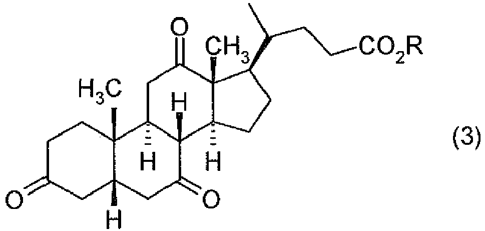 in which R has the abovementioned meanings;
b) DHCA is reduced by a 7β-HSDH and a 3α-HSDH prepared according to any of claim 6, 7, or 8 in any order or in the simultaneous presence of both enzymes to give the corresponding 12-keto ursodeoxycholic acid (12-Keto UDCA) of the formula (5) 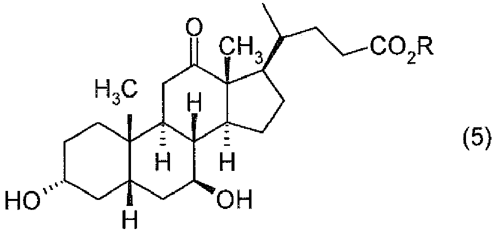 in which R has the abovementioned meanings; and subsequently
d) 12-Keto UDCA of the formula (5) is chemically reduced to give UDCA; and
e) the reaction product is optionally purified further.

16. Process according to any of claims 9 to 15, wherein the enzymatic redox step(s) is (are) coupled with a, in particular enzymatic, cofactor regeneration step.

## Revendications

1. Micro-organisme recombinant, dans lequel l'activité enzymatique de la 7α-hydroxystéroïde-déshydrogénase (7α-HSDH) est complètement inhibée, alors que l'activité enzymatique d'une hydroxystéroïde-déshydrogénase fonctionnellement différente de celle-ci est contenue de manière exprimable, le micro-organisme recombinant étant dérivé d'un micro-organisme précurseur exprimant la 7α-HSDH.

2. Micro-organisme recombinant selon la revendication 1, la séquence d'acide nucléique codant pour la 7α-HSDH étant désactivée par recombinaison homologue ou par disruption génique.

3. Micro-organisme recombinant selon l'une quelconque des revendications précédentes, qui exprime la HSDH fonctionnellement différente de la 7α-HSDH de manière recombinante.

4. Micro-organisme recombinant selon l'une quelconque des revendications 2 et 3, la 7α-HSDH désactivée présentant une séquence d'acides aminés selon la séquence SEQ ID NO : 10 ou étant codée par une séquence d'acide nucléique selon la séquence SEQ ID NO : 9.

5. Micro-organisme recombinant selon l'une quelconque des revendications précédentes, la HSDH exprimée, fonctionnellement différente de la 7α-HSDH, présentant une des séquences d'acides aminés suivantes :
a) une séquence de 3α-HSDH choisie parmi les séquences SEQ ID NO : 29 et 31
b) une séquence de 7β-HSDH choisie parmi la séquence SEQ ID NO : 25
c) une séquence de 12α-HSDH choisie parmi les séquences SEQ ID NO: 12, 14 et 16 ; ou
d) une séquence d'acides aminés dérivée de celles-ci, codant pour une HSDH, présentant un degré d'identité d'au moins 50% par rapport à l'une des séquences reprises sous a), b) et c).

6. Procédé pour la préparation recombinante d'une HSDH souhaitée, fonctionnellement différente de la 7α-HSDH, un micro-organisme recombinant selon l'une quelconque des revendications précédentes, dans lequel l'activité enzymatique de la 7α-HSDH est complètement inhibée, étant cultivé dans des conditions dans lesquelles la HSDH souhaitée est exprimée et la HSDH ainsi exprimée étant isolée, une 7α-HSDH fonctionnelle n'étant essentiellement plus détectable dans la HSDH isolée.

7. Procédé selon la revendication 6, la HSDH recombinante souhaitée étant choisie parmi une 3α-HSDH, une 3β-HSDH, une 7β-HSDH, une 11α-HSDH, une 11β-HSDH, une 12α-HSDH, une 12β-HSDH, une 17α-HSDH, une 17β-HSDH, une 20α-HSDH ou une 20β-HSDH.

8. Procédé selon la revendication 7, la HSDH recombinante souhaitée comprenant une des séquences d'acides aminés suivantes :
a) une séquence de 3α-HSDH choisie parmi les séquences SEQ ID NO : 29 et 31
b) une séquence de 7β-HSDH choisie parmi la séquence SEQ ID NO : 25
c) une séquence de 12α-HSDH choisie parmi les séquences SEQ ID NO: 12, 14 et 16 ; ou
d) une séquence d'acides aminés dérivée de celles-ci, présentant un degré d'identité d'au moins 50% par rapport à l'une des séquences reprises sous a), b) et c).

9. Procédé pour l'oxydation enzymatique sélective d'hydroxystéroïdes qui présentent, outre un groupe hydroxy dans au moins une des positions 3α, 3β, 11α, 11β, 12β, 12β, 17α, 17β, 20α ou 20β de la structure stéroïde, au moins un autre groupe hydroxy en position 7α de la structure stéroïde, l'hydroxystéroïde étant transformé en présence d'une 3α-HSDH, d'une 3β-HSDH, d'une 11α-HSDH, d'une 11β-HSDH, d'une 12α-HSDH, d'une 12β-HSDH, d'une 17α-HSDH, d'une 17β-HSDH, d'une 20α-HSDH ou d'une 20β-HSDH, préparée selon la revendication 6, 7 ou 8 ou en présence d'un micro-organisme recombinant selon l'une quelconque des revendications 1 à 5 et au moins un produit d'oxydation formé étant le cas échéant isolé de la charge réactionnelle.

10. Procédé selon la revendication 9, l'hydroxystéroïde étant l'acide cholique (AC) ou un dérivé de l'acide cholique, tel qu'en particulier un sel, un amide ou un ester d'alkyle.

11. Procédé selon la revendication 10, l'AC ou un dérivé de celui-ci étant transformé en acide 12-cétochénodésoxycholique (acide 12-céto-CDC) ou en dérivé correspondant.

12. Procédé pour la préparation d'acide ursodésoxycholique (AUDC) de formule (1) 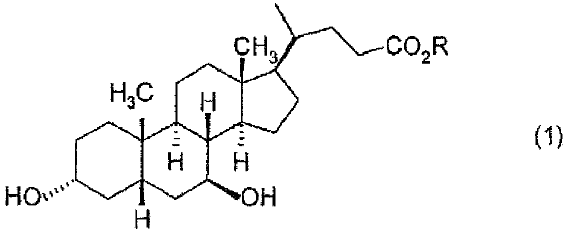 dans laquelle
R représente alkyle, H, un ion de métal alcalin ou N(R³)₄⁺, les radicaux R³ étant identiques ou différents et représentant H ou alkyle,
dans lequel
a) on oxyde un acide cholique (AC) de formule (2) 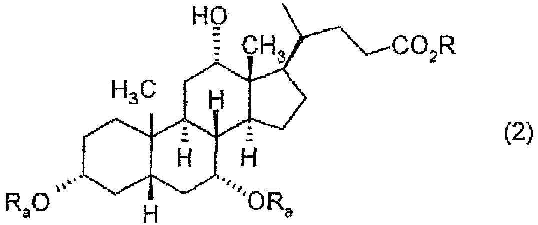 dans laquelle R présente les significations indiquées ci-dessus et les radicaux Rₐ sont identiques ou différents et représentent H ou acyle, en présence d'une 12α-HSDH préparée selon la revendication 6, 7 ou 8 ou en présence d'un micro-organisme recombinant selon l'une quelconque des revendications 1 à 5 en acide 12-cétochénodésoxycholique (acide 12-céto-CDC) de formule (3) 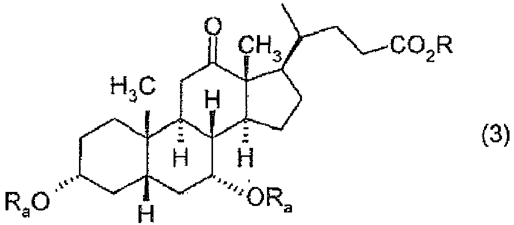 dans laquelle R et Rₐ présentent les significations indiquées ci-dessus et ensuite
b) on transforme l'acide 12-céto-CDC de formule (3) par désoxygénation en acide chténodésoxycholique (ACDC) de formule (4) 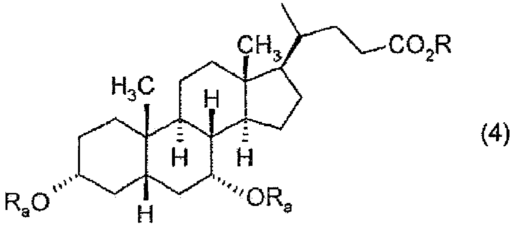 dans laquelle R et Rₐ présentent les significations indiquées ci-dessus et
c) on oxyde chimiquement l'ACDC de formule (4) en position 7 en acide 7-céto-lithocholique (ACLC) de formule (5) 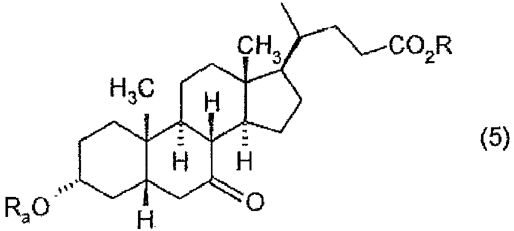 dans laquelle R et Rₐ présentent les significations indiquées ci-dessus
d) on réduit l'ACLC de formule (5) et
e) on purifie le cas échéant davantage le produit de réaction.

13. Procédé pour la réduction enzymatique sélective de cétostéroïdes qui présentent, outre au moins un groupe céto en position 3, 11, 12, 17 ou 20 de la structure stéroïde, au moins un autre groupe céto en position 7 de la structure stéroïde, le cétostéroïde étant transformé en présence d'une 3α-HSDH, d'une 3β-HSDH, d'une 7β-HSDH, d'une 11α-HSDH, d'une 11β-HSDH, d'une 12α-HSDH, d'une 12β-HSDH, d'une 17α-HSDH, d'une 17β-HSDH, d'une 20α-HSDH ou d'une 20β-HSDH, préparée selon la revendication 6, 7 ou 8 ou en présence d'un micro-organisme recombinant selon l'une quelconque des revendications 1 à 5 et au moins un produit de réduction formé étant le cas échéant isolé de la charge réactionnelle.

14. Procédé selon l'une quelconque des revendications 9 à 13, au moins une HSDH catalysant la réaction souhaitée étant utilisée sous forme dissoute, dispersée ou immobilisée ; ou le procédé étant réalisé en présence de cellules entières, le cas échéant immobilisées d'un micro-organisme recombinant selon l'une quelconque des revendications 1 à 5.

15. Procédé pour la préparation d'acide ursodésoxycholique (AUDC) de formule (1) 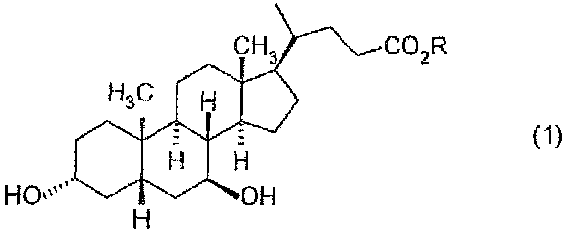 dans laquelle
R représente alkyle, H, un ion de métal alcalin ou N(R³)₄⁺, les radicaux R³ étant identiques ou différents et représentant H ou alkyle, dans lequel
a) on oxyde chimiquement le cas échéant un acide cholique (AC) de formule (2) 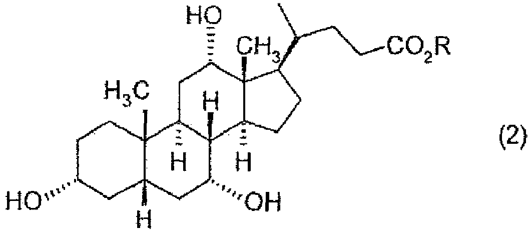 dans laquelle R présente les significations indiquées ci-dessus en acide déshydrocholique (ADHC) de formule (3) 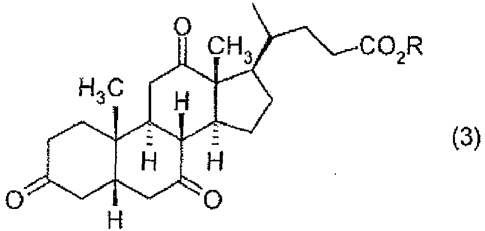 dans laquelle R présente les significations indiquées ci-dessus ;
b) on réduit l'ADHC à l'aide d'une 7β-HSDH et d'une 3α-HSDH préparées selon l'une quelconque des revendications 6, 7 ou 8 dans un ordre quelconque ou en présence simultanée des deux enzymes en acide 12-céto-ursodésoxycholique correspondant (acide 12-céto-UDC) de formule (5) 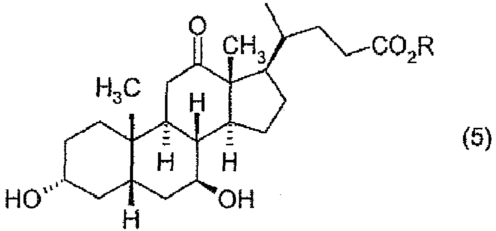 dans laquelle R présente les significations indiquées ci-dessus, puis
d) on réduit chimiquement l'acide 12-céto-UDC de formule (5) en AUDC ; et
e) on purifie le cas échéant davantage le produit de réaction.

16. Procédé selon l'une quelconque des revendications 9 à 15, la/les étape(s) redox enzymatique(s) étant couplée(s) à une étape de régénération de cofacteur, en particulier enzymatique.


### SEQUENCE LISTING

<110> PHARMAZEILL GmbH
<120> 7alpha-Hydroxysterioddehydrogenase-Knock-Out-Mutanten
<130> M/50273-PCT
<160> 38
<170> PatentIn version 3.3
<210> 1
   <211> 4334
   <212> DNA
   <213> Artificial
<220>
   <223> Plasmid
<400> 1
   gtgtaggctg gagctgcttc gaagttccta tactttctag agaataggaa cttcggaata      60
   ggaacttatg agctcagcca atcgactggc gagcggcatc gcattcttcg catcccgcct     120
   ctggcggatg caggaagatc aacggatctc ggcccagttg acccagggct gtcgccacaa     180
   tgtcgcggga gcggatcaac cgagcaaagg catgaccgac tggaccttcc ttctgaaggc     240
   tcttctcctt gagccacctg tccgccaagg caaagcgctc acagcagtgg tcattctcga     300
   gataatcgac gcgtaccaac ttgccatcct gaagaatggt gcagtgtctc ggcaccccat     360
   agggaacctt tgccatcaac tcggcaagat gcagcgtcgt gttggcatcg tgtcccacgc     420
   cgaggagaag tacctgccca tcgagttcat ggacacgggc gaccgggctt gcaggcgagt     480
   gaggtggcag ggcaatggat tcagagatga tctgctctgc ctgtggcccc gctgccgcaa     540
   aggcaaatgg atgggcgctg cgctttacat ttggcaggcg ccagaatgtg tcagagacaa     600
   ctccaaggtc cggtgtaacg ggcgacgtgg caggatcgaa cggctcgtcg tccagacctg     660
   accacgaggg catgacgagc gtccctcccg gacccagcgc agcacgcagg gcctcgatca     720
   gtccaagtgg cccatcttcg aggggccgga cgctacggaa ggagctgtgg accagcagca     780
   caccgccggg ggtaacccca aggttgagaa gctgaccgat gagctcggct tttcgccatt     840
   cgtattgcac gacattgcac tccaccgctg atgacatcag tcgatcatag cacgatcaac     900
   ggcactgttg caaatagtcg gtggtgataa acttatcatc cccttttgct gatggagctg     960
   cacatgaacc cattcaaagg ccggcatttt cagcgtgaca tcattctgtg ggccgtacgc    1020
   tggtactgca aatacggcat cagttaccgt gagctgcatt ttccgctgca taaccctgct    1080
   tcggggtcat tatagcgatt ttttcggtat atccatcctt tttcgcacga tatacaggat    1140
   tttgccaaag ggttcgtgta gactttcctt ggtgtatcca acggcgtcag ccgggcagga    1200
   taggtgaagt aggcccaccc gcgagcgggt gttccttctt cactgtccct tattcgcacc    1260
   tggcggtgct caacgggaat cctgctctgc gaggctggcg ggaacttcga agttcctata    1320
   ctttctagag aataggaact tcgaactgca ggtcgacgga tccccggaat atcaagctta 1380
   tcgataccgt cgacctcgag ggggggcccg gtacccaatt cgccctatag tgagtcgtat 1440
   tacaattcac tggccgtcgt tttacaacgt cgtgactggg aaaaccctgg cgttacccaa 1500
   cttaatcgcc ttgcagcaca tccccctttc gccagctggc gtaatagcga agaggcccgc 1560
   accgatcgcc cttcccaaca gttgcgcagc ctgaatggcg aatggcgcga cgcgccctgt 1620
   agcggcgcat taagcgcggc gggtgtggtg gttacgcgca gcgtgaccgc tacacttgcc 1680
   agcgccctag cgcccgctcc tttcgctttc ttcccttcct ttctcgccac gttcgccggc 1740
   tttccccgtc aagctctaaa tcgggggctc cctttagggt tccgatttag tgctttacgg 1800
   cacctcgacc ccaaaaaact tgattagggt gatggttcac gtagtgggcc atcgccctga 1860
   tagacggttt ttcgcccttt gacgttggag tccacgttct ttaatagtgg actcttgttc 1920
   caaactggaa caacactcaa ccctatctcg gtctattctt ttgatttata agggattttg 1980
   ccgatttcgg cctattggtt aaaaaatgag ctgatttaac aaaaatttaa cgcgaatttt 2040
   aacaaaatat taacgtttac aatttcccag gtggcacttt tcggggaaat gtgcgcggaa 2100
   cccctatttg tttatttttc taaatacatt caaatatgta tccgctcatg agacaataac 2160
   cctgataaat gcttcaataa tattgaaaaa ggaagagtat gagtattcaa catttccgtg 2220
   tcgcccttat tccctttttt gcggcatttt gccttcctgt ttttgctcac ccagaaacgc 2280
   tggtgaaagt aaaagatgct gaagatcagt tgggtgcacg agtgggttac atcgaactgg 2340
   atctcaacag cggtaagatc cttgagagtt ttcgccccga agaacgtttt ccaatgatga 2400
   gcacttttaa agttctgcta tgtggcgcgg tattatcccg tattgacgcc gggcaagagc 2460
   aactcggtcg ccgcatacac tattctcaga atgacttggt tgagtactca ccagtcacag 2520
   aaaagcatct tacggatggc atgacagtaa gagaattatg cagtgctgcc ataaccatga 2580
   gtgataacac tgcggccaac ttacttctga caacgatcgg aggaccgaag gagctaaccg 2640
   cttttttgca caacatgggg gatcatgtaa ctcgccttga tcgttgggaa ccggagctga 2700
   atgaagccat accaaacgac gagcgtgaca ccacgatgcc tgtagcaatg gcaacaacgt 2760
   tgcgcaaact attaactggc gaactactta ctctagcttc ccggcaacaa ttaatagact 2820
   ggatggaggc ggataaagtt gcaggaccac ttctgcgctc ggcccttccg gctggctggt 2880
   ttattgctga taaatctgga gccggtgagc gtgggtctcg cggtatcatt gcagcactgg 2940
   ggccagatgg taagccctcc cgtatcgtag ttatctacac gacggggagt caggcaacta 3000
   tggatgaacg aaatagacag atcgctgaga taggtgcctc actgattaag cattggtaac 3060
   tgtcagacca agtttactca tatatacttt agattgattt aaaacttcat ttttaattta 3120
   aaaggatcta ggtgaagatc ctttttgata atctcatgac caaaatccct taacgtgagt 3180
   tttcgttcca ctgagcgtca gaccccgtag aaaagatcaa aggatcttct tgagatcctt 3240
   tttttctgcg cgtaatctgc tgcttgcaaa caaaaaaacc accgctacca gcggtggttt 3300
   gtttgccgga tcaagagcta ccaactcttt ttccgaaggt aactggcttc agcagagcgc 3360
   agataccaaa tactgtcctt ctagtgtagc cgtagttagg ccaccacttc aagaactctg 3420
   tagcaccgcc tacatacctc gctctgctaa tcctgttacc agtggctgct gccagtggcg 3480
   ataagtcgtg tcttaccggg ttggactcaa gacgatagtt accggataag gcgcagcggt 3540
   cgggctgaac ggggggttcg tgcacacagc ccagcttgga gcgaacgacc tacaccgaac 3600
   tgagatacct acagcgtgag ctatgagaaa gcgccacgct tcccgaaggg agaaaggcgg 3660
   acaggtatcc ggtaagcggc agggtcggaa caggagagcg cacgagggag cttccagggg 3720
   gaaacgcctg gtatctttat agtcctgtcg ggtttcgcca cctctgactt gagcgtcgat 3780
   ttttgtgatg ctcgtcaggg gggcggagcc tatggaaaaa cgccagcaac gcggcctttt 3840
   tacggttcct ggccttttgc tggccttttg ctcacatgtt ctttcctgcg ttatcccctg 3900
   attctgtgga taaccgtatt accgcctttg agtgagctga taccgctcgc cgcagccgaa 3960
   cgaccgagcg cagcgagtca gtgagcgagg aagcggaaga gcgcccaata cgcaaaccgc 4020
   ctctccccgc gcgttggccg attcattaat gcagctggca cgacaggttt cccgactgga 4080
   aagcgggcag tgagcgcaac gcaattaatg tgagttagct cactcattag gcaccccagg 4140
   ctttacactt tatgcttccg gctcgtatgt tgtgtggaat tgtgagcgga taacaatttc 4200
   acacaggaaa cagctatgac catgattacg ccaagctcgg aattaaccct cactaaaggg 4260
   aacaaaagct ggagctccac cgcggtggcg gccgctctag aactagtgga tcccccgggc 4320
   tgcaggaatt cgat 4334
<210> 2
   <211> 7220
   <212> DNA
   <213> Artificial
<220>
   <223> Plasmid
<400> 2
   catcgattta ttatgacaac ttgacggcta catcattcac tttttcttca caaccggcac     60
   ggaactcgct cgggctggcc ccggtgcatt ttttaaatac ccgcgagaaa tagagttgat    120
   cgtcaaaacc aacattgcga ccgacggtgg cgataggcat ccgggtggtg ctcaaaagca    180
   gcttcgcctg gctgatacgt tggtcctcgc gccagcttaa gacgctaatc cctaactgct    240
   ggcggaaaag atgtgacaga cgcgacggcg acaagcaaac atgctgtgcg acgctggcga    300
   tatcaaaatt gctgtctgcc aggtgatcgc tgatgtactg acaagcctcg cgtacccgat    360
   tatccatcgg tggatggagc gactcgttaa tcgcttccat gcgccgcagt aacaattgct    420
   caagcagatt tatcgccagc agctccgaat agcgcccttc cccttgcccg gcgttaatga    480
   tttgcccaaa caggtcgctg aaatgcggct ggtgcgcttc atccgggcga aagaaccccg    540
   tattggcaaa tattgacggc cagttaagcc attcatgcca gtaggcgcgc ggacgaaagt 600
   aaacccactg gtgataccat tcgcgagcct ccggatgacg accgtagtga tgaatctctc 660
   ctggcgggaa cagcaaaata tcacccggtc ggcaaacaaa ttctcgtccc tgatttttca 720
   ccaccccctg accgcgaatg gtgagattga gaatataacc tttcattccc agcggtcggt 780
   cgataaaaaa atcgagataa ccgttggcct caatcggcgt taaacccgcc accagatggg 840
   cattaaacga gtatcccggc agcaggggat cattttgcgc ttcagccata cttttcatac 900
   tcccgccatt cagagaagaa accaattgtc catattgcat cagacattgc cgtcactgcg 960
   tcttttactg gctcttctcg ctaaccaaac cggtaacccc gcttattaaa agcattctgt 1020
   aacaaagcgg gaccaaagcc atgacaaaaa cgcgtaacaa aagtgtctat aatcacggca 1080
   gaaaagtcca cattgattat ttgcacggcg tcacactttg ctatgccata gcatttttat 1140
   ccataagatt agcggatcct acctgacgct ttttatcgca actctctact gtttctccat 1200
   acccgttttt ttgggaattc gagctctaag gaggttataa aaaatggata ttaatactga 1260
   aactgagatc aagcaaaagc attcactaac cccctttcct gttttcctaa tcagcccggc 1320
   atttcgcggg cgatattttc acagctattt caggagttca gccatgaacg cttattacat 1380
   tcaggatcgt cttgaggctc agagctgggc gcgtcactac cagcagctcg cccgtgaaga 1440
   gaaagaggca gaactggcag acgacatgga aaaaggcctg ccccagcacc tgtttgaatc 1500
   gctatgcatc gatcatttgc aacgccacgg ggccagcaaa aaatccatta cccgtgcgtt 1560
   tgatgacgat gttgagtttc aggagcgcat ggcagaacac atccggtaca tggttgaaac 1620
   cattgctcac caccaggttg atattgattc agaggtataa aacgaatgag tactgcactc 1680
   gcaacgctgg ctgggaagct ggctgaacgt gtcggcatgg attctgtcga cccacaggaa 1740
   ctgatcacca ctcttcgcca gacggcattt aaaggtgatg ccagcgatgc gcagttcatc 1800
   gcattactga tcgttgccaa ccagtacggc cttaatccgt ggacgaaaga aatttacgcc 1860
   tttcctgata agcagaatgg catcgttccg gtggtgggcg ttgatggctg gtcccgcatc 1920
   atcaatgaaa accagcagtt tgatggcatg gactttgagc aggacaatga atcctgtaca 1980
   tgccggattt accgcaagga ccgtaatcat ccgatctgcg ttaccgaatg gatggatgaa 2040
   tgccgccgcg aaccattcaa aactcgcgaa ggcagagaaa tcacggggcc gtggcagtcg 2100
   catcccaaac ggatgttacg tcataaagcc atgattcagt gtgcccgtct ggccttcgga 2160
   tttgctggta tctatgacaa ggatgaagcc gagcgcattg tcgaaaatac tgcatacact 2220
   gcagaacgtc agccggaacg cgacatcact ccggttaacg atgaaaccat gcaggagatt 2280
   aacactctgc tgatcgccct ggataaaaca tgggatgacg acttattgcc gctctgttcc 2340
   cagatatttc gccgcgacat tcgtgcatcg tcagaactga cacaggccga agcagtaaaa 2400
   gctcttggat tcctgaaaca gaaagccgca gagcagaagg tggcagcatg acaccggaca 2460
   ttatcctgca gcgtaccggg atcgatgtga gagctgtcga acagggggat gatgcgtggc 2520
   acaaattacg gctcggcgtc atcaccgctt cagaagttca caacgtgata gcaaaacccc 2580
   gctccggaaa gaagtggcct gacatgaaaa tgtcctactt ccacaccctg cttgctgagg 2640
   tttgcaccgg tgtggctccg gaagttaacg ctaaagcact ggcctgggga aaacagtacg 2700
   agaacgacgc cagaaccctg tttgaattca cttccggcgt gaatgttact gaatccccga 2760
   tcatctatcg cgacgaaagt atgcgtaccg cctgctctcc cgatggttta tgcagtgacg 2820
   gcaacggcct tgaactgaaa tgcccgttta cctcccggga tttcatgaag ttccggctcg 2880
   gtggtttcga ggccataaag tcagcttaca tggcccaggt gcagtacagc atgtgggtga 2940
   cgcgaaaaaa tgcctggtac tttgccaact atgacccgcg tatgaagcgt gaaggcctgc 3000
   attatgtcgt gattgagcgg gatgaaaagt acatggcgag ttttgacgag atcgtgccgg 3060
   agttcatcga aaaaatggac gaggcactgg ctgaaattgg ttttgtattt ggggagcaat 3120
   ggcgatgata agctagctta aaaaagcaaa agggccgcag atgcgaccct tgtgtatcaa 3180
   acaagacgat taaaaatctt cgttagtttc tgctacgcct tcgctatcat ctacagagaa 3240
   atccggcgtt gagttcgggt tgctcagcag caactcacgt actttcttct cgatctcttt 3300
   cgcggtttcc gggttatctt tcagccaggc agtcgcattc gctttaccct gaccgatctt 3360
   ctcacctttg tagctgtacc acgcgcctgc tttctcgatc agcttctctt ttacgcccag 3420
   gtcaaccagt tcgccgtaga agttgatacc ttcgccgtag aggatctgga attcagcctg 3480
   tttaaacggc gcagcgattt tgttcttcac cactttcacg cgggtttcgc tacccaccac 3540
   gttttcgccc tctttcaccg cgccgatacg acggatgtcg agacgaacag aggcgtagaa 3600
   tttcagcgcg ttaccaccgg tagtggtttc cgggttaccg aacatcacac caattttcat 3660
   acggatctgg ttgatgaaga tcagcagcgt gttggactgc ttcaggttac ccgccagctt 3720
   acgcatcgcc tggctcatca tacgtgccgc aaggcccatg tgagagtcgc cgatttcgcc 3780
   ttcgatttcc gctttcggcg tcagtgccgc cacggagtca acgacgataa cgtctactgc 3840
   gccagaacgc gccagggcgt cacagatttc cagtgcctgc tcgccggtgt ccggctggga 3900
   gcacagcagg ttgtcgatat cgacgcccag tttacgtgcg tagattgggt ccagcgcgtg 3960
   ttcagcatcg ataaacgcac aggttttacc ttcacgctgc gctgcggcga tcacctgcag 4020
   cgtcagcgtg gttttaccgg aagattccgg tccgtagatt tcgacgatac ggcccatcgg 4080
   cagaccacct gccccaagcg cgatatccag tgaaagcgaa ccggtagaga tggtttccac 4140
   atccatggaa cggtcttcac ccaggcgcat gatggagcct ttaccaaatt gtttctcaat 4200
   ctggcccagt gctgccgcca acgctttctg tttgttttcg tcgatagcca tttttactcc 4260
   tgtcatgcct agcccatggg tatggacagt tttccctttg atatgtaacg gtgaacagtt 4320
   gttctacttt tgtttgttag tcttgatgct tcactgatag atacaagagc cataagaacc 4380
   tcagatcctt ccgtatttag ccagtatgtt ctctagtgtg gttcgttgtt tttgcgtgag 4440
   ccatgagaac gaaccattga gatcatactt actttgcatg tcactcaaaa attttgcctc 4500
   aaaactggtg agctgaattt ttgcagttaa agcatcgtgt agtgtttttc ttagtccgtt 4560
   acgtaggtag gaatctgatg taatggttgt tggtattttg tcaccattca tttttatctg 4620
   gttgttctca agttcggtta cgagatccat ttgtctatct agttcaactt ggaaaatcaa 4680
   cgtatcagtc gggcggcctc gcttatcaac caccaatttc atattgctgt aagtgtttaa 4740
   atctttactt attggtttca aaacccattg gttaagcctt ttaaactcat ggtagttatt 4800
   ttcaagcatt aacatgaact taaattcatc aaggctaatc tctatatttg ccttgtgagt 4860
   tttcttttgt gttagttctt ttaataacca ctcataaatc ctcatagagt atttgttttc 4920
   aaaagactta acatgttcca gattatattt tatgaatttt tttaactgga aaagataagg 4980
   caatatctct tcactaaaaa ctaattctaa tttttcgctt gagaacttgg catagtttgt 5040
   ccactggaaa atctcaaagc ctttaaccaa aggattcctg atttccacag ttctcgtcat 5100
   cagctctctg gttgctttag ctaatacacc ataagcattt tccctactga tgttcatcat 5160
   ctgagcgtat tggttataag tgaacgatac cgtccgttct ttccttgtag ggttttcaat 5220
   cgtggggttg agtagtgcca cacagcataa aattagcttg gtttcatgct ccgttaagtc 5280
   atagcgacta atcgctagtt catttgcttt gaaaacaact aattcagaca tacatctcaa 5340
   ttggtctagg tgattttaat cactatacca attgagatgg gctagtcaat gataattact 5400
   agtccttttc ctttgagttg tgggtatctg taaattctgc tagacctttg ctggaaaact 5460
   tgtaaattct gctagaccct ctgtaaattc cgctagacct ttgtgtgttt tttttgttta 5520
   tattcaagtg gttataattt atagaataaa gaaagaataa aaaaagataa aaagaataga 5580
   tcccagccct gtgtataact cactacttta gtcagttccg cagtattaca aaaggatgtc 5640
   gcaaacgctg tttgctcctc tacaaaacag accttaaaac cctaaaggct taagtagcac 5700
   cctcgcaagc tcggttgcgg ccgcaatcgg gcaaatcgct gaatattcct tttgtctccg 5760
   accatcaggc acctgagtcg ctgtcttttt cgtgacattc agttcgctgc gctcacggct 5820
   ctggcagtga atgggggtaa atggcactac aggcgccttt tatggattca tgcaaggaaa 5880
   ctacccataa tacaagaaaa gcccgtcacg ggcttctcag ggcgttttat ggcgggtctg 5940
   ctatgtggtg ctatctgact ttttgctgtt cagcagttcc tgccctctga ttttccagtc 6000
   tgaccacttc ggattatccc gtgacaggtc attcagactg gctaatgcac ccagtaaggc 6060
   agcggtatca tcaacggggt ctgacgctca gtggaacgaa aactcacgtt aagggatttt 6120
   ggtcatgaga ttatcaaaaa ggatcttcac ctagatcctt ttaaattaaa aatgaagttt 6180
   taaatcaatc taaagtatat atgagtaaac ttggtctgac agttaccaat gcttaatcag 6240
   tgaggcacct atctcagcga ttaagggcac caataactgc cttaaaaaaa ttacgccccg 6300
   ccctgccact catcgcagta ctgttgtaat tcattaagca ttctgccgac atggaagcca 6360
   tcacagacgg catgatgaac ctgaatcgcc agcggcatca gcaccttgtc gccttgcgta 6420
   taatatttgc ccatggtgaa aacgggggcg aagaagttgt ccatattggc cacgtttaaa   6480
   tcaaaactgg tgaaactcac ccagggattg gctgagacga aaaacatatt ctcaataaac   6540
   cctttaggga aataggccag gttttcaccg taacacgcca catcttgcga atatatgtgt   6600
   agaaactgcc ggaaatcgtc gtggtattca ctccagagcg atgaaaacgt ttcagtttgc   6660
   tcatggaaaa cggtgtaaca agggtgaaca ctatcccata tcaccagctc accgtctttc   6720
   attgccatac ggaattccgg atgagcattc atcaggcggg caagaatgtg aataaaggcc   6780
   ggataaaact tgtgcttatt tttctttacg gtctttaaaa aggccgtaat atccagctga   6840
   acggtctggt tataggtaca ttgagcaact gactgaaatg cctcaaaatg ttctttacga   6900
   tgccattggg atatatcaac ggtggtatat ccagtgattt ttttctccat tttagcttcc   6960
   ttagctcctg aaaatctcga taactcaaaa aatacgcccg gtagtgatct tatttcatta   7020
   tggtgaaagt tggaacctct tacgtgccga aaagggaata agggcgacac ggaaatgttg   7080
   aatactcata ctcttccttt ttcaatatta ttgaagcatt tatcagggtt attgtctcat   7140
   gagcggatac atatttgaat gtatttagaa aaataaacaa ataggggttc cgcgcacatt   7200
   tccccgaaaa gtgccacctg                                               7220
<210> 3
   <211> 59
   <212> DNA
   <213> Artificial
<220>
   <223> PCR Primer
<400> 3
   gtgtttaatt ctgacaacct gagactcgac ggaaaaatga ttccggggat ccgtcgacc 59
<210> 4
   <211> 58
   <212> DNA
   <213> Artificial
<220>
   <223> PCR Primer
<400> 4
   ttaattgagc tcctgtaccc caccaccgga gacggtttat gtaggctgga gctgcttc 58
<210> 5
   <211> 33
   <212> DNA
   <213> Artificial
<220>
   <223> PCR Primer
<400> 5
   ggtattccat atgatctttg acggaaaggt cgc 33
<210> 6
   <211> 24
   <212> DNA
   <213> Artificial
<220>
   <223> PCR Primer
<400> 6
   cgggatccct aggggcgctg accc 24
<210> 7
   <211> 56
   <212> DNA
   <213> Artificial
<220>
   <223> PCR Primer
<400> 7
   gtttaacttt aagaaggaga tataccatga tctttgacgg aaaggtcgca atcatt 56
<210> 8
   <211> 56
   <212> DNA
   <213> Artificial
<220>
   <223> PCR Primer
<400> 8
   aatgattgcg acctttccgt caaagatcat ggtatatctc cttcttaaag ttaaac 56
<210> 9
   <211> 768
   <212> DNA
   <213> Escherichia coli
<220>
   <221> CDS
   <222> (1)..(768)
<400> 9
   gtg ttt aat tct gac aac ctg aga ctc gac gga aaa tgc gcc atc atc       48
   Val Phe Asn Ser Asp Asn Leu Arg Leu Asp Gly Lys Cys Ala Ile Ile
   1               5                   10                  15
   aca ggt gcg ggt gca ggt att ggt aaa gaa atc gcc att aca ttc gcg       96
   Thr Gly Ala Gly Ala Gly Ile Gly Lys Glu Ile Ala Ile Thr Phe Ala
               20                  25                  30
   aca gct ggc gca tct gtg gtg gtc agt gat att aac gcc gac gca gct      144
   Thr Ala Gly Ala Ser Val Val Val Ser Asp Ile Asn Ala Asp Ala Ala
           35                  40                  45
   aac cat gtt gta gac gaa att caa caa ctg ggt ggt cag gca ttt gcc      192
   Asn His Val Val Asp Glu Ile Gln Gln Leu Gly Gly Gln Ala Phe Ala
       50                  55                  60
   tgc cgt tgt gat att act tcc gaa cag gaa ctc tct gca ctg gca gac      240
   Cys Arg Cys Asp Ile Thr Ser Glu Gln Glu Leu Ser Ala Leu Ala Asp
   65                  70                  75                  80
   ttt gct atc agt aag ctg ggt aaa gtt gat att ctg gtt aac aac gcc       288
   Phe Ala Ile Ser Lys Leu Gly Lys Val Asp Ile Leu Val Asn Asn Ala
                   85                  90                  95
   ggt ggc ggt gga cct aaa ccg ttt gat atg cca atg gcg gat ttt cgc       336
   Gly Gly Gly Gly Pro Lys Pro Phe Asp Met Pro Met Ala Asp Phe Arg
               100                 105                 110
   cgt gct tat gaa ctg aat gtg ttt tct ttt ttc cat ctg tca caa ctt       384
   Arg Ala Tyr Glu Leu Asn Val Phe Ser Phe Phe His Leu Ser Gln Leu
           115                 120                 125
   gtt gcg cca gaa atg gaa aaa aat ggc ggt ggc gtt att ctg acc atc       432
   Val Ala Pro Glu Met Glu Lys Asn Gly Gly Gly Val Ile Leu Thr Ile
       130                 135                 140
   act tct atg gcg gca gaa aat aaa aat ata aac atg act tcc tat gca       480
   Thr Ser Met Ala Ala Glu Asn Lys Asn Ile Asn Met Thr Ser Tyr Ala
   145                 150                 155                 160
   tca tct aaa gct gcg gcc agt cat ctg gtc aga aat atg gcg ttt gac       528
   Ser Ser Lys Ala Ala Ala Ser His Leu Val Arg Asn Met Ala Phe Asp
                   165                 170                 175
   cta ggt gaa aaa aat att cgg gta aat ggc att gcg ccg ggg gca ata       576
   Leu Gly Glu Lys Asn Ile Arg Val Asn Gly Ile Ala Pro Gly Ala Ile
               180                 185                 190
   tta acc gat gcc ctg aaa tcc gtt att aca cca gaa att gaa caa aaa       624
   Leu Thr Asp Ala Leu Lys Ser Val Ile Thr Pro Glu Ile Glu Gln Lys
           195                 200                 205
   atg tta cag cac acg ccg atc aga cgt ctg ggc caa ccg caa gat att       672
   Met Leu Gln His Thr Pro Ile Arg Arg Leu Gly Gln Pro Gln Asp Ile
       210                 215                 220
   gct aac gca gcg ctg ttc ctt tgc tcg cct gct gcg agc tgg gta agc       720
   Ala Asn Ala Ala Leu Phe Leu Cys Ser Pro Ala Ala Ser Trp Val Ser
   225                 230                 235                 240
   gga caa att ctc acc gtc tcc ggt ggt ggg gta cag gag ctc aat taa       768
   Gly Gln Ile Leu Thr Val Ser Gly Gly Gly Val Gln Glu Leu Asn
                   245                 250                 255
<210> 10
   <211> 255
   <212> PRT
   <213> Escherichia coli
<400> 10
   Val Phe Asn Ser Asp Asn Leu Arg Leu Asp Gly Lys Cys Ala Ile Ile
   1               5                   10                  15
   Thr Gly Ala Gly Ala Gly Ile Gly Lys Glu Ile Ala Ile Thr Phe Ala
               20                  25                  30
   Thr Ala Gly Ala Ser Val Val Val Ser Asp Ile Asn Ala Asp Ala Ala
           35                  40                  45
   Asn His Val Val Asp Glu Ile Gln Gln Leu Gly Gly Gln Ala Phe Ala
       50                  55                  60
   Cys Arg Cys Asp Ile Thr Ser Glu Gln Glu Leu Ser Ala Leu Ala Asp
   65                  70                  75                  80
   Phe Ala Ile Ser Lys Leu Gly Lys Val Asp Ile Leu Val Asn Asn Ala
                   85                  90                  95
   Gly Gly Gly Gly Pro Lys Pro Phe Asp Met Pro Met Ala Asp Phe Arg
               100                 105                 110
   Arg Ala Tyr Glu Leu Asn Val Phe Ser Phe Phe His Leu Ser Gln Leu
           115                 120                 125
   Val Ala Pro Glu Met Glu Lys Asn Gly Gly Gly Val Ile Leu Thr Ile
       130                 135                 140
   Thr Ser Met Ala Ala Glu Asn Lys Asn Ile Asn Met Thr Ser Tyr Ala
   145                 150                 155                 160
   Ser Ser Lys Ala Ala Ala Ser His Leu Val Arg Asn Met Ala Phe Asp
                   165                 170                 175
   Leu Gly Glu Lys Asn Ile Arg Val Asn Gly Ile Ala Pro Gly Ala Ile
               180                 185                 190
   Leu Thr Asp Ala Leu Lys Ser Val Ile Thr Pro Glu Ile Glu Gln Lys
           195                 200                 205
   Met Leu Gln His Thr Pro Ile Arg Arg Leu Gly Gln Pro Gln Asp Ile
       210                 215                 220
   Ala Asn Ala Ala Leu Phe Leu Cys Ser Pro Ala Ala Ser Trp Val Ser
   225                 230                 235                 240
   Gly Gln Ile Leu Thr Val Ser Gly Gly Gly Val Gln Glu Leu Asn
                   245                 250                 255
<210> 11
   <211> 813
   <212> DNA
   <213> Clostridium sp.
<220>
   <221> CDS
   <222> (1)..(813)
<400> 11
   atg gat ttt att gat ttt aag gag atg ggc aga atg ggg atc ttt gac       48
   Met Asp Phe Ile Asp Phe Lys Glu Met Gly Arg Met Gly Ile Phe Asp
   1               5                   10                  15
   gga aag gtc gca atc att act ggc ggg ggc aag gcc aaa tcg atc ggc       96
   Gly Lys Val Ala Ile Ile Thr Gly Gly Gly Lys Ala Lys Ser Ile Gly
               20                  25                  30
   tac ggc att gcc gtg gcc tat gct aag gag ggg gcc aac ctg gtc ctg      144
   Tyr Gly Ile Ala Val Ala Tyr Ala Lys Glu Gly Ala Asn Leu Val Leu
           35                  40                  45
   acc ggc aga aac gag cag aaa ctg ctg gac gcc aag gag gag ctg gag      192
   Thr Gly Arg Asn Glu Gln Lys Leu Leu Asp Ala Lys Glu Glu Leu Glu
       50                  55                  60
   cgc ctc tac ggc atc aag gtg ttg ccg ctg gcg gtg gac gtc acc ccc      240
   Arg Leu Tyr Gly Ile Lys Val Leu Pro Leu Ala Val Asp Val Thr Pro
   65                  70                  75                  80
   agc gat gag tcg gag gac cgg gtc aag gaa gcc gtg cag aag gtc atc      288
   Ser Asp Glu Ser Glu Asp Arg Val Lys Glu Ala Val Gln Lys Val Ile
                   85                  90                  95
   gcc gaa ttc ggc cgc atc gac gtg ctg atc aac aac gcc cag gcg tcg      336
   Ala Glu Phe Gly Arg Ile Asp Val Leu Ile Asn Asn Ala Gln Ala Ser
               100                 105                 110
   gcc tcg ggc atc ccc ctg tcc atg cag acc aaa gac cac ttt gac ctg      384
   Ala Ser Gly Ile Pro Leu Ser Met Gln Thr Lys Asp His Phe Asp Leu
           115                 120                 125
   ggc atc tac tcc ggg ctc tac gcc acc ttc tac tac atg agg gag tgc      432
   Gly Ile Tyr Ser Gly Leu Tyr Ala Thr Phe Tyr Tyr Met Arg Glu Cys
       130                 135                 140
   tat ccc tac ctg aag gag acc cag ggc tcg gtc atc aac ttc gcc tcc      480
   Tyr Pro Tyr Leu Lys Glu Thr Gln Gly Ser Val Ile Asn Phe Ala Ser
   145                 150                 155                 160
   ggc gcc ggc ctc ttc ggc aac gtg ggt cag tgc tcc tac gcc gcc gcc      528
   Gly Ala Gly Leu Phe Gly Asn Val Gly Gln Cys Ser Tyr Ala Ala Ala
                   165                 170                 175
   aaa gag ggc atc cgc ggc ctc tcc cgc gtc gcg gcc acc gag tgg ggc      576
   Lys Glu Gly Ile Arg Gly Leu Ser Arg Val Ala Ala Thr Glu Trp Gly
               180                 185                 190
   aag gac aac atc aac gtc aac gtg gtc tgc ccc ctg gcc atg acc gcc      624
   Lys Asp Asn Ile Asn Val Asn Val Val Cys Pro Leu Ala Met Thr Ala
           195                 200                 205
   cag ctg gag aac ttc aag ctc tcc tac cct gag gcc tac gag aaa aac      672
   Gln Leu Glu Asn Phe Lys Leu Ser Tyr Pro Glu Ala Tyr Glu Lys Asn
       210                 215                 220
   ctc aga ggg gtg ccc atg ggc cgc ttc ggt gac ccc gag ctg gac atc      720
   Leu Arg Gly Val Pro Met Gly Arg Phe Gly Asp Pro Glu Leu Asp Ile
   225                 230                 235                 240
   ggc cgg gtc tgc gtg cag ctc ggc tcg ccc gac ttc aag tac atg tcc      768
   Gly Arg Val Cys Val Gln Leu Gly Ser Pro Asp Phe Lys Tyr Met Ser
                   245                 250                 255
   ggc gag acc ctc acc ctg gaa ggc ggc atg ggt cag cgc ccc tag          813
   Gly Glu Thr Leu Thr Leu Glu Gly Gly Met Gly Gln Arg Pro
               260                 265                 270
<210> 12
   <211> 270
   <212> PRT
   <213> Clostridium sp.
<400> 12
   Met Asp Phe Ile Asp Phe Lys Glu Met Gly Arg Met Gly Ile Phe Asp
   1               5                   10                  15
   Gly Lys Val Ala Ile Ile Thr Gly Gly Gly Lys Ala Lys Ser Ile Gly
               20                  25                  30
   Tyr Gly Ile Ala Val Ala Tyr Ala Lys Glu Gly Ala Asn Leu Val Leu
           35                  40                  45
   Thr Gly Arg Asn Glu Gln Lys Leu Leu Asp Ala Lys Glu Glu Leu Glu
       50                  55                  60
   Arg Leu Tyr Gly Ile Lys Val Leu Pro Leu Ala Val Asp Val Thr Pro
   65                  70                  75                  80
   Ser Asp Glu Ser Glu Asp Arg Val Lys Glu Ala Val Gln Lys Val Ile
                   85                  90                  95
   Ala Glu Phe Gly Arg Ile Asp Val Leu Ile Asn Asn Ala Gln Ala Ser
               100                 105                 110
   Ala Ser Gly Ile Pro Leu Ser Met Gln Thr Lys Asp His Phe Asp Leu
           115                 120                 125
   Gly Ile Tyr Ser Gly Leu Tyr Ala Thr Phe Tyr Tyr Met Arg Glu Cys
       130                 135                 140
   Tyr Pro Tyr Leu Lys Glu Thr Gln Gly Ser Val Ile Asn Phe Ala Ser
   145                 150                 155                 160
   Gly Ala Gly Leu Phe Gly Asn Val Gly Gln Cys Ser Tyr Ala Ala Ala
                   165                 170                 175
   Lys Glu Gly Ile Arg Gly Leu Ser Arg Val Ala Ala Thr Glu Trp Gly
               180                 185                 190
   Lys Asp Asn Ile Asn Val Asn Val Val Cys Pro Leu Ala Met Thr Ala
           195                 200                 205
   Gln Leu Glu Asn Phe Lys Leu Ser Tyr Pro Glu Ala Tyr Glu Lys Asn
       210                 215                 220
   Leu Arg Gly Val Pro Met Gly Arg Phe Gly Asp Pro Glu Leu Asp Ile
   225                 230                 235                 240
   Gly Arg Val Cys Val Gln Leu Gly Ser Pro Asp Phe Lys Tyr Met Ser
                   245                 250                 255
   Gly Glu Thr Leu Thr Leu Glu Gly Gly Met Gly Gln Arg Pro
               260                 265                 270
<210> 13
   <211> 774
   <212> DNA
   <213> Clostridium sp.
<220>
   <221> CDS
   <222> (1)..(774)
<400> 13
   atc ttt gac gga aag gtc gca atc att act ggc ggg ggc aag gcc aaa        48
   Ile Phe Asp Gly Lys Val Ala Ile Ile Thr Gly Gly Gly Lys Ala Lys
   1               5                   10                  15
   tcg atc ggc tac ggc att gcc gtg gcc tat gct aag gag ggg gcc aac        96
   Ser Ile Gly Tyr Gly Ile Ala Val Ala Tyr Ala Lys Glu Gly Ala Asn
               20                  25                  30
   ctg gtc ctg acc ggc aga aac gag cag aaa ctg ctg gac gcc aag gag       144
   Leu Val Leu Thr Gly Arg Asn Glu Gln Lys Leu Leu Asp Ala Lys Glu
           35                  40                  45
   gag ctg gag cgc ctc tac ggc atc aag gtg ttg ccg ctg gcg gtg gac       192
   Glu Leu Glu Arg Leu Tyr Gly Ile Lys Val Leu Pro Leu Ala Val Asp
       50                  55                  60
   gtc acc ccc agc gat gag tcg gag gac cgg gtc aag gaa gcc gtg cag       240
   Val Thr Pro Ser Asp Glu Ser Glu Asp Arg Val Lys Glu Ala Val Gln
   65                  70                  75                  80
   aag gtc atc gcc gaa ttc ggc cgc atc gac gtg ctg atc aac aac gcc       288
   Lys Val Ile Ala Glu Phe Gly Arg Ile Asp Val Leu Ile Asn Asn Ala
                   85                  90                  95
   cag gcg tcg gcc tcg ggc atc ccc ctg tcc atg cag acc aaa gac cac       336
   Gln Ala Ser Ala Ser Gly Ile Pro Leu Ser Met Gln Thr Lys Asp His
               100                 105                 110
   ttt gac ctg ggc atc tac tcc ggg ctc tac gcc acc ttc tac tac atg       384
   Phe Asp Leu Gly Ile Tyr Ser Gly Leu Tyr Ala Thr Phe Tyr Tyr Met
           115                 120                 125
   agg gag tgc tat ccc tac ctg aag gag acc cag ggc tcg gtc atc aac       432
   Arg Glu Cys Tyr Pro Tyr Leu Lys Glu Thr Gln Gly Ser Val Ile Asn
       130                 135                 140
   ttc gcc tcc ggc gcc ggc ctc ttc ggc aac gtg ggt cag tgc tcc tac       480
   Phe Ala Ser Gly Ala Gly Leu Phe Gly Asn Val Gly Gln Cys Ser Tyr
   145                 150                 155                 160
   gcc gcc gcc aaa gag ggc atc cgc ggc ctc tcc cgc gtc gcg gcc acc      528
   Ala Ala Ala Lys Glu Gly Ile Arg Gly Leu Ser Arg Val Ala Ala Thr
                   165                 170                 175
   gag tgg ggc aag gac aac atc aac gtc aac gtg gtc tgc ccc ctg gcc      576
   Glu Trp Gly Lys Asp Asn Ile Asn Val Asn Val Val Cys Pro Leu Ala
               180                 185                 190
   atg acc gcc cag ctg gag aac ttc aag ctc tcc tac cct gag gcc tac      624
   Met Thr Ala Gln Leu Glu Asn Phe Lys Leu Ser Tyr Pro Glu Ala Tyr
           195                 200                 205
   gag aaa aac ctc aga ggg gtg ccc atg ggc cgc ttc ggt gac ccc gag      672
   Glu Lys Asn Leu Arg Gly Val Pro Met Gly Arg Phe Gly Asp Pro Glu
       210                 215                 220
   ctg gac atc ggc cgg gtc tgc gtg cag ctc ggc tcg ccc gac ttc aag      720
   Leu Asp Ile Gly Arg Val Cys Val Gln Leu Gly Ser Pro Asp Phe Lys
   225                 230                 235                 240
   tac atg tcc ggc gag acc ctc acc ctg gaa ggc ggc atg ggt cag cgc      768
   Tyr Met Ser Gly Glu Thr Leu Thr Leu Glu Gly Gly Met Gly Gln Arg
                   245                 250                 255
   ccc tag                                                              774
   Pro
<210> 14
   <211> 257
   <212> PRT
   <213> Clostridium sp.
<400> 14
   Ile Phe Asp Gly Lys Val Ala Ile Ile Thr Gly Gly Gly Lys Ala Lys
   1               5                   10                  15
   Ser Ile Gly Tyr Gly Ile Ala Val Ala Tyr Ala Lys Glu Gly Ala Asn
               20                  25                  30
   Leu Val Leu Thr Gly Arg Asn Glu Gln Lys Leu Leu Asp Ala Lys Glu
           35                  40                  45
   Glu Leu Glu Arg Leu Tyr Gly Ile Lys Val Leu Pro Leu Ala Val Asp
       50                  55                  60
   Val Thr Pro Ser Asp Glu Ser Glu Asp Arg Val Lys Glu Ala Val Gln
   65                  70                  75                  80
   Lys Val Ile Ala Glu Phe Gly Arg Ile Asp Val Leu Ile Asn Asn Ala
                   85                  90                  95
   Gln Ala Ser Ala Ser Gly Ile Pro Leu Ser Met Gln Thr Lys Asp His
               100                 105                 110
   Phe Asp Leu Gly Ile Tyr Ser Gly Leu Tyr Ala Thr Phe Tyr Tyr Met
                   115                 120                 125
   Arg Glu Cys Tyr Pro Tyr Leu Lys Glu Thr Gln Gly Ser Val Ile Asn
       130                 135                 140
   Phe Ala Ser Gly Ala Gly Leu Phe Gly Asn Val Gly Gln Cys Ser Tyr
   145                 150                 155                 160
   Ala Ala Ala Lys Glu Gly Ile Arg Gly Leu Ser Arg Val Ala Ala Thr
                   165                 170                 175
   Glu Trp Gly Lys Asp Asn Ile Asn Val Asn Val Val Cys Pro Leu Ala
               180                 185                 190
   Met Thr Ala Gln Leu Glu Asn Phe Lys Leu Ser Tyr Pro Glu Ala Tyr
           195                 200                 205
   Glu Lys Asn Leu Arg Gly Val Pro Met Gly Arg Phe Gly Asp Pro Glu
       210                 215                 220
   Leu Asp Ile Gly Arg Val Cys Val Gln Leu Gly Ser Pro Asp Phe Lys
   225                 230                 235                 240
   Tyr Met Ser Gly Glu Thr Leu Thr Leu Glu Gly Gly Met Gly Gln Arg
                   245                 250                 255
   Pro
<210> 15
   <211> 777
   <212> DNA
   <213> Artificial
<220>
   <223> Mutant Q97H
<220>
   <221> CDS
   <222> (1)..(777)
<400> 15
   atg atc ttt gac gga aag gtc gca atc att act ggc ggg ggc aag gcc        48
   Met Ile Phe Asp Gly Lys Val Ala Ile Ile Thr Gly Gly Gly Lys Ala
   1               5                   10                  15
   aaa tcg atc ggc tac ggc att gcc gtg gcc tat gct aag gag ggg gcc        96
   Lys Ser Ile Gly Tyr Gly Ile Ala Val Ala Tyr Ala Lys Glu Gly Ala
               20                  25                  30
   aac ctg gtc ctg acc ggc aga aac gag cag aaa ctg ctg gac gcc aag       144
   Asn Leu Val Leu Thr Gly Arg Asn Glu Gln Lys Leu Leu Asp Ala Lys
           35                  40                  45
   gag gag ctg gag cgc ctc tac ggc atc aag gtg ttg ccg ctg gcg gtg       192
   Glu Glu Leu Glu Arg Leu Tyr Gly Ile Lys Val Leu Pro Leu Ala Val
       50                  55                  60
   gac gtc acc ccc agc gat gag tcg gag gac cgg gtc aag gaa gcc gtg       240
   Asp Val Thr Pro Ser Asp Glu Ser Glu Asp Arg Val Lys Glu Ala Val
   65                  70                  75                  80
   cag aag gtc atc gcc gaa ttc ggc cgc atc gac gtg ctg atc aac aac       288
   Gln Lys Val Ile Ala Glu Phe Gly Arg Ile Asp Val Leu Ile Asn Asn
                   85                  90                  95
   gcc cat gcg tcg gcc tcg ggc atc ccc ctg tcc atg cag acc aaa gac       336
   Ala His Ala Ser Ala Ser Gly Ile Pro Leu Ser Met Gln Thr Lys Asp
               100                 105                 110
   cac ttt gac ctg ggc atc tac tcc ggg ctc tac gcc acc ttc tac tac       384
   His Phe Asp Leu Gly Ile Tyr Ser Gly Leu Tyr Ala Thr Phe Tyr Tyr
           115                 120                 125
   atg agg gag tgc tat ccc tac ctg aag gag act cag ggc tcg gtc atc       432
   Met Arg Glu Cys Tyr Pro Tyr Leu Lys Glu Thr Gln Gly Ser Val Ile
       130                 135                 140
   aac ttc gcc tcc ggc gcc ggc ctc ttc ggc aac gtg ggt cag tgc tcc       480
   Asn Phe Ala Ser Gly Ala Gly Leu Phe Gly Asn Val Gly Gln Cys Ser
   145                 150                 155                 160
   tac gcc gcc gcc aaa gag ggc atc cgc ggc ctc tcc cgc gtc gcg gcc       528
   Tyr Ala Ala Ala Lys Glu Gly Ile Arg Gly Leu Ser Arg Val Ala Ala
                   165                 170                 175
   acc gag tgg ggc aag gac aac atc aac gtc aac gtg gtc tgc ccc ctg       576
   Thr Glu Trp Gly Lys Asp Asn Ile Asn Val Asn Val Val Cys Pro Leu
               180                 185                 190
   gcc atg acc gcc cag ctg gag aac ttc aag ctc tcc tac cct gag gcc       624
   Ala Met Thr Ala Gln Leu Glu Asn Phe Lys Leu Ser Tyr Pro Glu Ala
           195                 200                 205
   tac gag aaa aac ctc aga ggg gtg ccc atg ggc cgc ttc ggt gac ccc       672
   Tyr Glu Lys Asn Leu Arg Gly Val Pro Met Gly Arg Phe Gly Asp Pro
       210                 215                 220
   gag ctg gac atc ggc cgg gtc tgc gtg cag ctc ggc tcg ccc gac ttc       720
   Glu Leu Asp Ile Gly Arg Val Cys Val Gln Leu Gly Ser Pro Asp Phe
   225                 230                 235                 240
   aag tac atg tcc ggc gag acc ctc acc ctg gaa ggc ggc atg ggt cag       768
   Lys Tyr Met Ser Gly Glu Thr Leu Thr Leu Glu Gly Gly Met Gly Gln
                   245                 250                 255
   cgc ccc tag                                                           777
   Arg Pro
<210> 16
   <211> 258
   <212> PRT
   <213> Artificial
<220>
   <223> Synthetic Construct
<400> 16
   Met Ile Phe Asp Gly Lys Val Ala Ile Ile Thr Gly Gly Gly Lys Ala
   1               5                   10                  15
   Lys Ser Ile Gly Tyr Gly Ile Ala Val Ala Tyr Ala Lys Glu Gly Ala
               20                  25                  30
   Asn Leu Val Leu Thr Gly Arg Asn Glu Gln Lys Leu Leu Asp Ala Lys
           35                  40                  45
   Glu Glu Leu Glu Arg Leu Tyr Gly Ile Lys Val Leu Pro Leu Ala Val
       50                  55                  60
   Asp Val Thr Pro Ser Asp Glu Ser Glu Asp Arg Val Lys Glu Ala Val
   65                  70                  75                  80
   Gln Lys Val Ile Ala Glu Phe Gly Arg Ile Asp Val Leu Ile Asn Asn
                   85                  90                  95
   Ala His Ala Ser Ala Ser Gly Ile Pro Leu Ser Met Gln Thr Lys Asp
               100                 105                 110
   His Phe Asp Leu Gly Ile Tyr Ser Gly Leu Tyr Ala Thr Phe Tyr Tyr
           115                 120                 125
   Met Arg Glu Cys Tyr Pro Tyr Leu Lys Glu Thr Gln Gly Ser Val Ile
       130                 135                 140
   Asn Phe Ala Ser Gly Ala Gly Leu Phe Gly Asn Val Gly Gln Cys Ser
   145                 150                 155                 160
   Tyr Ala Ala Ala Lys Glu Gly Ile Arg Gly Leu Ser Arg Val Ala Ala
                   165                 170                 175
   Thr Glu Trp Gly Lys Asp Asn Ile Asn Val Asn Val Val Cys Pro Leu
               180                 185                 190
   Ala Met Thr Ala Gln Leu Glu Asn Phe Lys Leu Ser Tyr Pro Glu Ala
           195                 200                 205
   Tyr Glu Lys Asn Leu Arg Gly Val Pro Met Gly Arg Phe Gly Asp Pro
       210                 215                 220
   Glu Leu Asp Ile Gly Arg Val Cys Val Gln Leu Gly Ser Pro Asp Phe
   225                 230                 235                 240
   Lys Tyr Met Ser Gly Glu Thr Leu Thr Leu Glu Gly Gly Met Gly Gln
                   245                 250                 255
   Arg Pro
<210> 17
   <211> 53
   <212> DNA
   <213> Artificial
<220>
   <223> PCR Primer
<400> 17
   aaaaaagctt ataattatcc ttataggacg tcatggtgcg cccagatagg gtg 53
<210> 18
   <211> 60
   <212> DNA
   <213> Artificial
<220>
   <223> PCR Primer
<400> 18
   cagattgtac aaatgtggtg ataacagata agtcgtcatg tttaacttac ctttctttgt 60
<210> 19
   <211> 49
   <212> DNA
   <213> Artificial
<220>
   <223> PCR Primer
<400> 19
   tgaacgcaag tttctaattt cggtttccta tcgatagagg aaagtgtct 49
<210> 20
   <211> 27
   <212> DNA
   <213> Artificial
<220>
   <223> PCR Primer
<400> 20
   ttaattgagc tcctgtaccc caccacc 27
<210> 21
   <211> 30
   <212> DNA
   <213> Artificial
<220>
   <223> PCR Primer
<400> 21
   gtgtttaatt ctgacaacct gagactcgac 30
<210> 22
   <211> 1266
   <212> DNA
   <213> Artificial
<220>
   <223> FDH D221G Mutante mit His-Tag
<220>
   <221> CDS
   <222> (1)..(1266)
<400> 22
   atg gca aag gtc ctg tgc gtt ctt tac gat gat ccg gtc gac ggc tac       48
   Met Ala Lys Val Leu Cys Val Leu Tyr Asp Asp Pro Val Asp Gly Tyr
   1               5                   10                  15
   ccg aag acc tat gcc cgc gac gat ctt ccg aag atc gac cac tat ccg       96
   Pro Lys Thr Tyr Ala Arg Asp Asp Leu Pro Lys Ile Asp His Tyr Pro
               20                  25                  30
   ggc ggc cag atc ttg ccg acg ccg aag gcc atc gac ttc acg ccc ggg      144
   Gly Gly Gln Ile Leu Pro Thr Pro Lys Ala Ile Asp Phe Thr Pro Gly
           35                  40                  45
   cag ttg ctc ggc tcc gtc tcc ggc gag ctc ggc ctg cgc gaa tat ctc      192
   Gln Leu Leu Gly Ser Val Ser Gly Glu Leu Gly Leu Arg Glu Tyr Leu
       50                  55                  60
   gaa tcc aac ggc cac acc ctg gtc gtg acc tcc gac aag gac ggc ccc      240
   Glu Ser Asn Gly His Thr Leu Val Val Thr Ser Asp Lys Asp Gly Pro
   65                  70                  75                  80
   gac tcg gtg ttc gag cgc gag ctg gtc gat gcg gat gtc gtc atc tcc      288
   Asp Ser Val Phe Glu Arg Glu Leu Val Asp Ala Asp Val Val Ile Ser
                   85                  90                  95
   cag ccc ttc tgg ccg gcc tat ctg acg ccc gag cgc atc gcc aag gcc      336
   Gln Pro Phe Trp Pro Ala Tyr Leu Thr Pro Glu Arg Ile Ala Lys Ala
               100                 105                 110
   aag aac ctg aag ctc gcg ctc acc gcc ggc atc ggt tcc gac cac gtc      384
   Lys Asn Leu Lys Leu Ala Leu Thr Ala Gly Ile Gly Ser Asp His Val
           115                 120                 125
   gat ctt cag tcg gct atc gac cgc aac gtc acc gtg gcg gaa gtc acc      432
   Asp Leu Gln Ser Ala Ile Asp Arg Asn Val Thr Val Ala Glu Val Thr
       130                 135                 140
   tac tgc aac tcg atc agc gtc gcc gag cat gtg gtg atg atg atc ctg      480
   Tyr Cys Asn Ser Ile Ser Val Ala Glu His Val Val Met Met Ile Leu
   145                 150                 155                 160
   tcg ctg gtg cgc aac tat ctg ccc tcg cac gaa tgg gcg cgg aag ggc      528
   Ser Leu Val Arg Asn Tyr Leu Pro Ser His Glu Trp Ala Arg Lys Gly
                   165                 170                 175
   ggc tgg aac atc gcc gac tgc gtc tcc cac gcc tac gac ctc gag gcg      576
   Gly Trp Asn Ile Ala Asp Cys Val Ser His Ala Tyr Asp Leu Glu Ala
               180                 185                 190
   atg cat gtc ggc acc gtg gcc gcc ggc cgc atc ggt ctc gcg gtg ctg      624
   Met His Val Gly Thr Val Ala Ala Gly Arg Ile Gly Leu Ala Val Leu
           195                 200                 205
   cgc cgt ctg gcg ccg ttc gac gtg cac ctg cac tac acc ggc cgt cac      672
   Arg Arg Leu Ala Pro Phe Asp Val His Leu His Tyr Thr Gly Arg His
       210                 215                 220
   cgc ctg ccg gaa tcg gtc gag aag gag ctc aac ctc acc tgg cac gcg      720
   Arg Leu Pro Glu Ser Val Glu Lys Glu Leu Asn Leu Thr Trp His Ala
   225                 230                 235                 240
   acc cgc gag gac atg tat ccg gtt tgc gac gtg gtg acg ctg aac tgc      768
   Thr Arg Glu Asp Met Tyr Pro Val Cys Asp Val Val Thr Leu Asn Cys
                   245                 250                 255
   ccg ctg cac ccc gaa acc gag cac atg atc aat gac gag acg ctg aag      816
   Pro Leu His Pro Glu Thr Glu His Met Ile Asn Asp Glu Thr Leu Lys
               260                 265                 270
   ctg ttc aag cgt ggc gcc tac atc gtc aac acc gcc cgc ggc aag ctg      864
   Leu Phe Lys Arg Gly Ala Tyr Ile Val Asn Thr Ala Arg Gly Lys Leu
           275                 280                 285
   tgc gac cgc gat gcc gtg gca cgt gcg ctc gaa tcc ggc cgg ctg gcc      912
   Cys Asp Arg Asp Ala Val Ala Arg Ala Leu Glu Ser Gly Arg Leu Ala
       290                 295                 300
   ggc tat gcc ggc gac gtg tgg ttc ccg cag ccg gcg ccg aag gac cac      960
   Gly Tyr Ala Gly Asp Val Trp Phe Pro Gln Pro Ala Pro Lys Asp His
   305                 310                 315                 320
   ccc tgg cgg acg atg ccc tat aac ggc atg acc ccg cac atc tcc ggc     1008
   Pro Trp Arg Thr Met Pro Tyr Asn Gly Met Thr Pro His Ile Ser Gly
                   325                 330                 335
   acc acg ctg acc gcg cag gcg cgt tat gcg gcg ggc acc cgc gag atc     1056
   Thr Thr Leu Thr Ala Gln Ala Arg Tyr Ala Ala Gly Thr Arg Glu Ile
               340                 345                 350
   ctg gag tgc ttc ttc gag ggc cgt ccg atc cgc gac gaa tac ctc atc     1104
   Leu Glu Cys Phe Phe Glu Gly Arg Pro Ile Arg Asp Glu Tyr Leu Ile
           355                 360                 365
   gtg cag ggc ggc gct ctt gcc ggc acc ggc gcg cat tcc tac tcg aag     1152
   Val Gln Gly Gly Ala Leu Ala Gly Thr Gly Ala His Ser Tyr Ser Lys
       370                 375                 380
   ggc aat gcc acc ggc ggt tcg gaa gag gcc gcc aag ttc aag aag gcg     1200
   Gly Asn Ala Thr Gly Gly Ser Glu Glu Ala Ala Lys Phe Lys Lys Ala
   385                 390                 395                 400
   gct gag aat tcg agc tcc gtc gac aag ctt gcg gcc gca ctc gag cac     1248
   Ala Glu Asn Ser Ser Ser Val Asp Lys Leu Ala Ala Ala Leu Glu His
                   405                 410                 415
   cac cac cac cac cac tga                                             1266
   His His His His His
               420
<210> 23
   <211> 421
   <212> PRT
   <213> Artificial
<220>
   <223> Synthetic Construct
<400> 23
   Met Ala Lys Val Leu Cys Val Leu Tyr Asp Asp Pro Val Asp Gly Tyr
   1               5                   10                  15
   Pro Lys Thr Tyr Ala Arg Asp Asp Leu Pro Lys Ile Asp His Tyr Pro
               20                  25                  30
   Gly Gly Gln Ile Leu Pro Thr Pro Lys Ala Ile Asp Phe Thr Pro Gly
           35                  40                  45
   Gln Leu Leu Gly Ser Val Ser Gly Glu Leu Gly Leu Arg Glu Tyr Leu
       50                  55                  60
   Glu Ser Asn Gly His Thr Leu Val Val Thr Ser Asp Lys Asp Gly Pro
   65                  70                  75                  80
   Asp Ser Val Phe Glu Arg Glu Leu Val Asp Ala Asp Val Val Ile Ser
                   85                  90                  95
   Gln Pro Phe Trp Pro Ala Tyr Leu Thr Pro Glu Arg Ile Ala Lys Ala
               100                 105                 110
   Lys Asn Leu Lys Leu Ala Leu Thr Ala Gly Ile Gly Ser Asp His Val
           115                 120                 125
   Asp Leu Gln Ser Ala Ile Asp Arg Asn Val Thr Val Ala Glu Val Thr
       130                 135                 140
   Tyr Cys Asn Ser Ile Ser Val Ala Glu His Val Val Met Met Ile Leu
   145                 150                 155                 160
   Ser Leu Val Arg Asn Tyr Leu Pro Ser His Glu Trp Ala Arg Lys Gly
                   165                 170                 175
   Gly Trp Asn Ile Ala Asp Cys Val Ser His Ala Tyr Asp Leu Glu Ala
               180                 185                 190
   Met His Val Gly Thr Val Ala Ala Gly Arg Ile Gly Leu Ala Val Leu
           195                 200                 205
   Arg Arg Leu Ala Pro Phe Asp Val His Leu His Tyr Thr Gly Arg His
       210                 215                 220
   Arg Leu Pro Glu Ser Val Glu Lys Glu Leu Asn Leu Thr Trp His Ala
   225                 230                 235                 240
   Thr Arg Glu Asp Met Tyr Pro Val Cys Asp Val Val Thr Leu Asn Cys
                   245                 250                 255
   Pro Leu His Pro Glu Thr Glu His Met Ile Asn Asp Glu Thr Leu Lys
               260                 265                 270
   Leu Phe Lys Arg Gly Ala Tyr Ile Val Asn Thr Ala Arg Gly Lys Leu
           275                 280                 285
   Cys Asp Arg Asp Ala Val Ala Arg Ala Leu Glu Ser Gly Arg Leu Ala
       290                 295                 300
   Gly Tyr Ala Gly Asp Val Trp Phe Pro Gln Pro Ala Pro Lys Asp His
   305                 310                 315                 320
   Pro Trp Arg Thr Met Pro Tyr Asn Gly Met Thr Pro His Ile Ser Gly
                   325                 330                 335
   Thr Thr Leu Thr Ala Gln Ala Arg Tyr Ala Ala Gly Thr Arg Glu Ile
               340                 345                 350
   Leu Glu Cys Phe Phe Glu Gly Arg Pro Ile Arg Asp Glu Tyr Leu Ile
           355                 360                 365
   Val Gln Gly Gly Ala Leu Ala Gly Thr Gly Ala His Ser Tyr Ser Lys
       370                 375                 380
   Gly Asn Ala Thr Gly Gly Ser Glu Glu Ala Ala Lys Phe Lys Lys Ala
   385                 390                 395                 400
   Ala Glu Asn Ser Ser Ser Val Asp Lys Leu Ala Ala Ala Leu Glu His
                   405                 410                 415
   His His His His His
               420
<210> 24
   <211> 792
   <212> DNA
   <213> Collinsella aerofaciens
<220>
   <221> CDS
   <222> (1)..(792)
<400> 24
   atg aac ctg agg gag aag tac ggt gag tgg ggc ctg atc ctg ggc gcg        48
   Met Asn Leu Arg Glu Lys Tyr Gly Glu Trp Gly Leu Ile Leu Gly Ala
   1               5                   10                  15
   acc gag ggc gtc ggc aag gcg ttc tgc gag aag atc gcc gcc ggc ggc        96
   Thr Glu Gly Val Gly Lys Ala Phe Cys Glu Lys Ile Ala Ala Gly Gly
               20                  25                  30
   atg aac gtc gtc atg gtc ggc cgt cgc gag gag aag ctg aac gtg ctc      144
   Met Asn Val Val Met Val Gly Arg Arg Glu Glu Lys Leu Asn Val Leu
           35                  40                  45
   gca ggc gag atc cgc gag acc tac ggc gtg gag acc aag gtc gtg cgc      192
   Ala Gly Glu Ile Arg Glu Thr Tyr Gly Val Glu Thr Lys Val Val Arg
       50                  55                  60
   gcc gac ttt agc cag ccc ggc gct gcc gag acc gtc ttc gcc gcg acc      240
   Ala Asp Phe Ser Gln Pro Gly Ala Ala Glu Thr Val Phe Ala Ala Thr
   65                  70                  75                  80
   gag ggc ctg gac atg ggc ttc atg agc tac gtg gcc tgc ctg cac agc      288
   Glu Gly Leu Asp Met Gly Phe Met Ser Tyr Val Ala Cys Leu His Ser
                   85                  90                  95
   ttc ggt aag atc cag gac acc ccc tgg gag aag cac gag gcc atg atc      336
   Phe Gly Lys Ile Gln Asp Thr Pro Trp Glu Lys His Glu Ala Met Ile
               100                 105                 110
   aac gtc aac gtc gtg acc ttc ctc aag tgc ttc cac cac tac atg cgg      384
   Asn Val Asn Val Val Thr Phe Leu Lys Cys Phe His His Tyr Met Arg
           115                 120                 125
   atc ttt gcc gcc cag gac cgc ggc gcc gtg atc aac gtc tcg tcg atg      432
   Ile Phe Ala Ala Gln Asp Arg Gly Ala Val Ile Asn Val Ser Ser Met
       130                 135                 140
   acc ggc atc agc tcc agc ccc tgg aac ggc cag tac ggc gcg ggc aag      480
   Thr Gly Ile Ser Ser Ser Pro Trp Asn Gly Gln Tyr Gly Ala Gly Lys
   145                 150                 155                 160
   gcc ttc atc ctc aag atg acc gag gcc gtg gcc tgc gag tgc gag ggc      528
   Ala Phe Ile Leu Lys Met Thr Glu Ala Val Ala Cys Glu Cys Glu Gly
                   165                 170                 175
   acc ggc gtc gac gtc gag gtc atc acc ctc ggc acc acc cta acc ccc      576
   Thr Gly Val Asp Val Glu Val Ile Thr Leu Gly Thr Thr Leu Thr Pro
               180                 185                 190
   agc ctg ctg tcc aac ctc ccc ggc ggc ccg cag ggc gag gcc gtc atg      624
   Ser Leu Leu Ser Asn Leu Pro Gly Gly Pro Gln Gly Glu Ala Val Met
           195                 200                 205
   aag atc gcc ctc acc ccc gag gag tgc gtt gac gag gcc ttt gag aag      672
   Lys Ile Ala Leu Thr Pro Glu Glu Cys Val Asp Glu Ala Phe Glu Lys
       210                 215                 220
   ctg ggt aag gag ctc tcc gtc atc gcc ggc cag cgc aac aag gac tcc      720
   Leu Gly Lys Glu Leu Ser Val Ile Ala Gly Gln Arg Asn Lys Asp Ser
   225                 230                 235                 240
   gtc cac gac tgg aag gca aac cac acc gag gac gag tac atc cgc tac      768
   Val His Asp Trp Lys Ala Asn His Thr Glu Asp Glu Tyr Ile Arg Tyr
                   245                 250                 255
   atg ggg tcg ttc tac cgc gac tag                                      792
   Met Gly Ser Phe Tyr Arg Asp
               260
<210> 25
   <211> 263
   <212> PRT
   <213> Collinsella aerofaciens
<400> 25
   Met Asn Leu Arg Glu Lys Tyr Gly Glu Trp Gly Leu Ile Leu Gly Ala
   1               5                   10                  15
   Thr Glu Gly Val Gly Lys Ala Phe Cys Glu Lys Ile Ala Ala Gly Gly
               20                  25                  30
   Met Asn Val Val Met Val Gly Arg Arg Glu Glu Lys Leu Asn Val Leu
           35                  40                  45
   Ala Gly Glu Ile Arg Glu Thr Tyr Gly Val Glu Thr Lys Val Val Arg
       50                  55                  60
   Ala Asp Phe Ser Gln Pro Gly Ala Ala Glu Thr Val Phe Ala Ala Thr
   65                  70                  75                  80
   Glu Gly Leu Asp Met Gly Phe Met Ser Tyr Val Ala Cys Leu His Ser
                   85                  90                  95
   Phe Gly Lys Ile Gln Asp Thr Pro Trp Glu Lys His Glu Ala Met Ile
               100                 105                 110
   Asn Val Asn Val Val Thr Phe Leu Lys Cys Phe His His Tyr Met Arg
           115                 120                 125
   Ile Phe Ala Ala Gln Asp Arg Gly Ala Val Ile Asn Val Ser Ser Met
       130                 135                 140
   Thr Gly Ile Ser Ser Ser Pro Trp Asn Gly Gln Tyr Gly Ala Gly Lys
   145                 150                 155                 160
   Ala Phe Ile Leu Lys Met Thr Glu Ala Val Ala Cys Glu Cys Glu Gly
                   165                 170                 175
   Thr Gly Val Asp Val Glu Val Ile Thr Leu Gly Thr Thr Leu Thr Pro
               180                 185                 190
   Ser Leu Leu Ser Asn Leu Pro Gly Gly Pro Gln Gly Glu Ala Val Met
           195                 200                 205
   Lys Ile Ala Leu Thr Pro Glu Glu Cys Val Asp Glu Ala Phe Glu Lys
       210                 215                 220
   Leu Gly Lys Glu Leu Ser Val Ile Ala Gly Gln Arg Asn Lys Asp Ser
   225                 230                 235                 240
   Val His Asp Trp Lys Ala Asn His Thr Glu Asp Glu Tyr Ile Arg Tyr
                   245                 250                 255
   Met Gly Ser Phe Tyr Arg Asp
   260
<210> 26
   <211> 31
   <212> DNA
   <213> artificial
<220>
   <223> PCR Primer
<400> 26
   gggaattcca tatgaacctg agggagaagt a 31
<210> 27
   <211> 27
   <212> DNA
   <213> Artificial
<220>
   <223> PCR Primer
<400> 27
   cccaagcttc tagtcgcggt agaacga 27
<210> 28
   <211> 774
   <212> DNA
   <213> Comamonas testosteroni
<220>
   <221> CDS
   <222> (1)..(774)
<400> 28
   atg tcc atc atc gtg ata agc ggc tgc gcc acc ggc att ggt gcc gct       48
   acg cgc aag gtc ctg gag gcg gcc ggt cac cag atc gta ggc atc gat       96
   ata cgc gat gcg gaa gtg att gcc gat ctc tcg acg gcc gaa ggt cga      144
   aag cag gcg att gcc gat gta ctg gcg aag tgc agc aag ggc atg gac      192
   ggc ctg gtg ctg tgc gcc ggc ctg gga ccg cag acc aag gtg ctt ggc      240
   aat gtg gtt tcg gtc aat tat ttt ggc gcg acc gag ctg atg gat gcc      288
   ttt ttg cca gcg ctg aaa aaa ggc cat cag ccc gca gcc gtc gtc atc      336
   tcg tcc gtg gct tcc gcg cat ctg gct ttt gac aag aac cca ctg gcg      384
   ctg gca ctg gaa gcc ggc gag gaa gcc aag gcc cgc gcc att gtc gaa      432
   cat gcg gga gag cag ggc gga aat ctg gcc tat gcg ggc agc aag aat      480
   gct ttg acg gtg gct gtg cgc aaa cgc gcc gcc gcc tgg ggc gag gct      528
   ggc gtg cgc ctg aac acc atc gcc ccc ggt gca acc gag act ccc ttg      576
   ctg cag gcg ggc ctg cag gac ccg cgc tat ggc gaa tcc att gcc aag      624
   ttc gtt cct ccc atg ggc cgc cgt gcc gag ccg tcc gag atg gcg tcg      672
   gtc atc gcc ttt ttg atg agc ccg gcc gca agc tat gtg cat ggc gcg      720
   cag atc gtc att gat ggc ggc att gat gcg gtg atg cgc ccg aca cag      768
   ttc tga                                                              774
<210> 29
   <211> 257
   <212> PRT
   <213> Comamonas testosteroni
<400> 29
<210> 30
   <211> 969
   <212> DNA
   <213> Rattus norvegicus
<220>
   <221> CDS
   <222> (1)..(969)
<400> 30
   atg gat tcc ata tct ctg cgt gta gca cta aat gat ggt aac ttc att        48
   Met Asp Ser Ile Ser Leu Arg Val Ala Leu Asn Asp Gly Asn Phe Ile
   1               5                   10                  15
   cct gta ctg ggg ttt gga acc act gtg cct gag aag gtt gct aag gat        96
   Pro Val Leu Gly Phe Gly Thr Thr Val Pro Glu Lys Val Ala Lys Asp
               20                  25                  30
   gaa gtt atc aag gct act aaa ata gct ata gat aat gga ttc cgc cat       144
   Glu Val Ile Lys Ala Thr Lys Ile Ala Ile Asp Asn Gly Phe Arg His
           35                  40                  45
   ttt gac tct gct tat ttg tac gaa gta gaa gag gaa gtg ggc caa gcc       192
   Phe Asp Ser Ala Tyr Leu Tyr Glu Val Glu Glu Glu Val Gly Gln Ala
       50                  55                  60
   att aga agc aag att gaa gac ggc act gtg aag aga gaa gat ata ttc       240
   Ile Arg Ser Lys Ile Glu Asp Gly Thr Val Lys Arg Glu Asp Ile Phe
   65                  70                  75                  80
   tat act tca aag ctt tgg agc act ttc cat aga cca gag ctg gtc cga       288
   Tyr Thr Ser Lys Leu Trp Ser Thr Phe His Arg Pro Glu Leu Val Arg
                   85                  90                  95
   act tgc ttg gaa aag aca ctg aaa agc act caa ctg gac tat gtg gat       336
   Thr Cys Leu Glu Lys Thr Leu Lys Ser Thr Gln Leu Asp Tyr Val Asp
               100                 105                 110
   ctt tat att att cat ttc cca atg gct ttg cag cct gga gat ata ttt       384
   Leu Tyr Ile Ile His Phe Pro Met Ala Leu Gln Pro Gly Asp Ile Phe
           115                 120                 125
   ttc cca cga gat gag cat gga aaa cta ttg ttt gaa aca gtg gat atc       432
   Phe Pro Arg Asp Glu His Gly Lys Leu Leu Phe Glu Thr Val Asp Ile
       130                 135                 140
   tgt gac aca tgg gag gcc atg gaa aag tgt aag gat gca gga ttg gcc       480
   Cys Asp Thr Trp Glu Ala Met Glu Lys Cys Lys Asp Ala Gly Leu Ala
   145                 150                 155                 160
   aag tct att ggg gtg tcc aac ttt aac tgc agg cag ctg gag agg att       528
   Lys Ser Ile Gly Val Ser Asn Phe Asn Cys Arg Gln Leu Glu Arg Ile
                   165                 170                 175
   ctg aat aag cca ggg ctc aaa tac aag cct gtg tgc aac cag gtg gaa       576
   Leu Asn Lys Pro Gly Leu Lys Tyr Lys Pro Val Cys Asn Gln Val Glu
               180                 185                 190
   tgt cac ctt tat ctc aac cag agc aaa atg ctg gac tat tgt aag tca       624
   Cys His Leu Tyr Leu Asn Gln Ser Lys Met Leu Asp Tyr Cys Lys Ser
           195                 200                 205
   aaa gac atc att ctg gtt tcc tac tgc acg ctg gga agt tca cga gac       672
   Lys Asp Ile Ile Leu Val Ser Tyr Cys Thr Leu Gly Ser Ser Arg Asp
       210                 215                 220
   aaa aca tgg gtg gat cag aaa agt cca gtt ctc cta gat gat cca gtt       720
   Lys Thr Trp Val Asp Gln Lys Ser Pro Val Leu Leu Asp Asp Pro Val
   225                 230                 235                 240
   ctt tgt gcc ata gca aag aag tac aag caa acc cca gcc cta gtt gcc       768
   Leu Cys Ala Ile Ala Lys Lys Tyr Lys Gln Thr Pro Ala Leu Val Ala
                   245                 250                 255
   ctt cgc tac cag ctg cag cgt ggg gtt gtg ccc ctg atc agg agt ttc      816
   Leu Arg Tyr Gln Leu Gln Arg Gly Val Val Pro Leu Ile Arg Ser Phe
               260                 265                 270
   aac gcg aag cgg atc aaa gag cta aca cag gtt ttt gaa ttc cag ttg      864
   Asn Ala Lys Arg Ile Lys Glu Leu Thr Gln Val Phe Glu Phe Gln Leu
           275                 280                 285
   gct tca gag gac atg aaa gcc ctg gat ggc ttg aac aga aat ttc aga      912
   Ala Ser Glu Asp Met Lys Ala Leu Asp Gly Leu Asn Arg Asn Phe Arg
       290                 295                 300
   tac aac aat gca aaa tat ttt gat gac cat ccc aat cat cca ttt act      960
   Tyr Asn Asn Ala Lys Tyr Phe Asp Asp His Pro Asn His Pro Phe Thr
   305                 310                 315                 320
   gat gaa tag                                                          969
   Asp Glu
<210> 31
   <211> 322
   <212> PRT
   <213> Rattus norvegicus
<400> 31
   Met Asp Ser Ile Ser Leu Arg Val Ala Leu Asn Asp Gly Asn Phe Ile
   1               5                   10                  15
   Pro Val Leu Gly Phe Gly Thr Thr Val Pro Glu Lys Val Ala Lys Asp
               20                  25                  30
   Glu Val Ile Lys Ala Thr Lys Ile Ala Ile Asp Asn Gly Phe Arg His
           35                  40                  45
   Phe Asp Ser Ala Tyr Leu Tyr Glu Val Glu Glu Glu Val Gly Gln Ala
       50                  55                  60
   Ile Arg Ser Lys Ile Glu Asp Gly Thr Val Lys Arg Glu Asp Ile Phe
   65                  70                  75                  80
   Tyr Thr Ser Lys Leu Trp Ser Thr Phe His Arg Pro Glu Leu Val Arg
                   85                  90                  95
   Thr Cys Leu Glu Lys Thr Leu Lys Ser Thr Gln Leu Asp Tyr Val Asp
               100                 105                 110
   Leu Tyr Ile Ile His Phe Pro Met Ala Leu Gln Pro Gly Asp Ile Phe
           115                 120                 125
   Phe Pro Arg Asp Glu His Gly Lys Leu Leu Phe Glu Thr Val Asp Ile
       130                 135                 140
   Cys Asp Thr Trp Glu Ala Met Glu Lys Cys Lys Asp Ala Gly Leu Ala
   145 150 155 160
   Lys Ser Ile Gly Val Ser Asn Phe Asn Cys Arg Gln Leu Glu Arg Ile
   165 170 175
   Leu Asn Lys Pro Gly Leu Lys Tyr Lys Pro Val Cys Asn Gln Val Glu
   180 185 190
   Cys His Leu Tyr Leu Asn Gln Ser Lys Met Leu Asp Tyr Cys Lys Ser
   195 200 205
   Lys Asp Ile Ile Leu Val Ser Tyr Cys Thr Leu Gly Ser Ser Arg Asp
   210 215 220
   Lys Thr Trp Val Asp Gln Lys Ser Pro Val Leu Leu Asp Asp Pro Val
   225 230 235 240
   Leu Cys Ala Ile Ala Lys Lys Tyr Lys Gln Thr Pro Ala Leu Val Ala
   245 250 255
   Leu Arg Tyr Gln Leu Gln Arg Gly Val Val Pro Leu Ile Arg Ser Phe
   260 265 270
   Asn Ala Lys Arg Ile Lys Glu Leu Thr Gln Val Phe Glu Phe Gln Leu
   275 280 285
   Ala Ser Glu Asp Met Lys Ala Leu Asp Gly Leu Asn Arg Asn Phe Arg
   290 295 300
   Tyr Asn Asn Ala Lys Tyr Phe Asp Asp His Pro Asn His Pro Phe Thr
   305 310 315 320
   Asp Glu
<210> 32
   <211> 401
   <212> PRT
   <213> Mycobacterium vaccae
<400> 32
   Met Ala Lys Val Leu Cys Val Leu Tyr Asp Asp Pro Val Asp Gly Tyr
   1               5                   10                  15
   Pro Lys Thr Tyr Ala Arg Asp Asp Leu Pro Lys Ile Asp His Tyr Pro
               20                  25                  30
   Gly Gly Gln Ile Leu Pro Thr Pro Lys Ala Ile Asp Phe Thr Pro Gly
           35                  40                  45
   Gln Leu Leu Gly Ser Val Ser Gly Glu Leu Gly Leu Arg Glu Tyr Leu
       50                  55                  60
   Glu Ser Asn Gly His Thr Leu Val Val Thr Ser Asp Lys Asp Gly Pro
   65                  70                  75                  80
   Asp Ser Val Phe Glu Arg Glu Leu Val Asp Ala Asp Val Val Ile Ser
                   85                  90                  95
   Gln Pro Phe Trp Pro Ala Tyr Leu Thr Pro Glu Arg Ile Ala Lys Ala
               100                 105                 110
   Lys Asn Leu Lys Leu Ala Leu Thr Ala Gly Ile Gly Ser Asp His Val
           115                 120                 125
   Asp Leu Gln Ser Ala Ile Asp Arg Asn Val Thr Val Ala Glu Val Thr
       130                 135                 140
   Tyr Cys Asn Ser Ile Ser Val Ala Glu His Val Val Met Met Ile Leu
   145                 150                 155                 160
   Ser Leu Val Arg Asn Tyr Leu Pro Ser His Glu Trp Ala Arg Lys Gly
                   165                 170                 175
   Gly Trp Asn Ile Ala Asp Cys Val Ser His Ala Tyr Asp Leu Glu Ala
               180                 185                 190
   Met His Val Gly Thr Val Ala Ala Gly Arg Ile Gly Leu Ala Val Leu
           195                 200                 205
   Arg Arg Leu Ala Pro Phe Asp Val His Leu His Tyr Thr Asp Arg His
       210                 215                 220
   Arg Leu Pro Glu Ser Val Glu Lys Glu Leu Asn Leu Thr Trp His Ala
   225                 230                 235                 240
   Thr Arg Glu Asp Met Tyr Pro Val Cys Asp Val Val Thr Leu Asn Cys
                   245                 250                 255
   Pro Leu His Pro Glu Thr Glu His Met Ile Asn Asp Glu Thr Leu Lys
               260                 265                 270
   Leu Phe Lys Arg Gly Ala Tyr Ile Val Asn Thr Ala Arg Gly Lys Leu
           275                 280                 285
   Cys Asp Arg Asp Ala Val Ala Arg Ala Leu Glu Ser Gly Arg Leu Ala
       290                 295                 300
   Gly Tyr Ala Gly Asp Val Trp Phe Pro Gln Pro Ala Pro Lys Asp His
   305                 310                 315                 320
   Pro Trp Arg Thr Met Pro Tyr Asn Gly Met Thr Pro His Ile Ser Gly
                   325                 330                 335
   Thr Thr Leu Thr Ala Gln Ala Arg Tyr Ala Ala Gly Thr Arg Glu Ile
               340                 345                 350
   Leu Glu Cys Phe Phe Glu Gly Arg Pro Ile Arg Asp Glu Tyr Leu Ile
           355                 360                 365
   Val Gln Gly Gly Ala Leu Ala Gly Thr Gly Ala His Ser Tyr Ser Lys
       370                 375                 380
   Gly Asn Ala Thr Gly Gly Ser Glu Glu Ala Ala Lys Phe Lys Lys Ala
   385                 390                 395                 400
   Val
<210> 33
   <211> 29
   <212> DNA
   <213> Artificial
<220>
   <223> PCR Primer
<400> 33
   cgatcatatg gcaaaggtcc tgtgcgttc 29
<210> 34
   <211> 29
   <212> DNA
   <213> Artificial
<220>
   <223> PCR Primer
<400> 34
   gctagaattc tcagccgcct tcttgaact 29
<210> 35
   <211> 29
   <212> DNA
   <213> Artificial
<220>
   <223> PCR Primer
<400> 35
   cctgcactac accggccgtc accgcctgc 29
<210> 36
   <211> 23
   <212> DNA
   <213> Artificial
<220>
   <223> PCR Primer
<400> 36
   gctcgaattc tcagaccgcc ttc 23
<210> 37
   <211> 1206
   <212> DNA
   <213> Artificial
<220>
   <223> Formiate Deydrogenase Mutant
<220>
   <221> CDS
   <222> (1)..(1206)
<400> 37
   atg gca aag gtc ctg tgc gtt ctt tac gat gat ccg gtc gac ggc tac      48
   Met Ala Lys Val Leu Cys Val Leu Tyr Asp Asp Pro Val Asp Gly Tyr
   1               5                   10                  15
   ccg aag acc tat gcc cgc gac gat ctt ccg aag atc gac cac tat ccg      96
   Pro Lys Thr Tyr Ala Arg Asp Asp Leu Pro Lys Ile Asp His Tyr Pro
               20                  25                  30
   ggc ggc cag atc ttg ccg acg ccg aag gcc atc gac ttc acg ccc ggg     144
   Gly Gly Gln Ile Leu Pro Thr Pro Lys Ala Ile Asp Phe Thr Pro Gly
           35                  40                  45
   cag ttg ctc ggc tcc gtc tcc ggc gag ctc ggc ctg cgc gaa tat ctc     192
   Gln Leu Leu Gly Ser Val Ser Gly Glu Leu Gly Leu Arg Glu Tyr Leu
       50                  55                  60
   gaa tcc aac ggc cac acc ctg gtc gtg acc tcc gac aag gac ggc ccc     240
   Glu Ser Asn Gly His Thr Leu Val Val Thr Ser Asp Lys Asp Gly Pro
   65                  70                  75                  80
   gac tcg gtg ttc gag cgc gag ctg gtc gat gcg gat gtc gtc atc tcc     288
   Asp Ser Val Phe Glu Arg Glu Leu Val Asp Ala Asp Val Val Ile Ser
                   85                  90                  95
   cag ccc ttc tgg ccg gcc tat ctg acg ccc gag cgc atc gcc aag gcc     336
   Gln Pro Phe Trp Pro Ala Tyr Leu Thr Pro Glu Arg Ile Ala Lys Ala
               100                 105                 110
   aag aac ctg aag ctc gcg ctc acc gcc ggc atc ggt tcc gac cac gtc     384
   Lys Asn Leu Lys Leu Ala Leu Thr Ala Gly Ile Gly Ser Asp His Val
           115                 120                 125
   gat ctt cag tcg gct atc gac cgc aac gtc acc gtg gcg gaa gtc acc     432
   Asp Leu Gln Ser Ala Ile Asp Arg Asn Val Thr Val Ala Glu Val Thr
       130                 135                 140
   tac tgc aac tcg atc agc gtc gcc gag cat gtg gtg atg atg atc ctg     480
   Tyr Cys Asn Ser Ile Ser Val Ala Glu His Val Val Met Met Ile Leu
   145                 150                 155                 160
   tcg ctg gtg cgc aac tat ctg ccc tcg cac gaa tgg gcg cgg aag ggc      528
   Ser Leu Val Arg Asn Tyr Leu Pro Ser His Glu Trp Ala Arg Lys Gly
                   165                 170                 175
   ggc tgg aac atc gcc gac tgc gtc tcc cac gcc tac gac ctc gag gcg      576
   Gly Trp Asn Ile Ala Asp Cys Val Ser His Ala Tyr Asp Leu Glu Ala
               180                 185                 190
   atg cat gtc ggc acc gtg gcc gcc ggc cgc atc ggt ctc gcg gtg ctg      624
   Met His Val Gly Thr Val Ala Ala Gly Arg Ile Gly Leu Ala Val Leu
           195                 200                 205
   cgc cgt ctg gcg ccg ttc gac gtg cac ctg cac tac acc ggc cgt cac      672
   Arg Arg Leu Ala Pro Phe Asp Val His Leu His Tyr Thr Gly Arg His
       210                 215                 220
   cgc ctg ccg gaa tcg gtc gag aag gag ctc aac ctc acc tgg cac gcg      720
   Arg Leu Pro Glu Ser Val Glu Lys Glu Leu Asn Leu Thr Trp His Ala
   225                 230                 235                 240
   acc cgc gag gac atg tat ccg gtt tgc gac gtg gtg acg ctg aac tgc      768
   Thr Arg Glu Asp Met Tyr Pro Val Cys Asp Val Val Thr Leu Asn Cys
                   245                 250                 255
   ccg ctg cac ccc gaa acc gag cac atg atc aat gac gag acg ctg aag      816
   Pro Leu His Pro Glu Thr Glu His Met Ile Asn Asp Glu Thr Leu Lys
               260                 265                 270
   ctg ttc aag cgt ggc gcc tac atc gtc aac acc gcc cgc ggc aag ctg      864
   Leu Phe Lys Arg Gly Ala Tyr Ile Val Asn Thr Ala Arg Gly Lys Leu
           275                 280                 285
   tgc gac cgc gat gcc gtg gca cgt gcg ctc gaa tcc ggc cgg ctg gcc      912
   Cys Asp Arg Asp Ala Val Ala Arg Ala Leu Glu Ser Gly Arg Leu Ala
       290                 295                 300
   ggc tat gcc ggc gac gtg tgg ttc ccg cag ccg gcg ccg aag gac cac      960
   Gly Tyr Ala Gly Asp Val Trp Phe Pro Gln Pro Ala Pro Lys Asp His
   305                 310                 315                 320
   ccc tgg cgg acg atg ccc tat aac ggc atg acc ccg cac atc tcc ggc     1008
   Pro Trp Arg Thr Met Pro Tyr Asn Gly Met Thr Pro His Ile Ser Gly
                   325                 330                 335
   acc acg ctg acc gcg cag gcg cgt tat gcg gcg ggc acc cgc gag atc     1056
   Thr Thr Leu Thr Ala Gln Ala Arg Tyr Ala Ala Gly Thr Arg Glu Ile
               340                 345                 350
   ctg gag tgc ttc ttc gag ggc cgt ccg atc cgc gac gaa tac ctc atc     1104
   Leu Glu Cys Phe Phe Glu Gly Arg Pro Ile Arg Asp Glu Tyr Leu Ile
           355                 360                 365
   gtg cag ggc ggc gct ctt gcc ggc acc ggc gcg cat tcc tac tcg aag     1152
   Val Gln Gly Gly Ala Leu Ala Gly Thr Gly Ala His Ser Tyr Ser Lys
       370                 375                 380
   ggc aat gcc acc ggc ggt tcg gaa gag gcc gcc aag ttc aag aag gcg     1200
   Gly Asn Ala Thr Gly Gly Ser Glu Glu Ala Ala Lys Phe Lys Lys Ala
   385                 390                 395                 400
   gtc tga                                                             1206
   Val
<210> 38
   <211> 401
   <212> PRT
   <213> Artificial
<220>
   <223> Synthetic Construct
<400> 38
   Met Ala Lys Val Leu Cys Val Leu Tyr Asp Asp Pro Val Asp Gly Tyr
   1               5                   10                  15
   Pro Lys Thr Tyr Ala Arg Asp Asp Leu Pro Lys Ile Asp His Tyr Pro
               20                  25                  30
   Gly Gly Gln Ile Leu Pro Thr Pro Lys Ala Ile Asp Phe Thr Pro Gly
           35                  40                  45
   Gln Leu Leu Gly Ser Val Ser Gly Glu Leu Gly Leu Arg Glu Tyr Leu
       50                  55                  60
   Glu Ser Asn Gly His Thr Leu Val Val Thr Ser Asp Lys Asp Gly Pro
   65                  70                  75                  80
   Asp Ser Val Phe Glu Arg Glu Leu Val Asp Ala Asp Val Val Ile Ser
                   85                  90                  95
   Gln Pro Phe Trp Pro Ala Tyr Leu Thr Pro Glu Arg Ile Ala Lys Ala
               100                 105                 110
   Lys Asn Leu Lys Leu Ala Leu Thr Ala Gly Ile Gly Ser Asp His Val
           115                 120                 125
   Asp Leu Gln Ser Ala Ile Asp Arg Asn Val Thr Val Ala Glu Val Thr
       130                 135                 140
   Tyr Cys Asn Ser Ile Ser Val Ala Glu His Val Val Met Met Ile Leu
   145                 150                 155                 160
   Ser Leu Val Arg Asn Tyr Leu Pro Ser His Glu Trp Ala Arg Lys Gly
                   165                 170                 175
   Gly Trp Asn Ile Ala Asp Cys Val Ser His Ala Tyr Asp Leu Glu Ala
               180                 185                 190
   Met His Val Gly Thr Val Ala Ala Gly Arg Ile Gly Leu Ala Val Leu
           195                 200                 205
   Arg Arg Leu Ala Pro Phe Asp Val His Leu His Tyr Thr Gly Arg His
       210                 215                 220
   Arg Leu Pro Glu Ser Val Glu Lys Glu Leu Asn Leu Thr Trp His Ala
   225                 230                 235                 240
   Thr Arg Glu Asp Met Tyr Pro Val Cys Asp Val Val Thr Leu Asn Cys
                   245                 250                 255
   Pro Leu His Pro Glu Thr Glu His Met Ile Asn Asp Glu Thr Leu Lys
               260                 265                 270
   Leu Phe Lys Arg Gly Ala Tyr Ile Val Asn Thr Ala Arg Gly Lys Leu
           275                 280                 285
   Cys Asp Arg Asp Ala Val Ala Arg Ala Leu Glu Ser Gly Arg Leu Ala
       290                 295                 300
   Gly Tyr Ala Gly Asp Val Trp Phe Pro Gln Pro Ala Pro Lys Asp His
   305                 310                 315                 320
   Pro Trp Arg Thr Met Pro Tyr Asn Gly Met Thr Pro His Ile Ser Gly
                   325                 330                 335
   Thr Thr Leu Thr Ala Gln Ala Arg Tyr Ala Ala Gly Thr Arg Glu Ile
               340                 345                 350
   Leu Glu Cys Phe Phe Glu Gly Arg Pro Ile Arg Asp Glu Tyr Leu Ile
           355                 360                 365
   Val Gln Gly Gly Ala Leu Ala Gly Thr Gly Ala His Ser Tyr Ser Lys
       370                 375                 380
   Gly Asn Ala Thr Gly Gly Ser Glu Glu Ala Ala Lys Phe Lys Lys Ala
   385                 390                 395                 400
   Val